(19) Europäisches Patentamt
European Patent Office
Office européen des brevets

(11) EP 2 116 544 B1

(12) # EUROPÄISCHE PATENTSCHRIFT

(45) Veröffentlichungstag und Bekanntmachung des Hinweises auf die Patenterteilung:
**18.05.2016 Patentblatt 2016/20**

(21) Anmeldenummer: **09009500.1**

(22) Anmeldetag: **05.11.2003**

(51) Int Cl.:
***C07D 491/10*** (2006.01) ***C07D 495/10*** (2006.01)
***C07D 493/10*** (2006.01) ***C07D 471/10*** (2006.01)
***A61K 31/407*** (2006.01) ***A61P 25/00*** (2006.01)

(54) **Spirocyclische Cyclohexan-Derivate**
Spirocyclic cyclohexane derivatives
Dérivés de cyclohexane spirocycliques

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV**

(30) Priorität: **11.11.2002 DE 10252667**

(43) Veröffentlichungstag der Anmeldung:
**11.11.2009 Patentblatt 2009/46**

(62) Dokumentnummer(n) der früheren Anmeldung(en) nach Art. 76 EPÜ:
**03810963.3 / 1 560 835**

(73) Patentinhaber: **Grünenthal GmbH**
**52078 Aachen (DE)**

(72) Erfinder:
- **Hinze, Claudia**
  **53175 Bonn (DE)**
- **Aulenbacher, Otto**
  **52134 Herzogenrath (DE)**
- **Sundermann, Bernd**
  **61381 Friedrichsdorf (DE)**
- **Oberbörsch**
  **52074 Aachen (DE)**
- **Friderichs, Elmar**
  **52223 Stolberg (DE)**
- **Englberger, Werner**
  **52223 Stolberg (DE)**
- **Kögel, Babette-Yvonne**
  **52379 Langerwehe-Hamich (DE)**
- **Linz, Klaus**
  **53343 Wachtberg (DE)**
- **Schick, Hans**
  **13086 Berlin-Weissensee (DE)**
- **Sonnenschein, Helmut**
  **10245 Berlin (DE)**
- **Henkel, Birgitta**
  **12555 Berlin (DE)**
- **Rose, Valerie Sarah**
  **Saffron Walden, Essex CB10, 1XL (GB)**
- **Lipkin, Michael Jonathan**
  **Saffron Walden, Essex CB10, 1XL (GB)**

(74) Vertreter: **Bülle, Jan et al**
**Kutzenberger Wolff & Partner**
**Theodor-Heuss-Ring 23**
**50668 Köln (DE)**

(56) Entgegenhaltungen:
**EP-A- 0 466 548** **EP-A- 1 142 587**
**WO-A-99/64420** **DE-A1- 2 226 340**

- **MAYER J P ET AL: "Application Of The Pictet-Spengler Reaction In Combinatorial Chemistry" TETRAHEDRON LETTERS, ELSEVIER SCIENCE PUBLISHERS, AMSTERDAM, NL, Bd. 37, Nr. 32, 5. August 1996 (1996-08-05), Seiten 5633-5636, XP004030498 ISSN: 0040-4039**
- **NAGY T ET AL: "Synthesis and analgesic evaluation of some 1,1-disubstituted 3-carboxy-4-phenyl-1,2,3,4-tetrahydro-beta -carboline derivatives" EUROPEAN JOURNAL OF MEDICINAL CHEMISTRY, EDITIONS SCIENTIFIQUE ELSEVIER, PARIS, FR, Bd. 30, Nr. 7, 1995, Seiten 575-586, XP004040182 ISSN: 0223-5234**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **Ingwer Koch: "The Same Invention or Not the Same Invention - That is the Question. But What is the Answer", Internation federation of Intellectual Property Attorneys, 12th Open Forum, 1 January 2010 (2010-01-01), pages 1-25, XP055187290,**


Bemerkungen:

Die Akte enthält technische Angaben, die nach dem Eingang der Anmeldung eingereicht wurden und die nicht in dieser Patentschrift enthalten sind.

## Beschreibung

Die vorliegende Erfindung betrifft spirocyclische Cyclohexan-Derivate, Verfahren zu deren Herstellung, Arzneimittel enthaltend diese Verbindungen und die Verwendung von spirocyclischen Cyclohexan-Derivaten zur Herstellung von Arzneimitteln.

Das Heptadekapeptid Nociceptin ist ein endogener Ligand des ORL1 (Opioid-Receptor-Like)-Rezeptors (Meunier et al., Nature 377, 1995, S. 532-535), der zu der Familie der Opioid Rezeptoren gehört und in vielen Regionen des Gehirns und des Rückenmarks zu finden ist und eine hohe Affinität für den ORL1-Rezeptor aufweist. Der ORL1-Rezeptor ist homolog zu den μ, κ und δ Opioid-Rezeptoren und die Aminosäuresequenz des Nociceptin-Peptids weist eine starke Ähnlichkeit mit denen der bekannten Opioidpeptide auf. Die durch das Nociceptin induzierte Aktivierung des Rezeptors führt über die Kopplung mit Gi/o-Proteinen zu einer Inhibierung der Adenylatcyclase (Meunier et al., Nature 377, 1995, S. 532-535).

Das Nociceptin-Peptid zeigt nach intercerebroventicularer Applikation eine pronociceptive und hyperalgetische Aktivität in verschiedenen Tiermodellen (Reinscheid et al., Science 270, 1995, S. 792-794). Diese Befunde können als Hemmung der stressinduzierten Analgesie erklärt werden (Mogil et al., Neuroscience 75, 1996, S. 333-337). In diesem Zusammenhang konnte auch eine anxiolytische Aktivität des Nociceptin nachgewiesen werden (Jenck et al., Proc. Natl. Acad. Sci. USA 94, 1997, 14854-14858).

Auf der anderen Seite konnte in verschiedenen Tiermodellen, insbesondere nach intrathekaler Applikation, auch ein antinociceptiver Effekt von Nociceptin gezeigt werden. Nociceptin wirkt antinociceptiv in verschiedenen Schmerzmodellen, Beispielsweise im Tail Flick-Test in der Maus (King et al., Neurosci. Lett., 223, 1997, 113-116. In Modellen für neuropathische Schmerzen konnte ebenfalls eine antinociceptive Wirkung von Nociceptin nachgewiesen, die insofern besonders interessant ist, als dass die Wirksamkeit von Nociceptin nach Axotomie von Spinalnerven zunimmt. Dies steht im Gegensatz zu den klassischen Opioiden, deren Wirksamkeit unter diesen Bedingungen abnimmt (Abdulla und Smith, J. Neurosci., 18, 1998, S. 9685-9694).

Der ORL1-Rezeptor ist außerdem noch an der Regulation weiterer physiologischer und pathophysiologischer Prozesse beteiligt. Hierzu gehören unter anderem Lernen und Gedächtnisbildung (Manabe et al., Nature, 394, 1997, S. 577-581), Hörvermögen (Nishi et al., EMBO J., 16, 1997, S. 1858-1864) sowie zahlreiche weitere Prozesse. In einem Übersichtsartikel von Calo et al. (Br.J. Pharmacol., 129, 2000, 1261 - 1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf μ-Agonisten wie Morphin, Behandlung von Entzugserscheinungen, Reduzierung des Suchtpotentials von Opioiden, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, arterieller Blutdruck, Wasserspeicher-Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootropika diskutiert.

Entsprechend vielfältig sind die Anwendungsmöglichkeiten von Verbindungen, die an den ORL1-Rezeptor binden und diesen aktivieren oder inhibieren. Neben diesem spielen aber gerade im Bereich der Schmerztherapie aber auch anderen der genannten Indikationen Opioidrezeptoren wie der μ-Rezeptor, aber auch die anderen Subtypen dieser Opioid-rezeptoren, nämlich δ und κ eine große Rolle. Entsprechend ist es günstig, wenn die Verbindung auch Wirkung an diesen Opioidrezeptoren zeigen.

Aufgabe der vorliegenden Erfindung war es, Arzneimittel zur Verfügung zu stellen, die auf das Nociceptin/ORL1-Rezeptor-System wirken und damit für Arzneimittel insbesondere zur Behandlung der verschiedenen mit diesem System nach dem Stand der Technik in Verbindung stehenden Krankeiten bzw. zum Einsatz in den dort genannten Indikationen geeignet sind.

Gegenstand der Erfindung sind daher spirocyclische Cyclohexan-Derivate der allgemeinen Formel I,

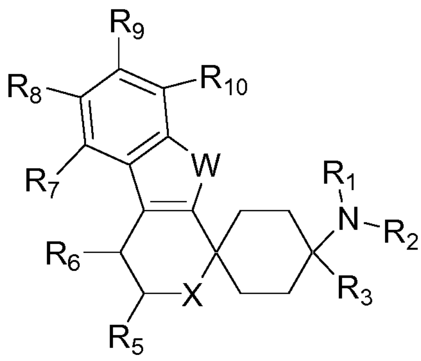

, worin

$R^1$ und $R^2$, unabhängig voneinander für H; CHO; $C_{1-5}$-Alkyl jeweils gesättigt oder ungesättigt, verzweigt oder unverzweigt, unsubstituiert stehen;
oder die Reste $R^1$ und $R^2$ zusammen für $CH_2CH_2OCH_2CH_2$, $CH_2CH_2NR^{11}CH_2CH_2$ oder $(CH_2)_{3-6}$ stehen,
wobei $R^{11}$ H; oder $C_{1-5}$-Alkyl, jeweils gesättigt oder ungesättigt, verzweigt oder unverzweigt, unsubstituiert bedeutet;
$R^3$ für $C_{1-5}$-Alkyl, jeweils gesättigt oder ungesättigt, verzweigt oder unverzweigt, einfach oder mehrfach substituiert oder unsubstituiert; $C_{3-8}$-(Hetero-)Cycloalkyl, jeweils gesättigt oder ungesättigt, einfach oder mehrfach substituiert oder unsubstituiert; Aryl, jeweils unsubstituiert oder einfach oder mehrfach substituiert; über $C_{1-3}$-Alkyl-Gruppe, jeweils gesättigt oder ungesättigt, verzweigt oder unverzweigt, gebundenes Aryl oder $C_{3-8}$-Hetero-)Cycloalkyl, jeweils unsubstituiert oder einfach oder mehrfach substituiert, steht;
W für $NR^4$, O oder S steht
und
$R^4$ für H; $C_{1-5}$-Alkyl, gesättigt oder ungesättigt, verzweigt oder unverzweigt, unsubstituiert oder einfach oder mehrfach substituiert; oder Aryl, substituiert oder unsubstituiert steht,
wobei $R^{12}$ H; $C_{1-5}$-Alkyl, jeweils gesättigt oder ungesättigt, verzweigt oder unverzweigt, einfach oder mehrfach substituiert oder unsubstituiert; $C_{3-8}$-(Hetero-)Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl, jeweils gesättigt oder ungesättigt, verzweigt oder unverzweigt, gebundenes Aryl, $C_{3-8}$-Hetero-)Cycloalkyl oder Heteroaryl, jeweils einfach oder mehrfach substituiert oder unsubstituiert; $OR^{13}$; $NR^{14}R^{15}$ bedeutet;
$R^5$ für (Hetero-)Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl, jeweils gesättigt oder ungesättigt, verzweigt oder unverzweigt, gebundenes $C_{3-8}$-(Hetero-)Cycloalkyl oder Heteroaryl, unsubstituiert oder einfach oder mehrfach substituiert, steht;
$R^6$ für H; F, Cl, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$-Alkyl, gesättigt oder ungesättigt, verzweigt oder unverzweigt, unsubstituiert oder einfach oder mehrfach substituiert; Aryl-, unsubstituiert oder einfach oder mehrfach substituiert; oder über $C_{1-3}$-Alkyl, jeweils gesättigt oder ungesättigt, verzweigt oder unverzweigt, gebundenes Aryl, unsubstituiert oder einfach oder mehrfach substituiert, steht;
$R^7$, $R^8$, $R^9$ und $R^{10}$ unabhängig voneinander für H, F, Cl, Br, I, $NO_2$, $CF_3$, $OR^{13}$, $SR^{13}$, $SO_2R^{13}$, $SO_2OR^{13}$, $SO_2NH_2$, CN, $COOR^{13}$, $NR^{14}R^{15}$; $C_{1-5}$-Alkyl, $C_{3-8}$-(Hetero-)Cycloalkyl, unsubstituiert oder einfach oder mehrfach substituiert; Aryl-, oder Heteroaryl, unsubstituiert oder einfach oder mehrfach substituiert; oder über $C_{1-3}$-Alkyl, jeweils gesättigt oder ungesättigt, verzweigt oder unverzweigt, gebundenes Aryl, $C_{3-8}$-(Hetero-)Cycloalkyl oder Heteroaryl, unsubstituiert oder einfach oder mehrfach substituiert, stehen;
wobei $R^{13}$ H; $C_{1-5}$-Alkyl jeweils gesättigt oder ungesättigt, verzweigt oder unverzweigt, unsubstituiert oder $C_{3-8}$-(Hetero-)Cycloalkyl, jeweils gesättigt oder ungesättigt, unsubstituiert bedeutet;
$R^{14}$ und $R^{15}$ unabhängig voneinander H; $C_{1-5}$-Alkyl, jeweils gesättigt oder ungesättigt, verzweigt oder unverzweigt, unsubstituiert bedeuten;
oder $R^{14}$ und $R^{15}$ zusammen $CH_2CH_2OCH_2CH_2$, $CH_2CH_2NR^{16}CH_2CH_2$ oder $(CH_{2)3-6}$ bilden,
wobei $R^{16}$ H; oder $C_{1-5}$-Alkyl gesättigt oder ungesättigt, verzweigt oder unverzweigt, unsubstituiert bedeutet;
X für $NR^{17}$ steht;
$R^{17}$ für H; $C_{1-5}$-Alkyl, gesättigt oder ungesättigt, verzweigt oder unverzweigt; $COR^{12}$ oder $SO_2R^{12}$,

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Bei der Zusammenfassung verschiedener Reste, Beispielsweise $R^7$, $R^8$, $R^9$ und $R^{10}$ sowie der Zusammenfassung von Resten an deren Substituenten, wie z. B. $OR^{13}$, $SR^{13}$, $SO_2R^{13}$ oder $COOR^{13}$, kann ein Substituent, z.B. $R^{13}$, für zwei oder mehrere Reste, Beispielsweise $R^7$, $R^8$, $R^9$ und $R^{10}$, innerhalb einer Substanz unterschiedliche Bedeutungen annehmen.

Die erfindungsgemäßen Verbindungen zeigen gute Bindung an den ORL1-Rezeptor, aber auch an andere Opioidrezeptoren.

Die Ausdrücke "$C1_{-5}$-Alkyl" und "$C_{1-3}$-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1, 2, 3, 4 oder 5 C-Atomen bzw. 1, 2 oder 3 C-Atomen, d.h. $C_{1-5}$-Alkanyle, $C_{2-5}$-Alkenyle und $C_{2-5}$-Alkinyle bzw. $C_{1-3}$-Alkanyle, $C_{2-3}$-Alkenyle und $C_{2-3}$-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, 2-Hexyl; Ethylenyl (Vinyl), Ethinyl, Propenyl ($-CH_2CH=CH_2$, $-CH=CH-CH_3$, $-C(=CH_2)-CH_3$), Propinyl ($-CH-C\equiv CH$, $-C=C-CH_3$), 1,1-Dimethylethyl, 1,1-Dimethylpropyl, Butenyl, Butinyl, Pentenyl und Pentinyl, umfaßt.

Der Ausdruck "(Hetero-)Cycloalkyl" oder "$C_{3-8}$-(Hetero-)Cycloalkyl" bedeutet für die Zwecke dieser Erfindung cyclische Kohlenwasserstoffe mit 3, 4, 5, 6, 7 oder 8 Kohlenstoffatomen, wobei die Kohlenwasserstoffe gesättigt oder ungesättigt (aber nicht aromatisch), unsubstituiert oder ein- oder mehrfach substituiert sein können. In Bezug auf (Hetero-)Cycloalkyl umfasst der Begriff auch gesättigte oder ungesättigte (aber nicht aromatische) (Hetero-)Cycloalkyle, in denen ein oder zwei Kohlenstoffatome durch ein Heteroatom S, N oder O ersetzt sind. Vorteilhaft ist $C_{3-8}$-(Hetero-)Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl enthält.

Unter dem Begriff $(CH_2)_{3-6}$ ist $-CH_2-CH_2-CH_2-$, $-CH_2-CH_2-CH_2-CH_2-$, $-CH_2-CH_2-CH_2-CH_2-CH_2-$und $CH_2-CH_2-CH_2-CH_2-CH_2-CH_2-$ zu verstehen.

Der Ausdruck "Aryl" bedeutet im Sinne dieser Erfindung carbocyclische Ringsysteme mit mindestens einem aromatischen Ring, aber ohne Heteroatome in nur einem der Ringe, u.a. Phenyle, Naphthyle und Phenanthrenyle, Fluoranthenyle, Fluorenyle, Indanyle und Tetralinyle. Die Aryl-Reste können auch mit weiteren gesättigten, (partiell) ungesättigten oder aromatischen Ringsystemen kondensiert sein. Besonders vorteilhaft sind Phenyl- oder Naphthyl-Reste.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind. Der Heterocyclus kann auch Teil eines bi- oder polycyclischen Systems sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann.

Im Zusammenhang mit "Alkyl" versteht man unter dem Begriff "substituiert" im Sinne dieser Erfindung die Substitution eines oder mehrerer Wasserstoffreste durch F, Cl, Br, I, -CN, $NH_2$, NH-Alkyl, NH-Hetero-Cycloalkyl, NH-Alkyl-OH, $N(Alkyl)_2$, $N(Hetero\text{-})Cycloalkyl)_2$, $N(Alkyl\text{-}OH)_2$, $NO_2$, SH, S-Alkyl, S-(Hetero-)Cycloalkyl, S-Alkyl-OH, S-Alkyl-SH, OH, O-Alkyl, O-(Hetero-)Cycloalkyl, O-Alkyl-OH, CHO, $C(=O)C^{1-6}$-Alkyl, $C(=S)C_{1-6}$-Alkyl, C(=O)-(Hetero-)CyCloalkyl, C(=S)-(Hetero-)Cycloalkyl, $CO_2H$, $CO_2$-Alkyl, $C(=O)NH_2$, C(=O)NH-Alkyl, C(=O)NH-(Hetero-)Cycloalkyl, $C(=O)N(Alkyl)_2$, $C(=O)N(Hetero\text{-})Cycloalkyl)_2$, SO-Alkyl, $SO_2$-Alkyl, $SO_2NH_2$, $SO_3H$, oder (Hetero-)Cycloalkyl, wobei unter mehrfach substituierten Resten solche Reste zu verstehen sind, die entweder an verschiedenen oder an gleichen Atomen mehrfach, z. B. zwei- oder dreifach, substituiert sind, Beispielsweise dreifach am gleichen C-Atom wie im Falle von $CF_3$ oder $-CH_2CF_3$ oder an verschiedenen Stellen wie im Falle von $-CH(OH)-CH=CH-CHCl_2$. Die Mehrfachsubstitution kann mit dem gleichen oder mit verschiedenen Substituenten erfolgen. Ggf. kann ein Substituent auch seinerseits substituiert sein; so umfaßt -OAlkyl u.a. auch $-O-CH_2-CH_2-O-CH_2-CH_2-OH$.

In Bezug auf "Aryl", sowie "(Hetero-)Cycloalkyl" versteht man im Sinne dieser Erfindung unter "ein- oder mehrfach substituiert" die ein- oder mehrfache, z.B. zwei-, dreivier- oder fünffache, Substitution eines oder mehrerer Wasserstoffatome des Ringsystems durch F, Cl, Br, I, CN, $NH_2$, NH-Alkyl, NH-(Hetero-)Cycloalkyl, NH-Alkyl-OH, $N(Alkyl)_2$, N(Hetero-)Cycloalkyl)2, $N(Alkyl\text{-}OH)_2$, $NO_2$, SH, S-Alkyl, S-(Hetero-)Cycloalkyl, S-Alkyl-OH, S-Alkyl-SH, OH, O-Alkyl, O-(Hetero-)Cycloalkyl, O-Alkyl-OH, CHO, $C(=O)C_{1-6}$-Alkyl, $C(=S)C_{1-6}$-Alkyl, C(=O)-(Hetero-)Cycloalkyl, C(=S)-Hetero-Cycloalkyl, $CO_2H$, $CO_2$-Alkyl, $C(=O)NH_2$, C(=O)NH-Alkyl, C(=O)NH-(Hetero-)Cycloalkyl, $C(=O)N(Alkyl)_2$, $C(-O)N(Hetero\text{-})Cycloalkyl)_2$, S(O)-Alkyl, Alkyl, $SO_2NH_2$, $SO_3H$, $CF_3$, =O, =S; Alkyl, (Hetero-)Cycloalkyl, an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten.

In Bezug auf "Heteroaryl" versteht man im Sinne dieser Erfindung unter "ein-oder mehrfach substituiert" die ein- oder mehrfache, z.B. zwei-, drei- vier- oder fünffache, Substitution eines oder mehrerer Wasserstoffatome des Ringsystems durch F, Cl, Br, I, CN, $NH_2$, NH-Alkyl, NH-Aryl, NH-Heteroaryl, NH-Alkyl-Aryl, NH-Alkyl-Heteroaryl, NH-(Hetero-)Cycloalkyl, NH-Alkyl-OH, $N(Alkyl)_2$, $N(Alkyl\text{-}Aryl)_2$, N((Hetero-)Cycloalkyl)$_2$, N(Alkyl-$OH_2$, $NO_2$, SH, S-Alkyl, S-(Hetero-)Cycloalkyl, S-Aryl, S-Heteroaryl, S-Alkyl-Aryl, S-Alkyl-Heteroaryl, S-Alkyl-OH, S-Alkyl-SH, OH, O-Alkyl, O-(Hetero-)Cycloalkyl, O-Aryl, O-Heteroaryl, O-Alkyl-Aryl, O-Alkyl-Heteroaryl, O-Alkyl-OH, CHO, C(=O)$C_{1-6}$-Alkyl, C(=S)$C_{1-6}$-Alkyl-C(=O-(Hetero-)Cycloakyl, C(=S)-(Hetero-)Cycloalkyl, $CO_2H$,$CO_2$-Alkyl, C(=O)$NH_2$, C(=O)NH-Alkyl, C(=O)NH-(Hetero-)Cycloalkyl, C(=O)N(Alkyl)$_2$, C(=O)N((Hetero-)Cycloalkyl)$_2$, S(O)-Alkyl, $SO_2$-Alkyl, $SO_2NH_2$, $SO_3H$, $CF_3$, =O, =S; Alkyl, (Hetero-)Cycloalkyl, Aryl und/oder Heteroaryl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, Beispielsweise am Stickstoff, protoniert - als Kation mit mindestens einem Anion, die physiologisch - insbesondere bei Anwendung im Menschen und/oder Säugetier - verträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, $\alpha$-Liponsäure, Acetylglycin, Acetylsalicylsäure, Hippursäure und/oder Asparaginsäure. Besonders bevorzugt ist das Hydrochlorid-Salz, das Citrat und das Hemicitrat.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid und das Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, $\alpha$-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - veträglich ist. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Für eine besonders bevorzugte Ausführungsform der erfindungsgemäßen spirocyclischen Cyclohexan-Derivate gilt, dass
$R^1$ und $R^2$ unabhängig voneinander H oder $CH_3$ bedeuten, wobei $R^1$ und $R^2$ nicht gleichzeitig H bedeuten.

Für eine besonders bevorzugte Ausführungsform der erfindungsgemäßen spirocyclischen Cyclohexan-Derivate gilt, dass
$R^3$ Phenyl, Benzyl oder Phenethyl, jeweils unsubstituiert oder am Ring ein- oder mehrfach substituiert, bedeutet,

insbesondere

$R^3$ Phenyl, Benzyl, Phenethyl, 2-Fluorphenyl, 3-Fluorphenyl, 4-Fluorphenyl, 2-Chlorphenyl, 4-Chlorphenyl, 3-Chlorphenyl, 2-Bromphenyl, 3-Bromphenyl, 4-Bromphenyl, 2-Cyanophenyl, 3-Cyanophenyl, 4-Cyanophenyl, 2-Methylphenyl, 3-Methylphenyl, 4-Methylphenyl, 2-Methoxyphenyl, 3-Methoxyphenyl, 4- Methoxyphenyl, 2-Trifluormethylphenyl, 3-Trifluormethylphenyl, 3-Trifluormethylphenyl, 2-Ethylphenyl, 3-Ethylphenyl, 4-Ethylphenyl, 2-Ethoxyphenyl, 3-Ethoxyphenyl, 4-Ethoxyphenyl, 2-Hydroxyphenyl, 3-Hydroxyphenyl, 4-Hydroxyphenyl, 2,3-Dichlorphenyl, 3,4-Dichlorphenyl, 3,5-Dichlorphenyl, 2,4-Dichlorphenyl, 2,3-Difluorphenyl, 3,4-Difluorphenyl, 3,5-Difluorphenyl, 2,4-Difluorphenyl2-Fluor-3-chlorphenyl, 2-Chlor-3-fluorphenyl, 2-Chlor-4-fluorphenyl, 2-Fluor-4-chlorphenyl, 4-Fluor-3-chlorphenyl, 4-Fluor-3-methylphenyl, 4-tert.-Butylphenyl, 4-Fluor-3-chlorphenyl, 4-Brom-3-fluorphenyl, 3,5-Bis(trifluormethyl)phenyl, 4-Chlor-2-trifluormethylphenyl, 2-Methoxy-5-methylphenyl, 5-Chlor-2-methoxyphenyl, 4-Phenoxyphenyl, 2-Methylthiophenyl, 3-Methylthiophenyl, 4-Methylthiophenyl, 5-Fluor-2-methoxyphenyl, 4-Chlor-3-trifluormethyl oder 4-Brom-2-methylphenyl ganz besonders bevorzugt Phenyl, Benzyl, Phenethyl, 4-Fluorphenyl und 3-Fluorphenyl. Weiterhin besonders bevorzugt sind Verbindungen, bei denen W $NR^4$ bedeutet, wobei $R^4$ für H, $CH_3$, $C_2H_5$, oder Phenyl, steht, und X NH, oder $NCOR^{12}$ bedeutet.

Für eine ganz besonders bevorzugte Ausführungsform der erfindungsgemäßen spirocyclischen Cyclohexan-Derivate gilt, dass
$R^1$ und $R^2$ unabhängig voneinander H oder $CH_3$, insbesondere $CH_3$ bedeuten,
$R^3$ Phenyl bedeutet.

Referenz-Verbindungen sind aus der Gruppe:

1,1-(3-Dimethylamino-3-phenylpentamethylen)-1,3,4,9-tetrahydropyrano[3,4-b]indol Hydrochlorid
1,1-(3-Dimethylamino-3-phenylpentamethylen)-1,3,4,9-tetrahydropyrano[3,4-b]indol Hemicitrat
1,1-(3-Dimethylamino-3-phenylpentamethylen)-1,3,4,9-tetrahydro-2-thia-9-azafluoren Hemicitrat
1,1-(3-Dimethylamino-3-phenylpentamethylen)-1,3,4,9-tetrahydro-2-thia-9-azafluoren Citrat
1,1-(3-Dimethylamino-3-phenylpentamethylen)-3,4-dihydro-1H-2-oxa-9-thiafluoren L-Tartrat
1,1-(3-Dimethylamino-3-(4-fluorphenyl)pentamethylen)-3,4-dihydro-1 H-2-oxa-9-thiafluoren Triflat
1,1-(3-Dimethylamino-3-phenylpentamethylen)-3,4-dihydro-1H-2,9-dioxafluoren Hemicitrat
1,1-(3-Dimethylamino-3-phenylpentamethylen)-3,4-dihydro-1 H-2,9-diazafluoren Dihydrochlorid
2-Acetyl-1,1-(3-dimethylamino-3-phenylpentamethylen)-3,4-dihydro-1 H-2,9-diazafluoren Hydrochlorid
1,1-(3-Dimethylamino-3-phenylpentamethylen)-6-methoxy-1,3,4,9-tetrahydropyrano[3,4-b]indol Hydrochlorid
1,1-(3-Dimethylamino-3-phenylpentamethylen)-3-methyl-1,3,4,9-tetrahydropyrano[3,4-b]indol Citrat
6-Brom-1,1-(3-dimethylamino-3-phenylpentamethylen)-3-methyl-1,3,4,9-tetrahydropyrano[3,4-b]indol Hemicitrat
1,1-(3-Dimethylamino-3-phenylpentamethylen)-3-methyl-6-nitro-1,3,4,9-tetrahydropyrano[3,4-b]indol Citrat
6-Chlor-1,1-(3-Dimethylamino-3-phenylpentamethylen)-3-methyl-1,3,4,9-tetrahydropyrano[3,4-b]indol Citrat
3,9-Dimethyl-1,1-(3-dimethylamino-3-phenylpentamethylen)-1,3,4,9-tetrahydropyrano[3,4-b]indol Citrat
1,1-(3-Dimethylamino-3-(4-fluorphenyl)pentamethylen)-1,3,4,9-tetra-hydro-pyrano[3,4-b]indol Hemicitrat
1,1-(3-Dimethylamino-3-(3-fluorphenyl)pentamethylen)-1,3,4,9-tetra-hydro-pyrano[3,4-b]indol Hemicitrat
1,1-(3-Dimethylamino-3-phenylpentamethylen)-6-fluor-1,3,4,9-tetrahydropyrano[3,4-b]indol Hemicitrat
1,1-(3-Dimethylamino-3-phenylpentamethylen)-6-methyl-1,3,4,9-tetrahydropyrano[3,4-b]indol Hemicitrat
1,1-(3-Dimethylamino-3-phenylpentamethylen)-9-phenyl-1,3,4,9-tetrahydropyrano[3,4-b]indol Citrat
1,1-(3-Methylamino-3-phenylpentamethylen)-1,3,4,9-tetra-hydro-pyrano-[3,4-b]indol Hemicitrat
1,1-(3-Dimethylamino-3-phenylpentamethylen)-6-methyl-3,4-dihydro-1 H-2,9-diazafluoren Citrat
2-Acetyl-1,1-(3-dimethylamino-3-phenylpentamethylen)-6-methyl-3,4-dihydro-1H-2,9-diazafluoren Citrat
1,1-(3-Dimethylamino-3-phenylpentamethylen)-7-fluor-3,4-dihydro-1H-2,9-diazafluoren Citrat
2-Acetyl-1,1-(3-dimethylamino-3-phenylpentamethylen)-7-fluor-3,4-dihydro-1H-2,9-diazafluoren Citrat
1,1-(3-Dimethylamino-3-phenylpentamethylen)-3-methyl-3,4-dihydro-1H-2,9-diazafluoren Citrat
1,1-(3-Dimethylamino-3-phenylpentamethylen)-6-fluor-3,4-dihydro-1H-2,9-diazafluoren Dihydrochlorid
1,1-(3-Dimethylamino-3-phenylpentamethylen)-6-fluor-3-methyl-1,3,4,9-tetra-hydro-pyrano-[3,4-b]indol Hemicitrat
3,6-Dimethyl-1,1-(3-dimethylamino-3-phenylpentamethylen)-1,3,4,9-tetra-hydro-pyrano-[3,4-b]indol Hemicitrat
3,6-Dimethyl-1,1-(3-dimethylamino-3-phenylpentamethylen)-1,3,4,9-tetra-hydro-pyrano-[3,4-b]indol Citrat
1,1-(3-Dimethylamino-3-phenylpentamethylen)-3-methyl-9-phenyl-1,3,4,9-tetrahydropyrano[3,4-b]indol Citrat
1,1-(3-Dimethylamino-3-(4-fluorphenyl)pentamethylen)-1,3,4,9-tetra-hydro-2-thia-9-azafluoren Methansulfonat
1,1-(3-Dimethylamino-3-(3-fluorphenyl)pentamethylen)-1,3,4,9-tetra-hydro-2-thia-9-azafluoren Methansulfonat
1,1-(3-Dimethylamino-3-phenylpentamethylen)-3,4-dihydro-1H-9-oxa-2-thiafluoren Citrat 1,1-(3-Dimethylamino-3-phenylpentamethylen)-1,2,3,4-tetrahydro-benzo[4,5]furo[2,3-c]pyridin Citrat
6,6-(3-Dimethylamino-3-phenylpentamethylen)-1,2,3,4,4a,6,7,11c-octahydro-5-oxa-7-azabenzo[c]fluoren Citrat
1,1-(3-Dimethylamino-3-phenylpentamethylen)-6-brom-1,3,4,9-tetrahydropyrano[3,4-b]indol Hemicitrat

1,1-(3-Dimethylamino-3-phenylpentamethylen)-1,3,4,9-tetra-hydro-pyrano[3,4-b]indol-6-ol Citrat
(3S)-1,1-(3-Dimethylamino-3-phenylpentamethylen)-3,4-dihydro-3-methoxycarbonyl-1 H-2,9-diazafluoren Citrat
(3S)-1,1-(3-Dimethylamino-3-phenylpentamethylen)-3,4-dihydro-1H-2,9-diazafluoren-3-methanol Citrat
1,1-(3-Dimethylamino-3-phenylethyl-pentamethylen)-3,4-dihydro-1H-2,9-diazafluoren 1,1-(3-Methylamino-3-phenylpentamethylen)-6-fluor-1,3,4,9-tetrahydropyrano[3,4-b]indol Hemicitrat
1,1-(3-Dimethylamino-3-(4-fluorphenyl)pentamethylen)-3,4-dihydro-1H-2,9-dithiafluoren Methansulfonat
1,1-(3-Dimethylamino-3-phenylpentamethylen)-3,4-dihydro-1H-2,9-dithiafluoren Citrat 1,1-(3-Dimethylamino-3-phenylpentamethylen)-1,3,4,9-tetra-hydro-2-thia-9-aza-fluoren 2-oxid Citrat
1,1-(3-Dimethylamino-3-benzylpentamethylen)-3,4-dihydro-1H-2,9-dithiafluoren gegebenenfalls auch als Gemisch.

Die erfindungsgemäßen Substanzen wirken Beispielsweise auf den im Zusammenhang mit verschiedenen Erkrankungen relevanten ORL1-Rezeptor, sodass sie sich als pharmazeutischer Wirkstoff in einem Arzneimittel eignen. Ein weiterer Gegenstand der Erfindung sind daher Arzneimittel enthaltend wenigstens ein erfindungsgemäßes spirocyclisches Cyclohexan-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen spirocyclischen Cyclohexan-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße spirocyclische Cyclohexan-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen spirocyclischen Cyclohexan-Derivate verzögert freisetzen. Die erfindungsgemäßen spirocyclischen Cyclohexan-Derivate können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,001 bis 0,5 mg/kg wenigstens eines erfindungsgemäßen spirocyclischen Cyclohexan-Derivats appliziert.

Für alle vorstehenden Formen der erfindungsgemäßen Arzneimittel ist es besonders bevorzugt, wenn das Arzneimittel neben wenigstens einem spirocyclischen Cyclohexan-Derivat noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittel liegt ein enthaltenes erfindungsgemäßes spirocyclisches Cyclohexan-Derivat als reines Diastereomer und/oder Enantiomer, als Razemat oder als nicht-äquimolare oder äquimolare Mischung der Diastereomere und/oder Enantiomere vor.

Wie in der Einleitung am Stand der Technik abzulesen, wurde der ORL1-Rezeptor insbesondere im Schmerzgeschehen identifiziert. Entsprechend können erfindungsgemäße spirocyclische Cyclohexan-Derivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung eines erfindungsgemäßen spirocyclischen Cyclohexan-Derivats zur Herstellung eines Arzneimittels zur Behandlung von Schmerz, insbesondere von akutem, viszeralem, neuropathischem oder chronischem Schmerz.

Ein weiterer Gegenstand der Erfindung ist die Verwendung eines erfindungsgemäßen spirocyclischen Cyclohexan-Derivats zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur

Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn ein verwendetes spirocyclisches Cyclohexan-Derivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Herstellung eines Arzneimittels zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen spirocyclischen Cyclohexan-Derivats, oder eines erfindungsgemäßen Arzneimittels.

Ein weiterer Gegenstand der Erfindung ist ein Verfahren zur Herstellung der erfindungsgemäßen spirocyclischen Cyclohexan-Derivate wie in der folgenden Beschreibung und Referenz-Beispielen ausgeführt. Insbesondere geeignet ist dabei ein, im folgenden Hauptverfahren genanntes, Verfahren zur Herstellung eines erfindungsgemäßen spirocyclischen Cyclohexan-Derivats mit folgenden Schritten,

wobei X, W, $R^1$, $R^2$, $R^3$, $R^5$, $R^6$, $R^7$, $R^8$, $R^9$ und $R^{10}$ die für erfindungsgemäße Verbindungen gemäß Formel I angegebene Bedeutung haben,

und

$R^{01}$ und $R^{02}$ die für erfindungsgemäße Verbindungen gemäß Formel I für $R^1$ und $R^2$ angegebene Bedeutung haben und zusätzlich unabhängig voneinander für eine Schutzgruppe stehen können:

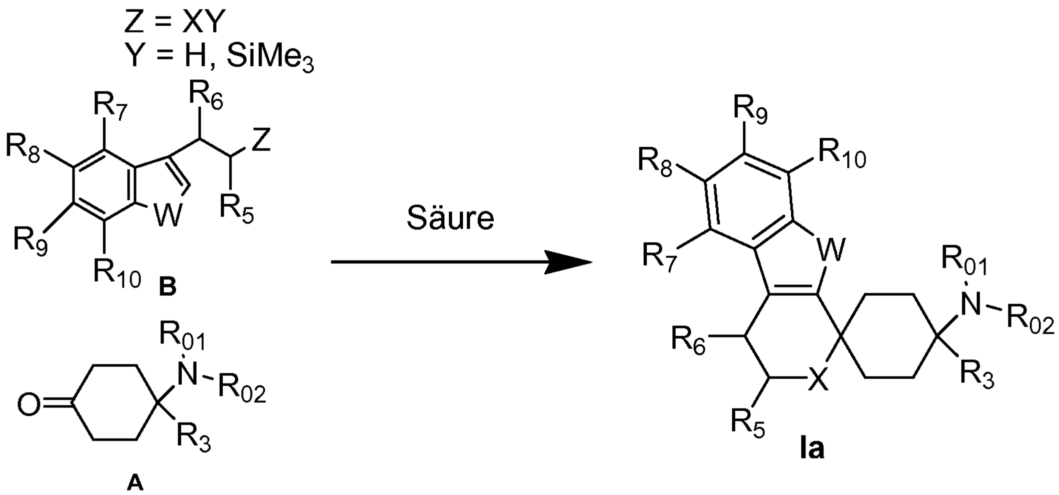


Zur Herstellung der Verbindungen der allgemeinen Formel Ia werden Ketone der allgemeinen Formel A mit Heteroaromaten der allgemeinen Formel B unter Zugabe von Säure oder deren Trimethylsilylester, Beispielsweise Trifluormethansulfonsäuretrimethylsilylester, Essigsäure, Phosphorsäure, Methansulfonsäure oder Trifluoressigsäure in einem geeigneten Lösungsmittel, Beispielsweise Dichlorethan, Dichlormethan, Chloroform, Acetonitril, Diethylether oder Nitromethan, umgesetzt.

Die Herstellung des Keton-Intermediats A erfolgt insbesondere nach folgender Vorschrift:

ein mit den Gruppen S1 und S2,die für Schutzgruppen stehen - Beispielsweise substituiertes oder unsubstituiertes Alkyl, insbesondere $(CH_2)n$ mit n = 2-4 - geschütztes Cyclohexan-1,4-dion gemäß Formel II wird in Gegenwart einer Verbindung der Formel $HNR^{01}R^{02}$ mit einem Cyanid, vorzugsweise Kaliumcyanid oder TMSCN, zu einem geschützten N-substituierten 1-Amino-4-oxo-cyclohexancarbonitrilderivat gemäß Formel III umgesetzt;

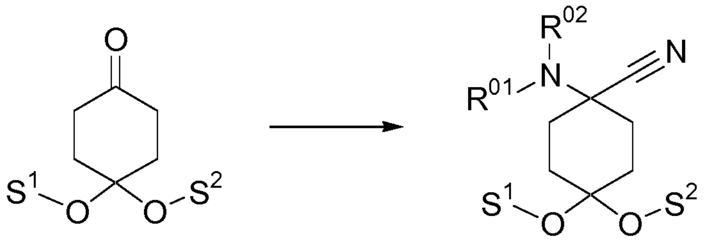

II III

gegebenenfalls wird anschließend in beliebiger Reihenfolge und gegebenenfalls wiederholt acyliert, alkyliert oder sulfoniert und/oder bei Verbindungen mit $R^{01}$ und/oder $R^{02}$ = Schutzgruppe, mindestens einmal eine Schutzgrupe abgespalten und gegebenfalls acyliert, alkyliert oder sulfoniert und/oder bei einer Verbindungen mit $R^{01}$ und/oder $R^{02}$ = H mindestens einmal eine Schutzgruppe eingeführt und gegebenfalls acyliert, alkyliert oder sulfoniert,

das Aminonitril gemäß Formel III wird mit metallorganischen Reagenzien, bevorzugt Grignard- oder Organolithiumreagenzien, der Formel Metall-$R^3$ umgesetzt, so daß eine Verbindung gemäß Formel IV entsteht;

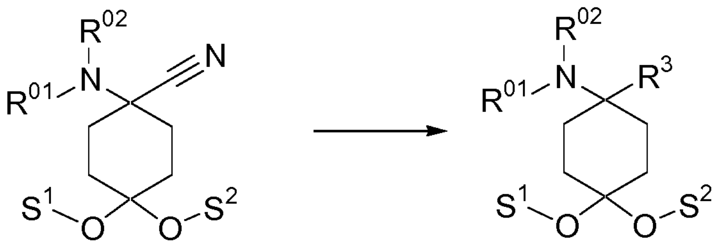

III IV

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ = Schutzgruppe, mindestens einmal eine Schutzgrupe abgespalten und gegebenfalls acyliert, alkyliert oder sulfoniert und/oder bei einer Verbindungen mit $R^{01}$ und/oder $R^{02}$ = H mindestens einmal eine Schutzgruppe eingeführt und gegebenfalls acyliert, alkyliert oder sulfoniert,

an der Verbindung gemäß Formel IV werden die Schutzgruppen S1 und S2 abgespalten, so daß ein 4-substituiertes 4-Aminocyclohexanonderivat gemäß Formel A entsteht;

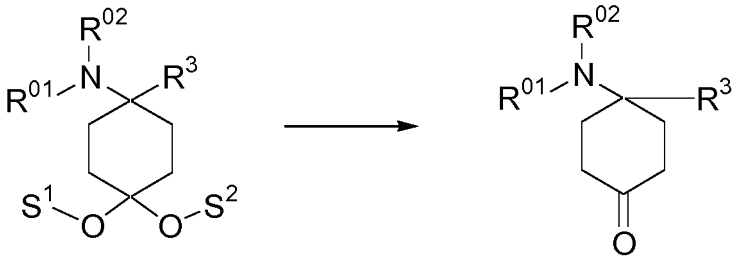

IV A

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ = Schutzgruppe, mindestens einmal eine Schutzgrupe abgespalten und gegebenfalls acyliert, alkyliert oder sulfoniert und/oder bei einer Verbindungen mit $R^{01}$ und/oder $R^{02}$ = H mindestens einmal eine Schutzgruppe eingeführt und gegebenfalls acyliert, alkyliert oder sulfoniert,

wobei X, W, $R^3$, $R^5$, $R^6$, $R^7$, $R^8$, $R^9$ und $R^{10}$ die für erfindungsgemäße Verbindungen gemäß Formel I angegebene Bedeutung haben,

und

$R^{01}$ und $R^{02}$ die für erfindungsgemäße Verbindungen gemäß Formel I für $R^1$ und $R^2$ angegebene Bedeutung haben und zusätzlich unabhängig voneinander für eine Schutzgruppe stehen können:

Alternativ kann die Herstellung auch nach folgendem Schema erfolgen, wobei X, W, $R^3$, $R^5$, $R^6$, $R^7$, $R^8$, $R^9$ und $R^{10}$ die für erfindungsgemäße Verbindungen gemäß Formel I angegebene Bedeutung haben,

und

$R^{01}$ und $R^{02}$ die für erfindungsgemäße Verbindungen gemäß Formel I für $R^1$ und $R^2$ angegebene Bedeutung haben und zusätzlich unabhängig voneinander für eine Schutzgruppe stehen können.

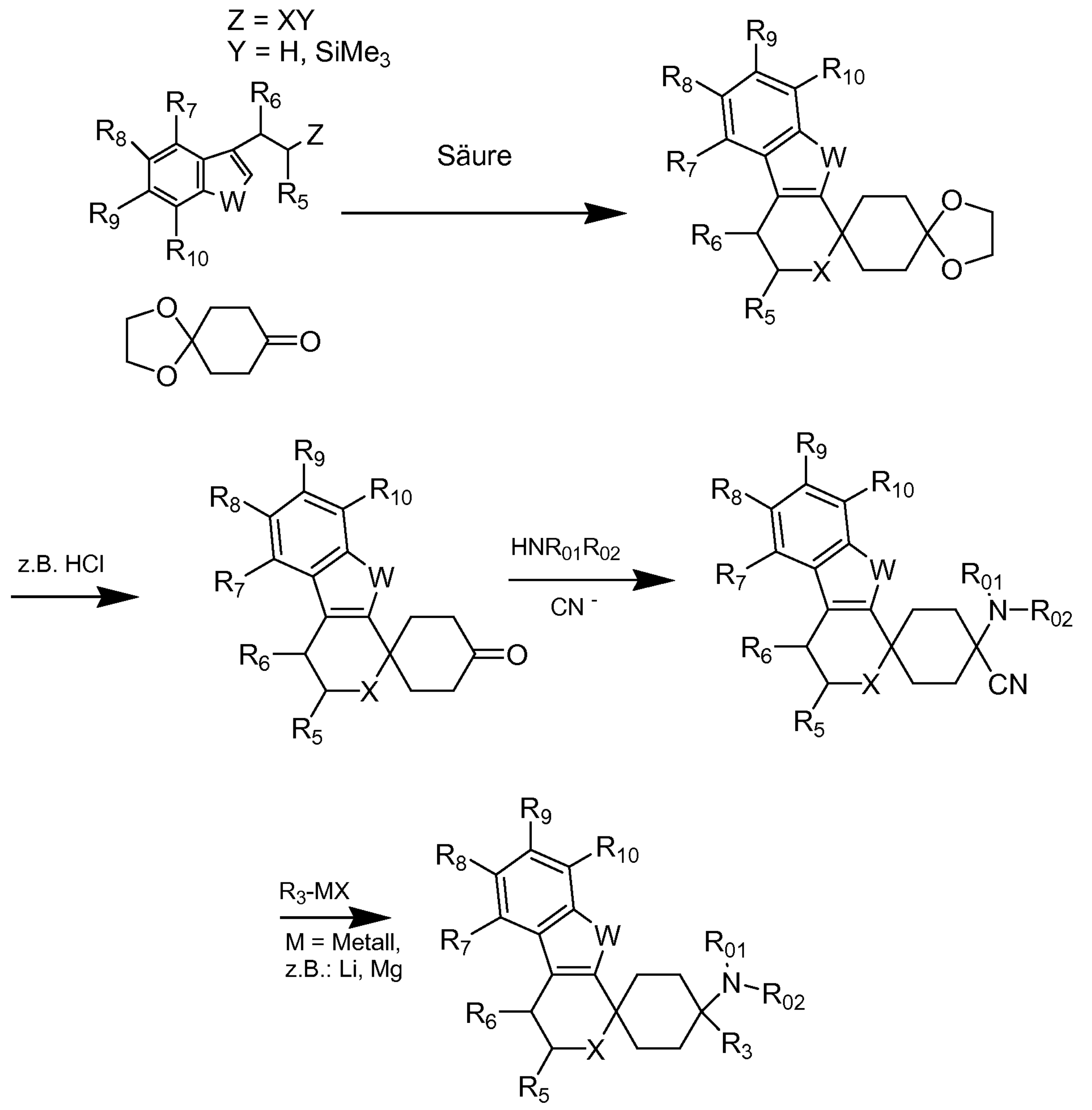


Spirocyclische Cyclohexanderivate der allgemeinen Formel I, bei denen X $NR^{17}$ und $R^{17}$ $COR^{12}$ oder $SO_2R^{12}$ bedeutet, können durch die Umsetzung von spirocyclischen Cyclohexanderivaten der allgemeinen Formel I, bei denen X NH bedeutet, durch Umsetzung mit einem Anhydrid oder einem Säurechlorid unter Zugabe einer Base, Beispielsweise Triethylamin, erhalten werden. Vorzugsweise findet diese Reaktion unter Mikrowelleneinstrahlung statt.

Eine Isolierung der erfindungsgemäßen Verbindungen durch Säulenchromatographie mit Kieselgel als stationärer Phase und Ethylacetat, Methanol, Ethanol, Gemischen aus Ethylacetat und Methanol oder Ethanol, oder Gemischen aus Ethylacetat und Diethylether als Laufmittel führt zu einer Auftrennung der unterschiedlich polaren Diastereoisomeren. Diese wurden aufgrund ihrer Laufzeit bei der Trennung als "unpolarstes Diastereoisomer" (kürzeste Laufzeit) bis "polarstes Diastereoisomer" (längste Laufzeit) charakterisiert.

Referenz-Beispiele

Die folgenden Referenz-Beispiele dienen zur näheren Erläuterung der Erfindung, schränken aber den allgemeinen Erfindungsgedanken nicht ein.

Die Ausbeuten der hergestellten Verbindungen sind nicht optimiert.

Alle Temperaturen sind unkorrigiert.

Die Angabe "Ether" bedeutet Diethylether, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur, "abs." absolut (wasserfrei), ,"rac." racemisch , "konz." konzentriert, "min" Minuten, "h" Stunden, "d" Tage, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/Volumen angegeben.

Die im folgenden eingesetzten Verbindungen waren entweder kommerziell erhältlich, oder ihre Herstellung ist aus dem Stand der Technik bekannt oder in für den Fachmann offensichtlicher Weise aus dem Stand der Technik abgeleitet worden. Insbesondere relevant sind hierfür die folgenden Zitate: Jirkovsky et al., J. Heterocycl. Chem., 12, 1975, 937-940; Campaigne et al., J. Heterocycl. Chem., 2, 1965, 231-235; Efange et al., J. Med. Chem., 41, 1998, 4486 - 4491; Ellingboe et al., J. Med. Chem., 35, 1992, 1176-1183; Pearson et al., Aust. J. Chem., 44, 1991, 907-917; Yokohama et al., Chem. Pharm. Bull., 40, 1992, 2391-2398; Beck et al., J. Chem. Soc. Perkin 1, 1992, 813-822; Shinada et al., Tetrahedron Lett., 39, 1996, 7099-7102; Garden et al., Tetrahedron, 58, 2002, 8399-8412; Lednicer et al., J. Med. Chem., 23, 1980, 424-430.

Referenz-Beispiel 1: 1,1-(3-Dimethylamino-3-phenylpentamethylen)-1,3,4,9-tetrahydropyrano[3,4-b]indol Hydrochlorid, unpolareres Diastereoisomer UND
Referenz-Beispiel 2: 1,1-(3-Dimethylamino-3-phenylpentamethylen)-1,3,4,9-tetrahydropyrano[3,4-b]indol Hydrochlorid, polareres Diastereoisomer UND
Referenz-Beispiel 3: 1,1-(3-Dimethylamino-3-phenylpentamethylen)-1,3,4,9-tetrahydropyrano[3,4-b]indol Hemicitrat, unpolareres Diastereoisomer

Methode A:

Unter Argon wurde zu einer Lösung von 4-Dimethylamino-4-phenylcyclohexanon (1,1 g, 5,07 mmol) und 3-(2-Trimethylsilanyloxyethyl)-1 H-indol (1,4 g, 6,01 mmol) in DCM (30 ml) bei -78 °C innerhalb von 5 min unter Rühren Trifluormethansulfonsäuretrimethylsilylester (1 ml, 5 mmol) gegeben. Der Ansatz wurde 1 h bei -78 °C gerührt. Anschließend wurde der Ansatz während eines Zeitraums von 4 h auf RT gebracht und weitere 10 h bei RT gerührt. Zur Aufarbeitung wurde das Reaktionsgemisch mit 1 M NaOH (30 ml) versetzt und 30 min gerührt. Die organische Phase wurde abgetrennt und die verbleibende wässrige Phase mit DCM (2 x 30 ml) extrahiert. Die vereinigten organischen Phasen wurden mit 1 M NaOH (1 x 30 ml) und Wasser (2 x 30 ml) gewaschen und über Natriumsulfat getrocknet. Nach Abdestillieren des Lösungsmittels wurde ein gelber Feststoff erhalten, der mit EE gewaschen wurde. Aus dem verbleibenden Rohprodukt wurde nach Umkristallisation aus Toluol das unpolarere Isomer von 1,1-(3-Dimethylamino-3-phenylpentamethylen)-1,3,4,9-tetrahydropyrano[3,4-b]indol in einer Ausbeute von 0,8 g isoliert, das einen Smp. von 279-284 °C aufwies. Die verbliebene Mutterlauge und die EE-Waschlösung wurden eingeengt. Mittels einer säulenchromatographischen Reinigung an Kieselgel zunächst mit EE/Ethanol (Volumenverhältnis 8 : 2) dann mit EE/Ethanol (Volumenverhältnis 1 : 1) konnte die bereits isolierte unpolarere Verbindung (150 mg) sowie ein weiteres, polareres Isomer abgetrennt werden. Das polarere Produkt wurde nach Umkristallisation aus Toluol in einer Ausbeute von 60 mg mit einem Smp. von 230-235 °C erhalten.

Methode B:

Tryptophol (322 mg, 2,0 mmol) und 4-Dimethylamino-4-phenylcyclohexanon (435 mg, 2,0 mmol) wurden unter Rühren und Kühlung mit Eis unter Argon in einem Gemisch aus Essigsäure (4 ml) und 85 massenproz. Phosphorsäure

(1 ml) gelöst. Das Gemisch wurde über Nacht bei RT gerührt. Der entstandene Feststoff wurde abgesaugt und mit Methanol gewaschen. Es wurde nur das polarere der zwei möglichen Diastereoisomeren als weißer Feststoff in einer Ausbeute von 600 mg mit einem Smp. von 280-284 °C erhalten.

Methode C:

Unter Argon wurden 4-Dimethylamino-4-phenylcyclohexanon (868 mg, 4 mmol) und Tryptophol (644 mg, 4 mmol) in abs. DCM (30 ml) vorgelegt. Zu der Lösung wurde Triethylamin (0,07 ml, 0,5 mmol) gegeben. Anschließend erfolgte eine sehr schnelle Zugabe von Trifluormethansulfonsäuretrimethylsilylester (0,9 ml, 4,7 mmol). Der Ansatz wurde 20 h bei RT gerührt. Zur Aufarbeitung wurde das Reaktionsgemisch mit 1 M NaOH (50 ml) versetzt und 30 min gerührt. Die organische Phase wurde abgetrennt und die verbleibende wässrige Phase mit DCM (3 × 30 ml) extrahiert. Die vereinigten organischen Phasen wurden mit Wasser (2 × 30 ml) gewaschen und über Natriumsulfat getrocknet. Der nach Abdestillieren des Lösungsmittels erhaltene weitgehend feste Rückstand wurde mit Methanol (40 ml) versetzt, erwärmt und 15 h gerührt. Bei dem suspendierten Feststoff handelt es sich um das unpolarere Diastereoisomer. Das polarere Diastereoisomer befand sich in der methanolischen Lösung. Das unpolarere Isomer wurde in einer Ausbeute von 1,20 g mit einem Smp. von 278-282 °C erhalten. Umkristallisation aus Isopropanol lieferte watteartige Kristalle, die ein Äquivalent Isopropanol enthielten. Der Smp. des umkristallisierten Produktes lag bei 289-293 °C.

Methode D:

Unter Argon wurden 4-Dimethylamino-4-phenylcyclohexanon (434 mg, 2 mmol) und Tryptophol (322 mg, 4 mmol) in abs. DCM (20 ml) vorgelegt. Anschließend erfolgte eine sehr schnelle Zugabe von Trifluormethansulfonsäuretrimethylsilylester (0,4 ml, 2,07 mmol). Der Ansatz wurde 18 h bei RT gerührt. Zur Aufarbeitung wurde das Reaktionsgemisch mit 1 M NaOH (20 ml) versetzt und 30 min gerührt. Die organische Phase wurde abgetrennt und die verbleibende wässrige Phase mit DCM (3 × 30 ml) extrahiert. Die vereinigten organischen Phasen wurden mit Wasser (2 × 30 ml) gewaschen und über Natriumsulfat getrocknet. Der nach Abdestillieren des Lösungsmittels erhaltene weitgehend feste Rückstand wurde mit Methanol (20 ml) versetzt, erwärmt und 15 h gerührt. Bei dem suspendierten Feststoff handelt es sich um das unpolarere Diastereoisomer. Das polarere Produkt befand sich in der methanolischen Lösung. Das unpolarere Diastereoisomer wurde in einer Ausbeute von 571 mg mit einem Smp. von 284-286 °C erhalten.

Methode E:

Unter Argon wurden 4-Dimethylamino-4-phenylcyclohexanon (651 mg, 3 mmol) und 3-(2-Trimethylsilanyloxyethyl)-1 H-indol (699 mg, 3 mmol) in abs. DCM (20 ml) gelöst. Anschließend erfolgte eine sehr schnelle Zugabe von Trifluormethansulfonsäure (0,28 ml, 3,16 mmol). Der Ansatz wurde 20 h bei RT gerührt. Zur Aufarbeitung wurde das Reaktionsgemisch mit 1 M NaOH (20 ml) versetzt und 30 min gerührt. Die organische Phase wurde abgetrennt und die verbleibende wässrige Phase mit DCM (3 × 30 ml) extrahiert. Die vereinigten organischen Phasen wurden mit Wasser (2 × 30 ml) gewaschen und über Natriumsulfat getrocknet. Bei dem nach Abdestillieren des Lösungsmittels erhaltenen festen Rückstand handelte es sich um das unpolarere Diastereoisomer (800 mg).

Referenz-Beispiel 1 - Hydrochlorid des unpolareren Diastereoisomers: Zur Herstellung des Hydrochlorids wurde das unpolarere Diastereoisomer von 1,1-(3-Dimethylamino-3-phenylpentamethylen)-1,3,4,9-tetrahydropyrano[3,4-b]indol (500 mg, 1,38 mmol) in 2-Butanon (40 ml) gelöst, mit Chlortrimethylsilan (250 µl, 1,98 mmol) versetzt und 3 h bei RT gerührt. Der entstandene Feststoff wurde abgesaugt. Das Hydrochlorid des unpolareren Diastereoisomers konnte so in einer Ausbeute von 420 mg als weißer Feststoff mit einem Smp. von 278-280 °C erhalten werden.

Zu Referenz-Beispiel 1 wurden Untersuchungen zur Herz-Kreislaufverträglichkeit durchgeführt. Es zeigte sich, dass Referenz-Beispiel 1 im Vergleich zu den beiden klinisch eingesetzten Opioiden Fentanyl und Sufentanil Vorteile bezüglich der Herz-Kreislaufverträglichkeit aufweist.

Referenz-Beispiel 2 - Hydrochlorid des polareren Diastereoisomers:

Zu einer Lösung des polareren Diastereoisomers von 1,1-(3-Dimethylamino-3-phenyl-pentamethylen)-1,3,4,9-tetrahydropyrano[3,4-b]indol (50 mg, 0,138 mmol) in 2-Butanon (10 ml) wurde Chlortrimethylsilan (25 µl, 0,198 mmol) gegeben. Nach einer Reaktionszeit von 2 h konnte das ausgefallene Hydrochlorid des polareren Diastereoisomers in einer Ausbeute von 36 mg mit einem Smp. von 271-272 °C isoliert werden.

Referenz-Beispiel 3 - Hemicitrat des unpolareren Diastereoisomers:

Zur Herstellung des Hemicitrates wurde das unpolarere Diastereoisomer von 1,1-(3-Di-methylamino-3-phenylpentamethylen)-1,3,4,9-tetrahydropyrano[3,4-b]indol (1,2 g, 3,33 mmol) in heißem Ethanol (350 ml) gelöst und mit einer ebenfalls heißen Lösung von Citronensäure (1,2 g, 6,25 mmol) in Ethanol (30 ml) versetzt. Nach Abkühlung wurde der Ansatz 4 h auf ca. 10 °C belassen. Der entstandene Feststoff wurde abgesaugt. Das Hemicitrat konnte so in einer Ausbeute von 1,05 g als weißer Feststoff mit einem Smp. von 259-265 °C erhalten werden.

Referenz-Beispiel 4: 1,1-(3-Dimethylamino-3-phenylpentamethylen)-1,3,4,9-tetrahydro-2-thia-9-azafluoren Hemicitrat, unpolareres Diastereoisomer UND

Referenz-Beispiel 5: 1,1-(3-Dimethylamino-3-phenylpentamethylen)-1,3,4,9-tetrahydro-2-thia-9-azafluoren Citrat, polareres Diastereoisomer

Methode A:

Unter Argon wurden 4-Dimethylamino-4-phenylcyclohexanon (326 mg, 1,5 mmol) und 2-(1H-Indol-3-yl)ethanthiol (266 mg, 1,5 mmol) in abs. DCM (10 ml) vorgelegt. Anschließend erfolgte die Zugabe des Methansulfonsäuretrimethylsilylesters (254 µl, 1,65 mmol). Der Ansatz wurde 4 d bei RT gerührt. Zur Aufarbeitung wurde das ausgefallene Methansulfonat abgesaugt und mit DCM (3 × 0,5 ml) gewaschen. Das Methansulfonat wurde in einer Ausbeute von 306 mg als weißer Feststoff mit einem Smp. von 243-245 °C erhalten. - Die DCM-Phase wurde alkalisch aufgearbeitet (1 M NaOH, 30 ml, 1h stark rühren), die Phasen getrennt und die DCM-Phase eingeengt. Der Rückstand wurde mit abs. Ethanol (10 ml) überschichtet und 30 min unter Rückfluss gerührt. Nach mehrstündigem Stehen bei RT wurde der Niederschlag abgesaugt, mit Ethanol (4 × 1ml) gewaschen und dann getrocknet. Es wurde ein Gemisch des unpolareren und polareren Diastereoisomers von 1,1-(3-Dimethylamino-3-phenylpentamethylen)-1,3,4,9-tetrahydro-2-thia-9-azafluoren in einer Ausbeute von 182 mg erhalten.

Methode B:

Unter Argon wurden 4-Dimethylamino-4-phenylcyclohexanon (386,5 mg, 1,78 mmol) und 2-(1H-Indol-3-yl)ethanthiol (315 mg, 1,78 mmol) in Eisessig (8 ml) gelöst. Die Mischung wurde auf 4 °C abgekühlt und 85 massenproz. Phosphorsäure (2 ml) zugetropft. Danach wurde der Ansatz 20 h bei RT gerührt. Zur Aufarbeitung wurde die entstandene Suspension auf 5 °C abgekühlt, mit 1 M NaOH (60 ml) versetzt und 1 h bei RT gerührt. Nach Zugabe von DCM (50 ml) wurde 2 h bei RT gerührt. Die klaren Phasen wurden getrennt. Die wässrige Phase wurde mit DCM (3 × 10 ml) extrahiert. Die vereinigten organischen Phasen wurden über Natriumsulfat getrocknet und das DCM abdestilliert. Eines der beiden Diastereoisomeren von 1,1-(3-Dimethylamino-3-phenylpentamethylen)-1,3,4,9-tetrahydro-2-thia-9-azafluoren wurde so als weißer Feststoff in einer Ausbeute von 603 mg mit einem Smp. von 236-238 °C erhalten.

Referenz-Beispiel 4 - Hemicitrat des unpolareren Diastereoisomers:

Das nach Methode A erhaltene Diastereoisomerengemisch (172 mg, 0,457 mmol) wurde in heißem Ethanol (130 ml) gelöst, mit Citronensäure (88,6 mg, 0,461 mmol) versetzt und 10 min bei 65 °C gerührt. Nach dem Abkühlung auf RT wurde der Ansatz 20 h gerührt. Der entstandene Feststoff wurde abgesaugt, mit kaltem Ethanol (2 × 0,5 ml) gewaschen und anschließend getrocknet. Es wurden 85 mg des Hemicitrats des unpolareren Diastereoisomers von 1,1-(3-Dimethylamino-3-phenylpentamethylen)-1,3,4,9-tetrahydro-2-thia-9-azafluoren erhalten (Smp. 241- 243 °C).

Referenz-Beispiel 5 - Citrat des polareren Diastereoisomers:

Die nach Referenz-Beispiel 4 erhaltene ethanolische Mutterlauge wurde auf 25 ml Lösung reduziert, mit 20 ml $Et_2O$ versetzt und bei RT 1 h gerührt. Der Niederschlag wurde abgesaugt, mit $Et_2O$ (3 × 2 ml) gewaschen und getrocknet (62 mg, Smp. 165- 169 °C, Diastereoisomerengemisch). Aus der Mutterlauge wurden durch Zugabe von weiteren 50 ml Diethylether nochmals ein weißer Feststoff erhalten. Auch dieser wurde abgesaugt, mit $Et_2O$ (3 × 2 ml) gewaschen und getrocknet. Es wurden 32 mg des Citrats des polareren Diastereoisomers von 1,1-(3-Dimethylamino-3-phenylpentamethylen)-1,3,4,9-tetrahydro-2-thia-9-azafluoren erhalten (Smp. 155-160 °C).

Referenz-Beispiel 6: 1,1-(3-Dimethylamino-3-phenylpentamethylen)-3,4-dihydro-1H-2-oxa-9-thiafluoren L-Tartrat

Methode A:

Unter Argon wurden 4-Dimethylamino-4-phenylcyclohexanon (217 mg, 1 mmol) und 2-(Benzo[b]thiophen-3-yl)ethanol (178 mg, 1 mmol) in abs. DCM (10 ml) vorgelegt. Anschließend erfolgte die Zugabe von Trifluormethansulfonsäuretrimethylsilylester (245 $\mu$l, 1,1 mmol). Der Ansatz wurde 24 h bei RT gerührt. Nach dieser Zeit war das Reaktionsgemisch hellbraun gefärbt und klar. Zur Aufarbeitung wurden 10 g Eis zugesetzt und die wässrige Phase wurde mit 1 M NaOH auf pH 11 eingestellt. Die Phasen wurden getrennt. Die wässrige Phase wurde mit DCM (3 $\times$ 10 ml) extrahiert. Die organischen Phasen wurden vereinigt, mit Wasser (2 $\times$ 3 ml) gewaschen, getrocknet und eingeengt. Aus dem Rückstand wurde durch Ausrühren mit Ethanol (15 ml) in der Siedehitze 1,1-(3-Dimethylamino-3-phenylpentamethylen)-3,4-dihydro-1H-2-oxa-9-thiafluoren als diastereoisomerenreiner weißer Feststoff in einer Ausbeute von 322 mg mit einem Smp. von 219-222 °C erhalten.

Methode B:

Unter Argon wurden 4-Dimethylamino-4-phenylcyclohexanon (231,4 mg, 1,06 mmol) und 2-(Benzo[b]thiophen-3-yl)ethanol (190 mg, 1,06 mmol) in abs. DCM (10 ml) vorgelegt. Anschließend erfolgte die Zugabe von Methansulfonsäure (130 $\mu$l, 2 mmol). Der Ansatz wurde 20 h bei RT gerührt. Nach dieser Zeit war das Reaktionsgemisch hellbraun gefärbt und klar. Zur Aufarbeitung wurden 20 ml 1 M NaOH zugesetzt und bei RT 30 min gerührt. Die Phasen wurden getrennt. Die wässrige Phase (pH 11) wurde mit DCM (3 $\times$ 10 ml) extrahiert. Die organischen Phasen wurden vereinigt, mit Wasser (4 $\times$ 10 ml) gewaschen, getrocknet und eingeengt. Aus dem Rückstand wurde durch Ausrühren mit Ethanol (10 ml) in der Siedehitze 1,1-(3-Dimethylamino-3-phenylpentamethylen)-3,4-dihydro-1H-2-oxa-9-thiafluoren als diastereoisomerenreiner weißer Feststoff in einer Ausbeute von 340 mg mit einem Smp. von 218-222 °C isoliert. Es wurde das gleiche Diastereoisomer wie nach Methode A erhalten.

Referenz-Beispiel 6 - L-Tartrat:

1,1-(3-Dimethylamino-3-phenylpentamethylen)-3,4-dihydro-1H-2-oxa-9-thiafluoren (110 mg, 0,29 mmol) wurde in heißem Ethanol (50 ml) gelöst und mit einer 0,1 M Lösung von L-Weinsäure (3,2 ml, 0,32 mmol) in Ethanol versetzt. Nach dem Abkühlen auf RT wurde der Ansatz 24 h gerührt. Nach 24 h wurde das Lösungsmittel bis auf ein Restvolumen von ca. 10 ml eingeengt. Der nun ausgefallene Feststoff wurde bei RT abgesaugt, mit Ethanol (3 $\times$ 1 ml) gewaschen und getrocknet. Das L-Tartrat von 1,1-(3-Dimethylamino-3-phenylpentamethylen)-3,4-dihydro-1H-2-oxa-9-thiafluoren wurde so in einer Ausbeute von 130 mg als weißer Feststoff mit einem Smp. von 220-224 °C erhalten.

Referenz-Beispiel 7: 1,1-(3-Dimethylamino-3-(4-fluorphenyl)pentamethylen)-3,4-dihydro-1 H-2-oxa-9-thiafluoren Triflat

Unter Argon wurden 4-Dimethylamino-4-(4-fluorophenyl)cyclohexanon (470,6 mg, 2 mmol) und 2-(Benzo[b]thiophen-3-yl)ethanol (356,5 mg, 2 mmol) in abs. DCM (20 ml) vorgelegt. Anschließend erfolgte die Zugabe von Trifluormethansulfonsäuretrimethylsilylester (0,425 ml, 2,2 mmol). Der Ansatz wurde 64 h bei RT gerührt. Zur Aufarbeitung wurde der ausgefallene Feststoff abgesaugt, mit DCM (3 $\times$ 1 ml) gewaschen und getrocknet. Das Triflat von 1,1-(3-Dimethylamino-3-(4-fluorphenyl)pentamethylen)-3,4-dihydro-1H-2-oxa-9-thiafluoren wurde in einer Ausbeute von 383 mg als diastereoisomerenreiner weißer Feststoff mit einem Smp. von 212-215 °C erhalten.

Referenz-Beispiel 8: 1,1-(3-Dimethylamino-3-phenylpentamethylen)-3,4-dihydro-1H-2,9-dioxafluoren Hemicitrat

Unter Argon wurden 4-Dimethylamino-4-phenylcyclohexanon (868 mg, 4 mmol) und 2-(Benzofuran-3-yl)ethanol (648 mg, 4 mmol) in abs. DCM (20 ml) vorgelegt. Anschließend erfolgte eine sehr schnelle Zugabe von Trifluormethansulfonsäuretrimethylsilylester (0,8 ml, 4,14 mmol). Der Ansatz wurde 2 h bei RT gerührt. Zur Aufarbeitung wurde das Reaktionsgemisch mit 1 M NaOH (20 ml) versetzt und 30 min gerührt. Die organische Phase wurde abgetrennt und die verbleibende wässrige Phase mit DCM (3 $\times$ 20 ml) extrahiert. Die vereinigten organischen Phasen wurden mit Wasser (2 $\times$ 30 ml) gewaschen und über Natriumsulfat getrocknet. Der nach Abdestillieren des Lösungsmittels erhaltene feste Rückstand wurde mit Methanol (30 ml) versetzt, erwärmt und 15 h gerührt. Der in Methanol unlösliche Anteil wurde abgesaugt. Auf diese Weise wurde eines der beiden möglichen Diastereoisomeren von 1,1-(3-Dimethylamino-3-phenylpentamethylen)-3,4-dihydro-1H-2,9-dioxafluoren in einer Ausbeute von 650 mg mit einem Smp. von 206-208 °C erhalten. Zur Herstellung des Hemicitrates wurde das erhaltene Rohprodukt (600 mg, 1,66 mmol) in heißem Ethanol (100 ml) gelöst und mit einer ebenfalls heißen Lösung von Citronensäure (600 mg, 3,12 mmol) in Ethanol (20 ml) versetzt. Nach dem Abkühlen auf ca. 5 °C fiel ein Feststoff aus, der nach 2h Stehen abgesaugt wurde. Das Hemicitrat von1,1-(3-Dimethylamino-3-phenylpentamethylen)-3,4-dihydro-1 H-2,9-dioxafluoren wurde so in einer Ausbeute von 626 mg als weißer Feststoff (Smp.: 201-202 °C) erhalten.

Referenz-Beispiel 9: 1,1-(3-Dimethylamino-3-phenylpentamethylen)-3,4-dihydro-1H-2,9-diazafluoren Dihydrochlorid, unpolareres Diastereoisomer

4-Dimethylamino-4-phenylcyclohexanon (1,09 g, 5 mmol) und Tryptamin (800 mg, 5 mmol) wurden unter Ausschluß von Sauerstoff in trockenem 1,2-Dichlorethan (50 ml) gelöst. Zu diesem Gemisch wurde unter Rühren Trifluoressigsäure (770 μl, 10 mmol) und Natriumsulfat (2 g) hinzugefügt. Nach einer Reaktionszeit von 15 h wurde die Reaktionsmischung erneut mit Trifluoressigsäure (3 ml) versetzt und weitere 16 h bei RT gerührt. Zur Aufarbeitung wurde das Lösungsmittel abdestilliert und der Rückstand mit Wasser (20 ml) versetzt. Diese wässrige Phase wurde mit NaOH (5 mol/l) auf pH 11 eingestellt und mit EE(3 × 30 ml) extrahiert. Die organische Phase wurde mit Natriumsulfat getrocknet und eingeengt. Das Produkt war ein Gemisch der zwei diastereoisomeren 1,1-(3-Dimethylamino-3-phenylpentamethylen)-3,4-dihydro-1H-2,9-diazafluorene, die durch Chromatographie an Kieselgel mit Methanol getrennt werden konnten. Das unpolarere Produkt wurde in einer Ausbeute von 557 mg (31 %) als weißer Feststoff erhalten. Zur Herstellung des Dihydrochlorids wurden diese 557 mg in 2-Butanon (7 ml) suspendiert und mit Chlortrimethylsilan (500 μl, 3,75 mmol) versetzt. Der dabei entstandene Feststoff wurde abgesaugt und getrocknet. Das Dihydrochlorid des unpolareren Diastereoisomers von 1,1-(3-Dimethylamino-3-phenylpentamethylen)-3,4-dihydro-1H-2,9-diazafluoren wurde so in einer Ausbeute von 670 mg als weißer Feststoff mit einem Smp. von 243-247 °C gewonnen.

Referenz-Beispiel 10: 1,1-(3-Dimethylamino-3-phenylpentamethylen)-3,4-dihydro-1H-2,9-diazafluoren Dihydrochlorid, polareres Diastereoisomer

Wie für Referenz-Beispiel 9 beschrieben wurden auch 449 mg des polareren Diastereoisomers von 1,1-(3-Dimethylamino-3-phenylpentamethylen)-3,4-dihydro-1H-2,9-diazafluoren als weißer Feststoff erhalten. Zur Herstellung des Dihydrochlorids wurden diese 449 mg in 2-Butanon (7 ml) suspendiert und mit Chlortrimethylsilan (417 μl, 3,13 mmol) versetzt. Der dabei entstandene Feststoff wurde abgesaugt und getrocknet. Das Dihydrochlorid des polareren Diastereoisomers von 1,1-(3-Dimethylamino-3-phenylpentamethylen)-3,4-dihydro-1 H-2,9-diazafluoren wurde so in einer Ausbeute von 540 mg als weißer Feststoff mit einem Smp. von 244-246 °C gewonnen

Referenz-Beispiel 11: 2-Acetyl-1,1-(3-dimethylamino-3-phenylpentamethylen)-3,4-dihydro-1H-2,9-diazafluoren Hydrochlorid, unpolareres Diastereoisomer

Methode A:

Das unpolarere Diastereoisomer von 1,1-(3-Dimethylamino-3-phenylpentamethylen)-3,4-dihydro-1H-2,9-diazafluoren (375 mg, 1,04 mmol) wurde in Pyridin (10 ml) gelöst. Danach wurde Acetanhydrid (985 μl, 10,43 mmol) zugetropft und 2 d bei RT gerührt. Zur Aufarbeitung wurde Pyridin abdestilliert und der Rückstand mit Wasser (10 ml) versetzt. Der Ansatz wurde mit 5M NaOH auf pH 11 eingestellt und mit EE (3 × 15 ml) extrahiert. Die organische Phase wurde mit Natriumsulfat getrocknet und eingedampft. Der Rückstand wurde durch Säulenchromatographie an Kieselgel mit Methanol gereinigt. Das Acetamid des unpolareren Diastereoisomers von 1,1-(3-Dimethylamino-3-phenylpentamethylen)-3,4-dihydro-1H-2,9-diazafluoren wurde so in einer Ausbeute von 356 mg als weißer Feststoff erhalten. Zur Herstellung des Hydrochlorids wurden diese 356 mg in 2-Butanon (5 ml) suspendiert und mit Chlortrimethylsilan (178 μl, 1,34 mmol) versetzt. Der dabei entstandene Feststoff wurde abgesaugt und getrocknet. Das Hydrochlorid des unpolareren Diastereoisomers von 2-Acetyl-1,1-(3-dimethylamino-3-phenylpentamethylen)-3,4-dihydro-1 H-2,9-diazafluoren wurde so in einer Ausbeute von 388 mg als weißer Feststoff mit einem Smp. von 220-223 °C erhalten.

Methode B:

Zu einer Suspension des unpolareren Diastereoisomers von 1,1-(3-Dimethylamino-3-phenylpentamethylen)-3,4-dihydro-1H-2,9-diazafluoren (80 mg; 0,22 mmol) in 15 ml Acetonitril wurde Triethylamin (0,31 ml; 2,23 mmol) und anschließend Acetanhydrid (0,21 ml; 2,23 mmol) gegeben. Die Reaktionsmischung wurde im verschlossenen Gefäß im Mikrowellenofen (MLS-Ethos 1600 der Firma MLS GmbH, Leutkirch im Allgäu, Deutschland) 10 Minuten lang bei 1000 Watt auf 130 °C erhitzt. Dann wurde 5M wässrige Kaliumhydroxid-Lösung (6 ml) und Wasser (4 ml) hinzu gegeben und die wässrige Phase mit Dichlormethan (3x10ml) extrahiert. Nach Abtrennen der organischen Phase, Trocknen mit Natriumsulfat und Entfernen des Lösungsmittels im Vakuum erfolgte die weitere Aufreinigung durch Säulenchromatographie an Kieselgel mit EE und Methanol. Man erhielt 49 mg der acetylierten Base. Die Fällung des Hydrochlorides konnte wie unter Methode A beschrieben durchgeführt werden.

Referenz-Beispiel 12: 2-Acetyl-1,1-(3-dimethylamino-3-phenylpentamethylen)-3,4-dihydro-1H-2,9-diazafluoren Hydrochlorid, polareres Diastereoisomer

Das polarere Diastereoisomer von 1,1-(3-Dimethylamino-3-phenylpentamethylen)-3,4-dihydro-1H-2,9-diazafluoren (375 mg, 1,04 mmol) wurde in Pyridin (10 ml) gelöst. Danach wurde Acetanhydrid (985 μl, 10,43 mmol) zugetropft und 2 d bei RT gerührt. Zur Aufarbeitung wurde Pyridin abdestilliert und der Rückstand mit Wasser H2O (10 ml) versetzt. Der Ansatz wurde mit 5M NaOH auf pH 11 eingestellt und mit EE (3 × 15 ml) extrahiert. Die organische Phase wurde mit Natriumsulfat getrocknet und eingedampft. Das Produkt wurde durch Säulenchromatographie an Kieselgel mit Methanol gereinigt. Das Acetamid des polareren Diastereoisomers von 1,1-(3-Dimethylamino-3-phenylpentamethylen)-3,4-dihydro-1H-2,9-diazafluoren wurde so in einer Ausbeute von 339 mg als weißer Feststoff erhalten. Zur Herstellung des Hydrochlorids wurden diese 339 mg in 2-Butanon (5 ml) suspendiert und mit Chlortrimethylsilan (168 μl, 1,27 mmol) versetzt. Der dabei entstandene Feststoff wurde abgesaugt und getrocknet. Das Hydrochlorid des polareren Diastereoisomers von 2-Acetyl-1,1-(3-dimethylamino-3-phenylpentamethylen)-3,4-dihydro-1H-2,9-diazafluoren wurde so in einer Ausbeute von 370 mg als weißer Feststoff mit einem Smp. von 186-188 °C erhalten.

Referenz-Beispiel 13: 1,1-(3-Dimethylamino-3-phenylpentamethylen)-6-methoxy-1,3,4,9-tetrahydropyrano[3,4-b]indol Hydrochlorid

Unter Argon wurde 4-Dimethylamino-4-phenylcyclohexanon (550 mg, 2,5 mmol) und 5-Methoxy-3-(2-trimethylsilanyloxy-ethyl)-1 H-indol (789 mg, 3 mmol) in abs. DCM (30 ml) vorgelegt. Die Lösung wurde mit Hilfe einer Eis-Kochsalz-Mischung auf ca. 0 °C gekühlt und unter Rühren innerhalb von 5 min mit Trifluormethansulfonsäuretrimethylsilylester (0,5 ml, 2,5 mmol) versetzt. Der Ansatz wurde weitere 3 Stunden im Eisbad gekühlt, während ca. 1 h auf RT gebracht und anschließend weitere 10 h bei RT gerührt. Zur Aufarbeitung wurde das Reaktionsgemisch mit 1 M NaOH (30 ml) versetzt und 30 min gerührt. Die organische Phase wurde abgetrennt und die verbleibende wässrige Phase mit DCM (2 × 30 ml) extrahiert. Die vereinigten organischen Phasen wurden mit Wasser (2 × 30 ml) gewaschen und über Natriumsulfat getrocknet. Der nach Abdestillieren des Lösungsmittels erhaltene, weitgehend feste Rückstand wurde mit Methanol (70 ml) versetzt, 2 h gerührt und die erhaltene Suspension filtriert. Es wurden 478 mg eines der beiden möglichen Diastereoisomeren von 1,1-(3-Dimethylamino-3-phenylpentamethylen)-6-methoxy-1,3,4,9-tetrahydropyrano[3,4-b]indol mit einem Smp. von 244-246 °C erhalten. 430 mg hiervon wurden in 2-Butanon (25 ml) gelöst, mit Chlortrimethylsilan (250 μl, 1,98 mmol) versetzt und 30 Minuten bei RT gerührt. Der entstandene Feststoff wurde abgesaugt. Das Hydrochlorid von 1,1-(3-Dimethylamino-3-phenylpentamethylen)-6-methoxy-1,3,4,9-tetrahydropyrano[3,4-b]indol wurde so in einer Ausbeute von 396 mg als weißer Feststoff mit einem Smp. von 279-280 °C erhalten.

Referenz-Beispiel 14: 1,1-(3-Dimethylamino-3-phenylpentamethylen)-3-methyl-1,3,4,9-tetrahydropyrano[3,4-b]indol Hemicitrat, unpolareres Diastereoisomer

Unter Argon wurden 4-Dimethylamino-4-phenylcyclohexanon (434 mg, 2 mmol) und 3-(2-Trimethylsilanyloxypropyl)-1 H-indol (592 mg, 2,4 mmol) in abs. DCM (15 ml) vorgelegt. Die Lösung wurde mit Hilfe einer Eis-Kochsalz-Mischung auf ca. 0 °C gekühlt und unter Rühren innerhalb von 5 min mit Trifluormethansulfonsäuretrimethylsilylester (0,39 ml, 2 mmol) versetzt. Der Ansatz wurde weitere 4 h im Eisbad gekühlt. Nach Erwärmen auf RT wurde weitere 20 h gerührt. Zur Aufarbeitung wurde das Reaktionsgemisch mit 1 M NaOH (20 ml) versetzt und 30 min gerührt. Die organische Phase wurde abgetrennt und die verbleibende wässrige Lösung mit DCM (2 × 30 ml) extrahiert. Die vereinigten organischen Phasen wurden mit Wasser (2 × 30 ml) gewaschen und über Natriumsulfat getrocknet. Der nach Abdestillieren des Lösungsmittels erhaltene weitgehend feste Rückstand wurde mit Methanol (70 ml) versetzt und 2 h gerührt. Es entstand eine Suspension, aus der durch Filtration das in Methanol schwer lösliche unpolarere Diastereoisomer von 1,1-(3-Dimethylamino-3-phenylpentamethylen)-3-methyl-1,3,4,9-tetrahydropyrano[3,4-b]indol in einer Ausbeute von 127 mg als weißer Feststoff mit einem Smp. von 306-312 °C erhalten wurde. 94 mg hiervon wurden in heißem Ethanol (50 ml) gelöst und mit einer ebenfalls heißen Lösung von Citronensäure (48 mg, 0,25 mmol) in Ethanol (10 ml) versetzt. Nach dem Abkühlen wurde der Ansatz 3 d stehen gelassen. Der entstandene Feststoff wurde abgesaugt. Das Hemicitrat des unpolareren Diastereoisomers von 1,1-(3-Dimethylamino-3-phenylpentamethylen)-3-methyl-1,3,4,9-tetrahydropyrano[3,4-b]indol konnte so in einer Ausbeute von 67 mg als weißer Feststoff (Zers. ab 280 °C) erhalten werden.

Referenz-Beispiel 15: 1,1-(3-Dimethylamino-3-phenylpentamethylen)-3-methyl-1,3,4,9-tetrahydropyrano[3,4-b]indol Citrat, polareres Diastereoisomer

4-Dimethylamino-4-phenylcyclohexanon (149 mg, 0,69 mmol) und 1-(1H-Indol-3-yl)propan-2-ol (120 mg, 0,69 mmol) wurden in konz. Essigsäure (4 ml) gelöst. Zu diesem Gemisch wurde Phosphorsäure (1 ml, 85 m%) langsam zugetropft. Nach einer Reaktionszeit von 5 min entstand eine rote Lösung aus der ein weißer Feststoff ausfiel. Es wurde

16 h bei RT gerührt. Zur Aufarbeitung wurde der Ansatz mit Wasser (20 ml) verdünnt, mit 5M NaOH auf pH 11 eingestellt und mit DCM (3 × 20 ml) extrahiert. Die organische Phase wurde mit Natriumsulfat getrocknet und eingedampft. Der Rückstand bestand hauptsächlich aus dem polareren Diastereoisomer, das in einer Ausbeute von 260 mg als weißer Feststoff erhalten werden konnte. Zur Herstellung des Citrates wurden diese 260 mg, 0,69 mmol) in heißem Ethanol (20 ml) suspendiert und mit einer ebenfalls heißen Lösung von Citronensäure (133 mg, 0,69 mmol) in Ethanol (5 ml) versetzt. Die Substanz ging dabei vollständig in Lösung und fiel auch bei Abkühlen auf ca. 5 °C nicht mehr aus. Ethanol wurde am Rotationsverdampfer entfernt und das Citrat des polareren Diastereoisomers von 1,1-(3-Dimethylamino-3-phenylpentamethylen)-3-methyl-1,3,4,9-tetrahydropyrano[3,4-b]indol konnte so in einer Ausbeute von 392 mg als weißer Feststoff (Smp.: 160-165 °C) erhalten werden.

Referenz-Beispiel 16: 6-Brom-1,1-(3-dimethylamino-3-phenylpentamethylen)-3-methyl-1,3,4,9-tetrahydropyrano[3,4-b]indol Hemicitrat

Unter Argon wurden 4-Dimethylamino-4-phenylcyclohexanon (651 mg, 3 mmol) und 5-Brom-3-(2-trimethylsilanyloxypropyl)-1 H-indol (975 mg, 3 mmol) in abs. DCM (15 ml) vorgelegt. Die Lösung wurde mit Hilfe einer Eis-Kochsalz-Mischung auf ca. 0 °C gekühlt und unter Rühren innerhalb von 5 min mit Trifluormethansulfonsäuretrimethylsilylester (0,6 ml, 3,1 mmol) versetzt. Der Ansatz wurde weitere 2 h im Eisbad gekühlt. Nach Erwärmen auf RT wurde weitere 20 h gerührt. Zur Aufarbeitung wurde das Reaktionsgemisch mit 1 M NaOH (30 ml) versetzt und 30 min gerührt. Die organische Phase wurde abgetrennt und die verbleibende wässrige Lösung mit DCM (2 × 30 ml) extrahiert. Die vereinigten organischen Extrakte wurden mit Wasser (2 × 30 ml) gewaschen und über Natriumsulfat getrocknet. Der nach Abdestillieren des Lösungsmittels erhaltene weitgehend feste Rückstand wurde mit Methanol (70 ml) versetzt und 1 h gerührt. Das nicht in Lösung gegangene Material wurde abgesaugt. Es erwies sich als eines der beiden möglichen racemischen Diastereoisomeren von 6-Brom-1,1-(3-dimethylamino-3-phenylpentamethylen)-3-methyl-1,3,4,9-tetrahydropyrano[3,4-b]indol, das so in einer Ausbeute von 260 mg (19 %) als weißer Feststoff mit einem Smp. von 287-293 °C in reiner Form erhalten wurde. 250 mg hiervon wurden in heißem Ethanol (120 ml) gelöst und mit einer ebenfalls heißen Lösung von Citronensäure (120 mg, 0,62 mmol) in Ethanol (10 ml) versetzt. Der Ansatz wurde abgekühlt und 20 h bei ca. 10 °C belassen. Der entstandene Feststoff wurde abgesaugt. Das Hemicitrat von 6-Brom-1,1-(3-dimethylamino-3-phenylpentamethylen)-3-methyl-1,3,4,9-tetrahydropyrano[3,4-b]indol konnte so in einer Ausbeute von 188 mg als weißer Feststoff (Smp. ab 230 °C Kristallumwandlung, ab 290 °C Sublimation) erhalten werden.

Referenz-Beispiel 17: 1,1-(3-Dimethylamino-3-phenylpentamethylen)-3-methyl-6-nitro-1,3,4,9-tetrahydropyrano[3,4-b]indol Citrat, unpolareres Diastereoisomer

Unter Argon wurden 4-Dimethylamino-4-phenylcyclohexanon (651 mg, 3 mmol) und 5-Nitro-3-(2-trimethylsilanyloxy-propyl)-1 H-indol (876 mg, 3 mmol) in abs. DCM (20 ml) vorgelegt. Die Lösung wurde mit Hilfe einer Eis-Kochsalz-Mischung auf ca. 0 °C gekühlt und unter Rühren innerhalb von 5 min mit Trifluormethansulfonsäuretrimethylsilylester (0,6 ml, 3,1 mmol) versetzt. Der Ansatz wurde weitere 2 h im Eisbad gekühlt. Nach Erwärmen auf RT wurde weitere 70 h gerührt. Zur Aufarbeitung wurde das Reaktionsgemisch mit 1 M NaOH (50 ml) sowie DCM (20 ml) versetzt und 30 min gerührt. Die organische Phase wurde abgetrennt und die verbleibende wässrige Lösung mit DCM (3 × 40 ml) extrahiert. Die vereinigten organischen Phasen wurden mit Wasser (2 × 30 ml) gewaschen und über Natriumsulfat getrocknet. Der nach Abdestillieren des Lösungsmittels erhaltene, glasige Rückstand wurde mit Methanol (30 ml) versetzt und 1 h gerührt. Der im Methanol unlösliche Feststoff erwies sich als Diastereoisomerengemisch. Durch säulenchromatographische Auftrennung an Kieselgel (Laufmittel: EE) konnten die beiden racemischen Diastereoisomeren getrennt werden. Das unpolarere Produkt wurde in einer Ausbeute von 154 mg als weißer Feststoff mit einem Smp. von 252-265 °C in reiner Form erhalten. 134 mg hiervon wurden in heißem Ethanol (150 ml) gelöst und mit einer ebenfalls heißen Lösung von Citronensäure (110 mg, 0,57 mmol) in Ethanol (20 ml) versetzt. Der Ansatz wurde abgekühlt und 20 h bei ca. 10 °C belassen. Der entstandene Feststoff wurde abgesaugt. Das Citrat des unpolareren Diastereoisomers von 1,1-(3-Dimethylamino-3-phenylpentamethylen)-3-methyl-6-nitro-1,3,4,9-tetrahydropyrano[3,4-b]indol wurde so in einer Ausbeute von 117 mg mit einem Smp. von 258-262 °C erhalten.

Referenz-Beispiel 18: 1,1-(3-Dimethylamino-3-phenylpentamethylen)-3-methyl-6-nitro-1,3,4,9-tetrahydropyrano[3,4-b]indol Citrat, polareres Diastereoisomer

Wie für Referenz-Beispiel 17 beschrieben wurden auch 120 mg des polareren Diastereoisomers von 1,1-(3-Dimethylamino-3-phenylpentamethylen)-3-methyl-6-nitro-1,3,4,9-tetrahydropyrano[3,4-b]indol mit einem Smp. von 230-240 °C erhalten. Diese 120 mg wurden in heißem Ethanol (120 ml) gelöst und mit einer ebenfalls heißen Lösung von Citronensäure (100 mg, 0,52 mmol) in Ethanol (10 ml) versetzt. Die Lösung wurde abgekühlt und im Vakuum bis zur Trockene eingeengt. Der erhaltene Rückstand wurde in Wasser (10 ml) aufgenommen, wobei das Citrat als kristalliner

Feststoff anfiel. Nach Filtration und Trocknung wurde das Citrat des polareren Diastereoisomers von 1,1-(3-Dimethylamino-3-phenylpentamethylen)-3-methyl-6-nitro-1,3,4,9-tetrahydropyrano[3,4-b]indol in einer Ausbeute von 76 mg mit einen Smp. von 190-192 °C erhalten.

Referenz-Beispiel 19: 6-Chlor-1,1-(3-Dimethylamino-3-phenylpentamethylen)-3-methyl-1,3,4,9-tetrahydropyrano[3,4-b]indol Citrat, unpolareres Diastereoisomer

Unter Argon wurden 4-Dimethylamino-4-phenylcyclohexanon (600 mg, 2,76 mmol) und 5-Chlor-3-(2-trimethylsilanyloxypropyl)-1 H-indol (846 mg, 3 mmol) in abs. DCM (30 ml) vorgelegt. Die Lösung wurde mit Hilfe einer Eis-Kochsalz-Mischung auf ca. 0 °C gekühlt und unter Rühren innerhalb von 5 min mit Trifluormethansulfonsäuretrimethylsilylester (0,6 ml, 3,1 mmol) versetzt. Der Ansatz wurde weitere 2 h im Eisbad gekühlt. Nach Erwärmen auf RT wurde weitere 18 h gerührt. Zur Aufarbeitung wurde das Reaktionsgemisch mit 1 M NaOH (30 ml) versetzt und 30 min gerührt. Die organische Phase wurde abgetrennt und die verbleibende wässrige Lösung mit DCM (2 × 30 ml) extrahiert. Die vereinigten organischen Phasen wurden mit Wasser (2 × 30 ml) gewaschen und über Natriumsulfat getrocknet. Der nach Abdestillieren des Lösungsmittels erhaltene ölige Rückstand wurde mit Methanol (50 ml) versetzt und 1 h gerührt. Es entstand eine Suspension. Der im Methanol unlösliche Feststoff wurde abgetrennt und durch säulenchromatographische Auftrennung an Kieselgel (Laufmittel: EE) konnte das unpolarere Diastereoisomer von 6-Chlor-1,1-(3-Dimethylamino-3-phenylpentamethylen)-3-methyl-1,3,4,9-tetrahydropyrano[3,4-b]indol in einer Ausbeute von 60 mg als weißer Feststoff (Smp: ab 180 °C) erhalten werden. 55 mg hiervon wurden in heißem Ethanol (40 ml) gelöst und mit einer ebenfalls heißen Lösung von Citronensäure (50 mg, 0,26 mmol) in Ethanol (10 ml) versetzt. Die Lösung wurde abgekühlt und im Vakuum bis zur Trockene eingeengt. Der erhaltene Rückstand wurde in Wasser (10 ml) aufgenommen, wobei das Citrat des unpolareren Diastereoisomers von 6-Chlor-1,1-(3-Dimethylamino-3-phenylpentamethylen)-3-methyl-1,3,4,9-tetrahydropyrano[3,4-b]indol als kristalliner Feststoff anfiel. Nach Filtration und Trocknung wurden 36 mg mit einen Smp. von 185-195 °C erhalten.

Referenz-Beispiel 20: 6-Chlor-1,1-(3-Dimethylamino-3-phenylpentamethylen)-3-methyl-1,3,4,9-tetrahydropyrano[3,4-b]indol Citrat, polareres Diastereoisomer

4-Dimethylamino-4-phenylcyclohexanon (217 mg, 1 mmol) und 1-(5-Chlor-1 H-indol-3-yl)-propan-2-ol (209 mg, 1 mmol) wurden in konz. Essigsäure (4 ml) gelöst. Zu diesem Gemisch wurde Phosphorsäure (1 ml, 85 m%) langsam zugetropft. Nach einer Reaktionszeit von 60 min entstand eine rote Lösung. Es wurde 20 h bei RT gerührt. Zur Aufarbeitung wurde der Ansatz mit Wasser (20 ml) verdünnt, mit 5M NaOH auf pH 11 eingestellt und mit DCM (3 × 20 ml) extrahiert. Die organische Phase wurde mit Natriumsulfat getrocknet und eingedampft. Das Produkt bestand fast ausschließlich aus dem polareren Diastereoisomer von 6-Chlor-1,1-(3-Dimethylamino-3-phenylpentamethylen)-3-methyl-1,3,4,9-tetrahydropyrano[3,4-b]indol (390 mg gelber Feststoff). Diese 390 mg wurden in heißem Ethanol (20 ml) suspendiert und mit einer ebenfalls heißen Lösung von Citronensäure (385 mg, 2 mmol) in Ethanol (10 ml) versetzt. Beim Abkühlen auf ca. 5 °C fiel das Citrat des polareren Diastereoisomers von 6-Chlor-1,1-(3-Dimethylamino-3-phenylpentamethylen)-3-methyl-1,3,4,9-tetrahydropyrano[3,4-b]indol aus. Es wurde abgesaugt und getrocknet (768 mg gelber Feststoff, Smp. 155-160 °C).

Referenz-Beispiel 21: 3,9-Dimethyl-1,1-(3-dimethylamino-3-phenylpentamethylen)-1,3,4,9-tetrahydropyrano[3,4-b]indol Citrat

4-Dimethylamino-4-phenylcyclohexanon (434 mg, 2 mmol) und 1-Methyl-3-(2-trimethylsilanyloxy-propyl)-1 H-indol (622 mg, 2 mmol) wurden unter Argon in abs. DCM (20 ml) vorgelegt, unter Rühren mit Trifluormethansulfonsäure (0,18 ml, 2 mmol) versetzt und 20 h gerührt. Zur Aufarbeitung wurde das Reaktionsgemisch mit 1 M NaOH (20 ml) versetzt und 30 min gerührt. Die organische Phase wurde abgetrennt und die verbleibende wässrige Lösung mit DCM (2 × 30 ml) extrahiert. Die vereinigten organischen Phasen wurden mit Wasser (2 × 30 ml) gewaschen und über Natriumsulfat getrocknet. Der nach Abdestillieren des Lösungsmittels erhaltene Rückstand wurde mit Methanol (50 ml) versetzt und 1 h gerührt. Der in Methanol unlösliche Feststoff wurde abgetrennt und getrocknet. Auf diese Weise wurde eines der beiden möglichen Diastereoisomeren von 3,9-Dimethyl-1,1-(3-dimethylamino-3-phenylpentamethylen)-1,3,4,9-tetrahydropyrano[3,4-b]indol erhalten (560 mg, Smp. 210-212 °C). 388 mg hiervon wurden in heißem Ethanol (50 ml) gelöst und mit einer ebenfalls heißen Lösung von Citronensäure (384 mg, 2 mmol) in Ethanol (20 ml) versetzt. Die Lösung wurde abgekühlt und im Vakuum bis zur Trockene eingeengt. Der erhaltene Rückstand wurde in Wasser (20 ml) aufgenommen, wobei das Citrat nach Anreiben als kristalliner Feststoff anfiel. Zur Vervollständigung der Fällung wurde die wässrige Lösung über Nacht stehen gelassen. Nach Filtration und Trocknung wurden 285 mg des Citrats von 3,9-Dimethyl-1,1-(3-dimethylamino-3-phenylpentamethylen)-1,3,4,9-tetrahydropyrano[3,4-b]indol (Smp. 156-158 °C) erhalten.

Referenz-Beispiel 22: 1,1-(3-Dimethylamino-3-(4-fluorphenyl)pentamethylen)-1,3,4,9-tetrahydropyrano[3,4-b]indol Hemicitrat

4-Dimethylamino-4-(4-fluorphenyl)cyclohexanon (705 mg, 3 mmol) und Tryptophol (483 mg, 3 mmol) wurden unter Argon in abs. DCM (20 ml) vorgelegt. Anschließend erfolgte eine sehr schnelle Zugabe von Trifluormethansulfonsäuretrimethylsilylester (0,6 ml, 3,1 mmol). Der Ansatz wurde 18 h bei RT gerührt. Zur Aufarbeitung wurde das Reaktionsgemisch mit 1 M NaOH (20 ml) versetzt und 30 min gerührt. Die organische Phase wurde abgetrennt und die verbleibende wässrige Phase mit DCM (3 × 30 ml) extrahiert. Die vereinigten organischen Phasen wurden mit Wasser (2 × 30 ml) gewaschen und über Natriumsulfat getrocknet. Der nach Abdestillieren des Lösungsmittels erhaltene, feste Rückstand wurde mit Methanol (20 ml) versetzt, erwärmt und 15 h gerührt. Der in der Suspension enthaltene Feststoff wurde abgesaugt. Auf diese Weise wurde eines der beiden möglichen Diastereoisomeren von 1,1-(3-Dimethylamino-3-(4-fluorphenyl)pentamethylen)-1,3,4,9-tetrahydropyrano[3,4-b]indol erhalten (755 mg, Smp. 292-302 °C). Diese 755 mg wurden in heißem Ethanol (400 ml) gelöst und mit einer ebenfalls heißen Lösung von Citronensäure (600 mg, 3,12 mmol) in Ethanol (50 ml) versetzt. Nach dem Abkühlen auf ca. 5 °C wurde der Ansatz 2 h stehen gelassen. Der entstandene Feststoff wurde abgesaugt. Es wurde das Hemicitrat von 1,1-(3-Dimethylamino-3-(4-fluorphenyl)pentamethylen)-1,3,4,9-tetrahydropyrano[3,4-b]indol erhalten (632 mg weißer Feststoff, Smp. 241-250 °C unter Zersetzung).

Referenz-Beispiel 23: 1,1-(3-Dimethylamino-3-(3-fluorphenyl)pentamethylen)-1,3,4,9-tetrahydropyrano[3,4-b]indol Hemicitrat

4-Dimethylamino-4-(3-fluorphenyl)cyclohexanon (434 mg, 1,84 mmol) und Tryptophol (296 mg, 1,84 mmol) wurden unter Argon in abs. DCM (20 ml) vorgelegt. Anschließend erfolgte eine sehr schnelle Zugabe von Trifluormethansulfonsäuretrimethylsilylester (0,38 ml, 1,97 mmol). Der Ansatz wurde 20 h bei RT gerührt. Zur Aufarbeitung wurde das Reaktionsgemisch mit 1 M NaOH (20 ml) versetzt und 30 min gerührt. Die organische Phase wurde abgetrennt und die verbleibende wässrige Phase mit DCM (3 × 100 ml) extrahiert. Die vereinigten organischen Phasen wurden mit Wasser (2 × 30 ml) gewaschen und über Natriumsulfat getrocknet. Der nach Abdestillieren des Lösungsmittels erhaltene feste Rückstand wurde mit Methanol (20 ml) versetzt, erwärmt und 15 h gerührt. Der in der Suspension enthaltene Feststoff wurde abgesaugt. Auf diese Weise wurde eins der beiden möglichen Diastereoisomeren erhalten (482 mg, Smp. 298-301 °C). Diese 482 mg wurden in heißem Ethanol (400 ml) gelöst und mit einer ebenfalls heißen Lösung von Citronensäure (490 mg, 2,55 mmol) in Ethanol (50 ml) versetzt. Nach dem Abkühlen auf ca. 5 °C wurde der Ansatz 2 h stehen gelassen. Der entstandene Feststoff wurde abgesaugt. Es wurde das Hemicitrat von 1,1-(3-Dimethylamino-3-(3-fluorphenyl)pentamethylen)-1,3,4,9-tetrahydropyrano[3,4-b]indol erhalten (351 mg weißer Feststoff, Smp. 286-291 °C, ab 245 Kristallumwandlung, oberhalb 280 °C Sublimation).

Referenz-Beispiel 24: 1,1-(3-Dimethylamino-3-phenylpentamethylen)-6-fluor-1,3,4,9-tetrahydropyrano[3,4-b]indol Hemicitrat, unpolareres Diastereoisomer

Unter Argon wurden 4-Dimethylamino-4-phenylcyclohexanon (651 mg, 3 mmol) und 2-(5-Fluor-1 H-indol-3-yl)-ethanol ("5-Fluortryptophol", 537 mg, 3 mmol) in abs. DCM (20 ml) vorgelegt. Anschließend erfolgte eine sehr schnelle Zugabe von Trifluormethansulfonsäuretrimethylsilylester (0,6 ml, 3,1 mmol). Der Ansatz wurde 20 h bei RT gerührt. Zur Aufarbeitung wurde das Reaktionsgemisch mit 1 M NaOH (30 ml) versetzt und 30 min gerührt. Die organische Phase wurde abgetrennt und die verbleibende wässrige Phase mit DCM (3 × 60 ml) extrahiert. Die vereinigten organischen Phasen wurden mit Wasser (2 × 30 ml) gewaschen und über Natriumsulfat getrocknet. Der nach Abdestillieren des Lösungsmittels erhaltene feste Rückstand wurde mit Methanol (30 ml) versetzt, erwärmt und 15 h gerührt. Der in der Suspension enthaltene Feststoff wurde abgesaugt und getrocknet. Es wurden 955 mg des unpolareren Diastereoisomers von 1,1-(3-Dimethylamino-3-phenylpentamethylen)-6-fluor-1,3,4,9-tetrahydropyrano[3,4-b]indol erhalten (Smp. 284-292 °C). 850 mg hiervon wurden in heißem Ethanol (900 ml) gelöst und mit einer ebenfalls heißen Lösung von Citronensäure (1 g, 5,2 mmol) in Ethanol (20 ml) versetzt. Nach ca. 15 Minuten fielen in der Siedehitze Kristalle aus. Nach dem Abkühlen auf ca. 5 °C wurde der Ansatz 2 h stehen gelassen. Der entstandene Feststoff wurde abgesaugt. Es wurden 640 mg des Hemicitrats als weißer Feststoff erhalten (Smp. 258-282 °C).

Referenz-Beispiel 25: 1,1-(3-Dimethylamino-3-phenylpentamethylen)-6-fluor-1,3,4,9-tetrahydropyrano[3,4-b]indol Hemicitrat, polareres Diastereoisomer

4-Dimethylamino-4-phenylcyclohexanon (217 mg, 1 mmol) und 2-(5-Fluor-1 H-indol-3-yl)-ethanol ("5-Fluortryptophol", 179 mg, 1 mmol) wurden in konz. Essigsäure (4 ml) gelöst. Zu diesem Gemisch wurde Phosphorsäure (1 ml, 85 m%) langsam zugetropft. Es wurde 16 h bei RT gerührt. Zur Aufarbeitung wurde der Ansatz mit Wasser (20 ml) verdünnt, mit 5M NaOH auf pH 11 eingestellt und mit DCM (3 × 20 ml) extrahiert. Die vereinigten organischen Phase

wurde mit Natriumsulfat getrocknet und eingedampft. Der Rückstand (364 mg weißer Feststoff) wurde in heißem Ethanol (20 ml) suspendiert und mit einer ebenfalls heißen Lösung von Citronensäure (185 mg, 0,96 mmol) in Ethanol (5 ml) versetzt. Der Rückstand ging dabei vollständig in Lösung und fiel auch bei Abkühlen auf ca. 5 °C nicht mehr aus. Ethanol wurde am Rotationsverdampfer entfernt und das Hemicitrat des polareren Diastereoisomers von 1,1-(3-Dimethylamino-3-phenylpentamethylen)-6-fluor-1,3,4,9-tetrahydropyrano[3,4-b]indol wurde so in einer Ausbeute von 548 mg als weißer Feststoff (Smp. 148-155 °C) erhalten.

Referenz-Beispiel 26: 1,1-(3-Dimethylamino-3-phenylpentamethylen)-6-methyl-1,3,4,9-tetrahydropyrano[3,4-b]indol Hemicitrat

Unter Argon wurden 4-Dimethylamino-4-phenylcyclohexanon (325 mg, 1,5 mmol) und 2-(5-Methyl-1 H-indol-3-yl)-ethanol ("5-Methyltryptophol", 262 mg, 1,5 mmol) in abs. DCM (10 ml) vorgelegt. Anschließend erfolgte eine sehr schnelle Zugabe von Trifluormethansulfonsäuretrimethylsilylester (0,3 ml, 1,55 mmol). Der Ansatz wurde 24 h bei RT gerührt. Zur Aufarbeitung wurde das Reaktionsgemisch mit 1 M NaOH (20 ml) versetzt und 30 min gerührt. Die organische Phase wurde abgetrennt und die verbleibende wässrige Phase mit DCM (3 × 20 ml) extrahiert. Die vereinigten organischen Phasen wurden mit Wasser (2 × 30 ml) gewaschen und über Natriumsulfat getrocknet. Der nach Abdestillieren des Lösungsmittels erhaltene feste Rückstand wurde mit Methanol (30 ml) versetzt, erwärmt und 15 h gerührt. Der suspendierte Feststoff wurde abgesaugt. Es wurde eines der beiden möglichen Diastereoisomeren von 1,1-(3-Dimethylamino-3-phenylpentamethylen)-6-methyl-1,3,4,9-tetrahydropyrano[3,4-b]indol erhalten (430 mg, Smp. 259-270 °C). 350 mg hiervon wurden in heißem Ethanol (300 ml) gelöst und mit einer ebenfalls heißen Lösung von Citronensäure (300 mg, 1,56 mmol) in Ethanol (10 ml) versetzt. Nach ca. 15 Minuten fielen in der Siedehitze Kristalle aus. Nach dem Abkühlen auf ca. 5 °C wurde der Ansatz 2 h stehen gelassen. Der entstandene Feststoff wurde abgesaugt. Das Hemicitrat von 1,1-(3-Dimethylamino-3-phenylpentamethylen)-6-methyl-1,3,4,9-tetrahydropyrano[3,4-b]indol konnte so in einer Ausbeute von 380 mg erhalten werden (weißer Feststoff, Smp. 243-265 °C).

Referenz-Beispiel 27: 1,1-(3-Dimethylamino-3-phenylpentamethylen)-9-phenyl-1,3,4,9-tetrahydropyrano[3,4-b]indol Citrat

Unter Argon wurden 4-Dimethylamino-4-phenylcyclohexanon (325 mg, 1,5 mmol) und 2-(1-Phenyl-1 H-indol-3-yl)-ethanol (355 mg, 1,5 mmol) in abs. DCM (20 ml) vorgelegt. Anschließend erfolgte eine sehr schnelle Zugabe von Trifluormethansulfonsäure (0,14 ml, 1,58 mmol). Der Ansatz wurde 20 h bei RT gerührt. Zur Aufarbeitung wurde das Reaktionsgemisch mit 1 M NaOH (30 ml) versetzt und 30 min gerührt. Die organische Phase wurde abgetrennt und die verbleibende wässrige Phase mit DCM (3 × 60 ml) extrahiert. Die vereinigten organischen Phasen wurden mit Wasser (2 × 30 ml) gewaschen und über Natriumsulfat getrocknet. Der nach Abdestillieren des Lösungsmittels erhaltene feste Rückstand wurde mit Methanol (30 ml) versetzt, erwärmt und 15 h gerührt. Der suspendierte Feststoff wurde abgesaugt. Es wurde eines der beiden möglichen Diastereoisomeren von 1,1-(3-Dimethylamino-3-phenylpentamethylen)-9-phenyl-1,3,4,9-tetrahydropyrano[3,4-b]indol erhalten (385 mg, Smp. 256-261 °C). 672 mg dieses Diastereoisomers von 1,1-(3-Dimethylamino-3-phenylpentamethylen)-9-phenyl-1,3,4,9-tetrahydropyrano[3,4-b]indol wurden in heißem Ethanol (500 ml) gelöst und mit einer ebenfalls heißen Lösung von Citronensäure (500 g, 2,6 mmol) in Ethanol (20 ml) versetzt. Anschließend wurde die Lösung auf ca. 100 ml eingeengt. Nach dem Abkühlen auf ca. 5 °C wurde der Ansatz 48 h stehen gelassen. Der entstandene Feststoff wurde abgesaugt und getrocknet. Es wurden 570 mg des Citrats von 1,1-(3-Dimethylamino-3-phenylpentamethylen)-9-phenyl-1,3,4,9-tetrahydropyrano[3,4-b]indol erhalten (weißer Feststoff, Smp. 255-260 °C, ab 205 °C Kristallumwandlung).

Referenz-Beispiel 28: 1,1-(3-Methylamino-3-phenylpentamethylen)-1,3,4,9-tetrahydropyrano[3,4-b]indol Hemicitrat

Unter Argon wurden 4-Methylamino-4-phenyl-cyclohexanon (609 mg, 3 mmol) und Tryptophol (483 mg, 3 mmol) in abs. DCM (20 ml) vorgelegt. Anschließend erfolgte eine sehr schnelle Zugabe von Trifluormethansulfonsäure (0,28 ml, 3,16 mmol). Der Ansatz wurde 20 h bei RT gerührt. Zur Aufarbeitung wurde das Reaktionsgemisch mit 1 M NaOH (20 ml) versetzt und 30 min gerührt. Die organische Phase wurde abgetrennt und die verbleibende wässrige Phase mit DCM (3 × 30 ml) extrahiert. Die vereinigten organischen Extrakte wurden mit Wasser (2 × 30 ml) gewaschen und über Natriumsulfat getrocknet. Der nach Abdestillieren des Lösungsmittels erhaltene feste Rückstand wurde mit Methanol (30 ml) versetzt, erwärmt und 15 h gerührt. Der in der Suspension enthaltene Feststoff wurde abgesaugt. Auf diese Weise wurde eins der beiden möglichen Diastereoisomeren von 1,1-(3-Methylamino-3-phenylpentamethylen)-1,3,4,9-tetrahydropyrano[3,4-b]indol in einer Ausbeute von 630 mg erhalten (Smp. 260-262 °C). 600 mg hiervon wurden in heißem Ethanol (150 ml) gelöst und mit einer ebenfalls heißen Lösung von Citronensäure (600 mg, 3,12 mmol) in Ethanol (10 ml) versetzt. Nach dem Abkühlen auf ca. 5 °C wurde der Ansatz 12 h stehen gelassen. Der entstandene Feststoff wurde abgesaugt. Es wurden 663 mg des Hemicitrats von 1,1-(3-Methylamino-3-phenylpentamethylen)-1,3,4,9-tetrahydropyrano[3,4-b]indol erhalten (weißer Feststoff, Smp. 252-254 °C).

Referenz-Beispiel 29: 1,1-(3-Dimethylamino-3-phenylpentamethylen)-6-methyl-3,4-dihydro-1H-2,9-diazafluoren Citrat

4-Dimethylamino-4-phenylcyclohexanon (1,2 g, 5,53 mmol) und 5-Methyltryptamin (963 mg, 5,53 mmol) wurden unter Ausschluß von Sauerstoff in trockenem Methanol (40 ml) gelöst. Zu diesem Gemisch wurde Natriumsulfat (2 g) hinzugefügt. Nach einer Reaktionszeit von 24 h wurde das Methanol abdestilliert und der Rückstand in 1,2-Dichlorethan (40 ml) suspendiert. Die Reaktionsmischung wurde mit Trifluoressigsäure (4 ml) versetzt und 18 h bei RT gerührt. Zur Aufarbeitung wurde der Ansatz mit Wasser (30 ml) verdünnt, mit NaOH (5 mol/l) auf pH 11 eingestellt und mit 1,2-Dichlorethan (3 × 30 ml) extrahiert. Die organische Phase wurde mit Natriumsulfat getrocknet und eingeengt. Der braune feste Rückstand wurde aus Methanol umkristallisiert. Es wurden 236 mg eines weißen Feststoffs erhalten. 100 mg hiervon wurden in heißem Ethanol (10 ml) gelöst und mit einer ebenfalls heißen Lösung von Citronensäure (62 mg, 0,32 mmol) in Ethanol (1 ml) versetzt. Nach dem Abkühlen auf ca. 5 °C wurde der Ansatz 4 h stehen gelassen. Der entstandene Feststoff wurde abgesaugt. Das Citrat eines Diastereoisomers von 1,1-(3-Dimethylamino-3-phenylpentamethylen)-6-methyl-3,4-dihydro-1H-2,9-diazafluoren wurde so in einer Ausbeute von 150 mg erhalten (als weißer Feststoff, Smp. 205-206 °C).

Referenz-Beispiel 30: 2-Acetyl-1,1-(3-dimethylamino-3-phenylpentamethylen)-6-methyl-3,4-dihydro-1H-2,9-diazafluoren Citrat

120 mg (0,32 mmol) des nach Referenz-Beispiel 29 hergestellten 1,1-(3-Dimethylamino-3-phenylpentamethylen)-6-methyl-3,4-dihydro-1H-2,9-diazafluorens wurden in Pyridin (10 ml) gelöst. Danach wurde Acetanhydrid (305 µl, 3,2 mmol) zugetropft und 3 d bei RT gerührt. Zur Aufarbeitung wurde Pyridin eingeengt, der Ansatz mit Wasser (10 ml) verdünnt, mit 5M NaOH auf pH 11 eingestellt und mit EE (3 × 10 ml) extrahiert. Die vereinigten organischen Phasen wurde mit Natriumsulfat getrocknet und eingedampft, der erhaltene Rückstand wurde durch Säulenchromatographie an Kieselgel mit Methanol gereinigt. Es wurden 120 mg eines weißen Schaums erhalten, die in heißem Ethanol (10 ml) gelöst und mit einer ebenfalls heißen Lösung von Citronensäure (67 mg, 0,35 mmol) in Ethanol (1 ml) versetzt wurden. Nach dem Abkühlen auf ca. 5 °C wurde der Ansatz 4 h stehen gelassen. Der entstandene Feststoff wurde abgesaugt. Das Citrat von 2-Acetyl-1,1-(3-dimethylamino-3-phenylpentamethylen)-6-methyl-3,4-dihydro-1H-2,9-diazafluoren wurde in einer Ausbeute von 175 mg erhalten (weißer Feststoff, Smp.: 162-167 °C).

Referenz-Beispiel 31: 1,1-(3-Dimethylamino-3-phenylpentamethylen)-7-fluor-3,4-dihydro-1H-2,9-diazafluoren Citrat, polareres Diastereoisomer

4-Dimethylamino-4-phenylcyclohexanon (544 mg, 2,5 mmol) und 6-Fluortryptamin (445 mg, 2,5 mmol) wurden in trockenem Methanol (20 ml) gelöst. Zu diesem Gemisch wurde Natriumsulfat (1 g) hinzugefügt. Nach einer Reaktionszeit von 24 h wurde Methanol abdestilliert und der Rückstand in 1,2-Dichlorethan (20 ml) suspendiert. Die Reaktionsmischung wurde mit Trifluoressigsäure (2 ml) versetzt und 18 h bei RT gerührt. Zur Aufarbeitung wurde der Ansatz mit Wasser (20 ml) verdünnt, mit NaOH (5 mol/l) auf pH 11 eingestellt und mit 1,2-Dichlorethan (3 × 20 ml) extrahiert. Die vereinigten organischen Phasen wurden mit Natriumsulfat getrocknet und eingeengt. Der feste, weiße Rückstand wurde aus Methanol umkristallisiert und aus der Mutterlauge das polarere Diastereoisomer (300 mg weißer Feststoff) erhalten. Diese 300 mg wurden in heißem Ethanol (20 ml) gelöst und mit einer ebenfalls heißen Lösung von Citronensäure (193 mg, 1 mmol) in Ethanol (2 ml) versetzt. Nach dem Abkühlen auf ca. 5 °C wurde der Ansatz 4 h stehen gelassen. Der entstandene Feststoff wurde abgesaugt und getrocknet. Das Citrat von 1,1-(3-Dimethylamino-3-phenylpentamethylen)-7-fluor-3,4-dihydro-1H-2,9-diazafluoren wurde so in einer Ausbeute von 430 mg erhalten (weißer Feststoff, Smp.: 224-226 °C).

Referenz-Beispiel 32: 2-Acetyl-1,1-(3-dimethylamino-3-phenylpentamethylen)-7-fluor-3,4-dihydro-1H-2,9-diazafluoren Citrat, unpolareres Diastereoisomer

Der nach Referenz-Beispiel 31 durch Umkristallisation aus Methanol erhaltene Rückstand wurde nochmals aus EE umkristallisiert. Es wurden 330 mg des unpolareren Diastereoisomers von 1,1-(3-Dimethylamino-3-phenylpentamethylen)-7-fluor-3,4-dihydro-1H-2,9-diazafluoren als weißer Feststoff erhalten. 150 mg hiervon wurden in Pyridin (10 ml) gelöst. Danach wurde Acetanhydrid (380 µl, 4 mmol) zugetropft und 3 d bei RT gerührt. Zur Aufarbeitung wurde eingeengt, der Ansatz mit Wasser (10 ml) verdünnt, mit 5M NaOH auf pH 11 eingestellt und mit EE (3 × 10 ml) extrahiert. Die vereinigten organischen Phasen wurde mit Natriumsulfat getrocknet und eingedampft. Der erhaltene Rückstand wurde durch Säulenchromatographie an Kieselgel mit Methanol gereinigt. Die erhaltenen 154 mg 2-Acetyl-1,1-(3-dimethylamino-3-phenylpentamethylen)-7-fluor-3,4-dihydro-1H-2,9-diazafluoren wurden in heißem Ethanol (10 ml) gelöst

und mit einer ebenfalls heißen Lösung von Citronensäure (87 mg, 0,45 mmol) in Ethanol (1 ml) versetzt. Nach dem Abkühlen auf ca. 5 °C wurde der Ansatz 4 h stehen gelassen. Der entstandene Feststoff wurde abgesaugt. Das Citrat des unpolareren Diastereoisomers von 2-Acetyl-1,1-(3-dimethylamino-3-phenylpentamethylen)-7-fluor-3,4-dihydro-1H-2,9-diazafluoren wurde so in einer Ausbeute von 230 mg erhalten (weißer Feststoff, Smp. 135-140 °C).

Referenz-Beispiel 33: 1,1-(3-Dimethylamino-3-phenylpentamethylen)-3-methyl-3,4-dihydro-1H-2,9-diazafluoren Citrat

4-Dimethylamino-4-phenylcyclohexanon (435 mg, 2 mmol) und rac. 2-(1H-Indol-3-yl)-1-methylethylamin ("DL-α-Methyltryptamin", 348 mg, 2 mmol) wurden in trockenem Methanol (20 ml) gelöst. Zu diesem Gemisch wurde Natriumsulfat (1 g) hinzugefügt. Nach einer Reaktionszeit von 24 h wurde Methanol abdestilliert und der Rückstand in 1,2-Dichlorethan (20 ml) suspendiert. Die Reaktionsmischung wurde mit Trifluoressigsäure (2 ml) versetzt und 16 h bei RT gerührt. Zur Aufarbeitung wurde der Ansatz mit Wassser (20 ml) verdünnt, mit NaOH (5 mol/l) auf pH 11 eingestellt und mit 1,2-Dichlorethan (3 × 20 ml) extrahiert. Die organische Phase wurde mit Natriumsulfat getrocknet und eingeengt. Der Rückstand war ein verunreinigtes Gemisch der beiden diastereoisomeren 1,1-(3-Dimethylamino-3-phenylpentamethylen)-3-methyl-3,4-dihydro-1H-2,9-diazafluorene, die durch Umkristallisation aus Methanol gereinigt, aber nicht getrennt werden konnten (660 mg weißer Feststoff). 200 mg hiervon wurden in heißem Ethanol (15 ml) gelöst und mit einer ebenfalls heißen Lösung von Citronensäure (124 mg, 0,64 mmol) in Ethanol (2 ml) versetzt. Nach dem Abkühlen auf ca. 5 °C wurde der Ansatz 3 h stehen gelassen. Der entstandene Feststoff wurde abgesaugt und getrocknet. Das Citrat von 1,1-(3-Dimethylamino-3-phenylpentamethylen)-3-methyl-3,4-dihydro-1H-2,9-diazafluoren wurde so in einer Ausbeute von 140 mg erhalten (weißer Feststoff, Smp. 209-212 °C). Bei dieser Citratfällung wurde nur eins der beiden Diastereoisomeren erhalten.

Referenz-Beispiel 34: 1,1-(3-Dimethylamino-3-phenylpentamethylen)-6-fluor-3,4-dihydro-1H-2,9-diazafluoren Dihydrochlorid

4-Dimethylamino-4-phenylcyclohexanon (1,01 g, 4,64 mmol) und 5-Fluortryptamin (827 mg, 4,64 mmol) wurden in trockenem Methanol (40 ml) gelöst. Zu diesem Gemisch wurde Natriumsulfat (2 g) hinzugefügt. Nach einer Reaktionszeit von 24 h wurde Methanol abdestilliert und der Rückstand in 1,2-Dichlorethan (40 ml) suspendiert. Die Reaktionsmischung wurde mit Trifluoressigsäure (4 ml) versetzt und 16 h bei RT gerührt. Zur Aufarbeitung wurde der Ansatz mit Wasser (40 ml) verdünnt, mit NaOH (5 mol/l) auf pH 11 eingestellt und mit 1,2-Dichlorethan (3 × 25 ml) extrahiert. Die vereinigten organischen Phasen wurden mit Natriumsulfat getrocknet und eingeengt. Der erhaltene braune feste Rückstand wurde aus Methanol umkristallisiert, das erhaltene Gemisch aus polarerem und unpolarerem Diastereoisomer von 1,1-(3-Dimethylamino-3-phenylpentamethylen)-6-fluor-3,4-dihydro-1H-2,9-diazafluoren (110 mg weißer Feststoff) wurde in 2-Butanon (3 ml) gelöst und mit Chlortrimethylsilan (97 µl, 0,73 mmol) versetzt. Der dabei entstandene Feststoff wurde abgesaugt und getrocknet. Das erhaltene Dihydrochlorid von 1,1-(3-Dimethylamino-3-phenylpentamethylen)-6-fluor-3,4-dihydro-1H-2,9-diazafluoren (131 mg weißer Feststoff, Smp. 228-232 °C) war ein 60:40 Gemisch der beiden Diastereoisomeren.

Referenz-Beispiel 35: 1,1-(3-Dimethylamino-3-phenylpentamethylen)-6-fluor-3-methyl-1,3,4,9-tetrahydropyrano[3,4-b]indol Hemicitrat

Unter Argon wurden 4-Dimethylamino-4-phenylcyclohexanon (651 mg, 3 mmol) und 1-(5-Fluor-1 H-indol-3-yl)-propan-2-ol (579 mg, 3 mmol) in abs. DCM (20 ml) vorgelegt. Anschließend erfolgte eine sehr schnelle Zugabe von Trifluormethansulfonsäuretrimethylsilylester (0,6 ml, 3,1 mmol). Der Ansatz wurde 20 h bei RT gerührt. Zur Aufarbeitung wurde das Reaktionsgemisch mit 1 M NaOH (20 ml) versetzt und 30 min gerührt. Die organische Phase wurde abgetrennt und die verbleibende wässrige Phase mit DCM (3 × 30 ml) extrahiert. Die vereinigten organischen Phasen wurden mit Wasser (2 × 30 ml) gewaschen und über Natriumsulfat getrocknet. Der nach Abdestillieren des Lösungsmittels erhaltene feste Rückstand wurde mit Methanol (25 ml) versetzt, erwärmt und 15 h gerührt. Der in Methanol unlösliche Feststoff wurde abgesaugt. Auf diese Weise wurde eines der beiden Diastereoisomeren von1,1-(3-Dimethylamino-3-phenylpentamethylen)-6-fluor-3-methyl-1,3,4,9-tetrahydropyrano[3,4-b]indol in einer Ausbeute von 856 mg (Smp. 232-236 °C) erhalten. 800 mg hiervon wurden in heißem Ethanol (200 ml) gelöst und mit einer ebenfalls heißen Lösung von Citronensäure (600 mg, 3,12 mmol) in Ethanol (20 ml) versetzt. Nach dem Abkühlen auf ca. 5 °C war keine Kristallbildung zu beobachten. Die Lösung wurde im Vakuum eingeengt. Der Rückstand wurde mit Wasser (30 ml) versetzt. Nach Anreiben fiel ein Niederschlag aus, der nach vollständiger Kristallisation abgesaugt wurde (Hemicitrat von 1,1-(3-Dimethylamino-3-phenylpentamethylen)-6-fluor-3-methyl-1,3,4,9-tetrahydropyrano[3,4-b]indol, 807 mg weißer Feststoff, Smp. 180-182 °C).

Referenz-Beispiel 36: 3,6-Dimethyl-1,1-(3-dimethylamino-3-phenylpentamethylen)-1,3,4,9-tetrahydropyrano[3,4-b]indol Hemicitrat, unpolareres Diastereoisomer

Unter Argon wurden 4-Dimethylamino-4-phenylcyclohexanon (651 mg, 3 mmol) und 1-(5-Methyl-1 H-indol-3-yl)-propan-2-ol (567 mg, 3 mmol) in abs. DCM (20 ml) vorgelegt. Anschließend erfolgte eine sehr schnelle Zugabe von Trifluormethansulfonsäuretrimethylsilylester (0,6 ml, 3,1 mmol). Der Ansatz wurde 20 h bei RT gerührt. Zur Aufarbeitung wurde das Reaktionsgemisch mit 1 M NaOH (30 ml) versetzt und 30 min gerührt. Die organische Phase wurde abgetrennt und die verbleibende wässrige Phase mit DCM (3 × 30 ml) extrahiert. Die vereinigten organischen Phasen wurden mit Wasser (2 × 30 ml) gewaschen und über Natriumsulfat getrocknet. Der nach Abdestillieren des Lösungsmittels erhaltene feste Rückstand wurde mit Methanol (30 ml) versetzt, erwärmt und 15 h gerührt. Durch Abfiltrieren des in Methanol unlöslichen Feststoffs wurde das unpolarere der beiden möglichen racemischen Diastereoisomeren von 3,6-Dimethyl-1,1-(3-dimethylamino-3-phenylpentamethylen)-1,3,4,9-tetrahydropyrano[3,4-b]indol erhalten (840 mg, Smp. 292-296 °C). 600 mg hiervon wurden in heißem Ethanol (300 ml) gelöst und mit einer ebenfalls heißen Lösung von Citronensäure (400 mg, 2,08 mmol) in Ethanol (20 ml) versetzt. Bereits in der Siedehitze begann ein Feststoff auszufallen. Zur Vervollständigung der Kristallisation wurde die Lösung 15 h bei ca. 5 °C belassen. Der Niederschlag wurde anschließend abgetrennt und getrocknet. Das Hemicitrat des unpolareren Diastereoisomers von 3,6-Dimethyl-1,1-(3-dimethylamino-3-phenylpentamethylen)-1,3,4,9-tetrahydropyrano-[3,4-b]indol konnte so in einer Ausbeute von 630 mg erhalten werden (weißer Feststoff, Smp. 258-276 °C).

Referenz-Beispiel 37: 3,6-Dimethyl-1,1-(3-dimethylamino-3-phenylpentamethylen)-1,3,4,9-tetrahydropyrano[3,4-b]indol Citrat, polareres Diastereoisomer

4-Dimethylamino-4-phenylcyclohexanon (217 mg, 1 mmol) und 1-(5-Methyl-1 H-indol-3-yl)-propan-2-ol (189 mg, 1 mmol) wurden in konz. Essigsäure (4 ml) gelöst. Zu diesem Gemisch wurde Phosphorsäure (1 ml, 85 m%) langsam zugetropft. Nach einer Reaktionszeit von 60 min entstand eine rote Lösung. Es wurde 20 h bei RT gerührt. Zur Aufarbeitung wurde der Ansatz mit Wasser (20 ml) verdünnt, mit 5M NaOH auf pH 11 eingestellt und mit DCM (3 × 20 ml) extrahiert. Die organische Phase wurde mit Natriumsulfat getrocknet und eingedampft. Der Rückstand (370 mg weißer Feststoff) wurde in heißem Ethanol (20 ml) suspendiert und mit einer ebenfalls heißen Lösung von Citronensäure (385 mg, 2 mmol) in Ethanol (10 ml) versetzt. Der Rückstand ging dabei vollständig in Lösung, fiel aber bei Abkühlen auf ca. 5 °C wieder aus. Das Citrat des polareren Diastereoisomers von 3,6-Dimethyl-1,1-(3-dimethylamino-3-phenylpentamethylen)-1,3,4,9-tetrahydropyrano[3,4-b]indol wurde abgesaugt und getrocknet (690 mg weißer Feststoff, Smp. 162-168 °C).

Referenz-Beispiel 38: 1,1-(3-Dimethylamino-3-phenylpentamethylen)-3-methyl-9-phenyl-1,3,4,9-tetrahydropyrano[3,4-b]indol Citrat

4-Dimethylamino-4-phenylcyclohexanon (435 mg, 2 mmol) und 2-(1-Phenyl-1 H-indol-3-yl)-ethanol (503 mg, 2 mmol) wurden in konz. Essigsäure (8 ml) gelöst. Zu diesem Gemisch wurde Phosphorsäure (2 ml, 85 m%) langsam zugetropft. Nach einer Reaktionszeit von 30 min entstand eine rote Lösung. Es wurde 20 h bei RT gerührt. Zur Aufarbeitung wurde der Ansatz mit Wasser (40 ml) verdünnt, mit 5M NaOH auf pH 11 eingestellt und mit DCM (3 × 30 ml) extrahiert. Die organische Phase wurde mit Natriumsulfat getrocknet und eingedampft. Der Rückstand enthielt nur eines der beiden möglichen racemischen Diastereoisomeren des Zielprodukts und konnte in einer Ausbeute von 900 mg als weißer Feststoff erhalten werden. Diese 900 mg wurden in heißem Ethanol (50 ml) suspendiert und mit einer ebenfalls heißen Lösung von Citronensäure (770 mg, 4 mmol) in Ethanol (15 ml) versetzt. Der beim Abkühlen auf ca. 5 °C ausgefallene Feststoff wurde abgesaugt und getrocknet. Das Citrat von 1,1-(3-Dimethylamino-3-phenylpentamethylen)-3-methyl-9-phenyl-1,3,4,9-tetrahydropyrano[3,4-b]indol konnte so in einer Ausbeute von 1,2 g als weißer Feststoff erhalten werden (Smp. 253-256 °C).

Referenz-Beispiel 39: 1,1-(3-Dimethylamino-3-(4-fluorphenyl)pentamethylen)-1,3,4,9-tetrahydro-2-thia-9-azafluoren Methansulfonat

Unter Argon wurden 4-Dimethylamino-4-(4-fluorphenyl)cyclohexanon (353 mg, 1,5 mmol) und 2-(1H-Indol-3-yl)ethanthiol (266 mg, 1,5 mmol) in abs. DCM (10 ml) vorgelegt. Anschließend erfolgte die Zugabe des Methansulfonsäuretrimethylsilylesters (254 µl, 1,65 mmol). Nach 20 h Rühren bei RT war kein Niederschlag sichtbar. Der Reaktionsmischung wurde nochmals Methansulfonsäuretrimethylsilylester (254 µl, 1,65 mmol) zugesetzt. Danach rührte der Ansatz 3 d bei RT. Zur Aufarbeitung wurde das ausgefallene Methansulfonat abgesaugt, mit DCM (3 × 1 ml) und Diethylether (3 × 3 ml) gewaschen. Das Methansulfonat eines der beiden möglichen Diastereoisomeren von 1,1-(3-Dimethylamino-3-(4-fluorphenyl)pentamethylen)-1,3,4,9-tetrahydro-2-thia-9-azafluoren wurde in einer Ausbeute von 550 mg als weißer

Feststoff erhalten (Smp. 245-250 °C).

Referenz-Beispiel 40: 1,1-(3-Dimethylamino-3-(3-fluorphenyl)pentamethylen)-1,3,4,9-tetrahydro-2-thia-9-azafluoren Methansulfonat

Unter Argon wurden 4-Dimethylamino-4-(3-fluorphenyl)cyclohexanon (353 mg, 1,5 mmol) und 2-(1H-Indol-3-yl)ethanthiol (266 mg, 1,5 mmol) in abs. DCM (10 ml) vorgelegt. Anschließend erfolgte die Zugabe von Methansulfonsäure (195 μl, 3 mmol). Nach 2 h Rühren bei RT war die Reaktionsmischung eine klare Lösung. Nach weiteren 16 h Rühren bei RT war viel weißer Niederschlag ausgefallen. Die Suspension wurde mit DCM (5 ml) verdünnt. Der Niederschlag wurde abgesaugt, mit DCM (3 × 1 ml) gewaschen und getrocknet. Das Methansulfonat eines der beiden möglichen Diastereoisomeren von 1,1-(3-Dimethylamino-3-(3-fluorphenyl)pentamethylen)-1,3,4,9-tetrahydro-2-thia-9-azafluoren wurde als cremefarbener Feststoff erhalten (695 mg, Smp. 258-260 °C).

Referenz-Beispiel 41: 1,1-(3-Dimethylamino-3-phenylpentamethylen)-3,4-dihydro-1H-9-oxa-2-thiafluoren Citrat

Unter Argon wurden 4-Dimethylamino-4-phenylcyclohexanon (2,06 g, 9,5 mmol) und 2-(Benzofuran-3-yl)ethanthiol (1,70 g, Rohprodukt, enthält laut NMR ca. 80 % gewünschtes Thiol) in abs. DCM (25 ml) vorgelegt. Anschließend erfolgte die Zugabe von Methansulfonsäure (680 μl, 10,45 mmol). Der Ansatz wurde 4 d bei RT gerührt. Zur Aufarbeitung wurde der Ansatz mit Wasser (15 ml) versetzt. Die wässrige Phase wurde abgetrennt und mit DCM (3 × 20 ml) extrahiert. Die vereinigten organischen Phasen wurden mit 2M Schwefelsäure gewaschen und eingeengt. Der klebrige, gelbe Rückstand wurde mit Diethylether (3 × 10 ml) gewaschen und anschließend mit 2M NaOH (20 ml) versetzt. Das erhaltene Gemisch wurde mit Diethylether (3 × 15 ml) extrahiert. Die etherische Phase wurde über Natriumsulfat getrocknet und eingeengt. Aus dem erhaltenen Rückstand wurde eines der beiden möglichen Diastereoisomeren des Zielprodukts durch Säulenchromatographie an Kieselgel mit EE/Ethanol im Volumenverhältnis 9 : 1 isoliert (112 mg weißer Feststoff, Smp. 160-165 °C). Diese 112 mg wurden in siedendem Ethanol (12ml) gelöst, mit einer ethanolischen Lösung (2 ml) von Citronensäure (62 mg, 0,324 mmol) versetzt und 10 min gerührt. Nach Abkühlung wurde das Lösungsmittel auf ca. 5 ml eingeengt und auf ca. 5 °C gebracht. Der nach ca. 6 h ausgefallene weiße Niederschlag wurde abgetrennt und getrocknet. Es wurden 112 mg des Citrats von 1,1-(3-Dimethylamino-3-phenylpentamethylen)-3,4-dihydro-1 H-9-oxa-2-thiafluoren erhalten (weißer Feststoff, Smp. 207-209 °C).

Referenz-Beispiel 42: 1,1-(3-Dimethylamino-3-phenylpentamethylen)-1,2,3,4-tetrahydro-benzo[4,5]furo[2,3-c]pyridin Citrat

2-(Benzofuran-3-yl)ethylamin (0,74 g, 4,6 mmol) und 4-Dimethylamino-4-phenylcyclohexanon (1,01 g, 4,6 mmol) wurden in Methanol (35 ml) gelöst und 24 h bei RT gerührt. Danach wurde der Ansatz zur Trockne eingedampft. Der Rückstand wurde in trockenem 1,2-Dichlorethan (40 ml) suspendiert und mit Trifluoressigsäure (4 ml) versetzt. Das Gemisch wurde 24 h bei RT gerührt. Zur Aufarbeitung wurde mit 5M NaOH ein pH von 11 eingestellt. Nach anschließender Zugabe von EE (20 ml) fiel ein Diastereoisomer des Zielprodukts als weißer Niederschlag aus. Nach 15 min wurde der Niederschlag abgesaugt und getrocknet (867 mg, Smp. 193-196 °C). 400 mg hiervon wurden in heißem Ethanol (9 ml) gelöst und mit einer ebenfalls heißen, ethanolischen Lösung von Citronensäure (212 mg, 1,1 mmol in 3 ml Ethanol) versetzt. Dabei fiel sofort ein weißer Niederschlag aus. Zur Vervollständigung der Fällung wurde der Ansatz 4 h bei ca. 5 °C belassen. Der entstandene Feststoff wurde abgesaugt. Das Citrat von 1,1-(3-Dimethylamino-3-phenylpentamethylen)-1,2,3,4-tetrahydro-benzo[4,5]furo[2,3-c]pyridin wurde so in einer Ausbeute von 400 mg erhalten (weißer Feststoff, Smp. 222-224 °C).

Referenz-Beispiel 43: 6,6-(3-Dimethylamino-3-phenylpentamethylen)-1,2,3,4,4a,6,7,11c-octahydro-5-oxa-7-azabenzo[c]fluoren Citrat

Unter Argon wurden 4-Dimethylamino-4-phenylcyclohexanon (261 mg, 1,2 mmol) und rac. 2-(1H-Indol-3-yl)-cyclohexanol (260 mg, 1,2 mmol) in abs. DCM (20 ml) vorgelegt. Anschließend erfolgte eine schnelle Zugabe von Trifluormethansulfonsäuretrimethylsilylester (0,25 ml, 1,3 mmol). Der Ansatz wurde 20 h bei RT gerührt. Zur Aufarbeitung wurde das Reaktionsgemisch mit 1 M NaOH (20 ml) versetzt und 30 min gerührt. Die organische Phase wurde abgetrennt und die verbleibende wässrige Phase mit DCM (3 × 30 ml) extrahiert. Die vereinigten organischen Phasen wurden mit Wasser (2 × 30 ml) gewaschen, über Natriumsulfat getrocknet und bis zur Trockne eingeengt. Der nach Zugabe von Methanol (ca. 25 ml) aus dem Rückstand gebildete Feststoff bestand laut NMR aus den beiden zu erwartenden diastereoisomeren Zielprodukten. Das Gemisch wurde zur Vervollständigung der Ausfällung 2 h auf ca. 5 °C gekühlt. Anschließend wurde der Feststoff abgesaugt und getrocknet. Auf diese Weise wurde das Diastereoisomerengemisch des Zielproduktes in einer Ausbeute von 277 mg mit einem Smp. von 150-170 °C gewonnnen. 250 mg hiervon wurden in

heißem Ethanol (200 ml) gelöst und mit einer ebenfalls heißen Lösung von Citronensäure (192 mg, 1 mmol) in Ethanol (20 ml) versetzt. Auch nach Abkühlung der Reaktionsmischung auf ca. 5 °C war keine Kristallbildung zu beobachten. Die Lösung wurde deshalb im Vakuum auf ca. 30 ml eingeengt und 3 d bei ca. 5 °C belassen. Es wurden 190 mg des Citrats eines ca. 60:40 Gemisches der beiden diastereoisomeren 6,6-(3-Dimethylamino-3-phenylpentamethylen)-1,2,3,4,4a,6,7,11c-octahydro-5-oxa-7-azabenzo[c]fluorene erhalten (weißer Feststoff, Smp. 184-192 °C).

Referenz-Beispiel 44: 1,1-(3-Dimethylamino-3-phenylpentamethylen)-6-brom-1,3,4,9-tetrahydropyrano[3,4-b]indol Hemicitrat

4-Dimethylamino-4-phenylcyclohexanon (326 mg, 1,5 mmol) und 5-Brom-3-(2-trimethylsilanyloxy-ethyl)-1 H-indol (468 mg, 1,5 mmol) wurden in DCM (50 ml) vorgelegt. Anschließend erfolgte eine schnelle Zugabe von Trifluormethansulfonsäure (0,145 ml, 1,51 mmol). Der Ansatz wurde 15 h bei RT gerührt. Zur Aufarbeitung wurde das Reaktionsgemisch mit 2M NaOH (10 ml) versetzt und 30 min gerührt. Die organische Phase wurde abgetrennt und die wässrige Phase mit DCM (3 × 30 ml) extrahiert. Die vereinigten organischen Phasen wurden mit Wasser (2 × 30 ml) gewaschen und über Natriumsulfat getrocknet. Der nach Abdestillieren des Lösungsmittels erhaltene feste Rückstand wurde mit Methanol (30 ml) versetzt, erwärmt und 15 h bei RT gerührt. Der in Methanol suspendierte Feststoff wurde abgesaugt. Eines der beiden Diastereoisomeren des Zielproduktes wurde so in einer Ausbeute von 583 mg (Smp. 271-281 °C) erhalten. 550 mg hiervon wurden in heißem Ethanol (300 ml) gelöst und mit einer ebenfalls heißen ethanolischen Citronensäurelösung (385 mg, 2 mmol in 20 ml) versetzt. Bereits in der Siedehitze fiel ein kristalliner Feststoff aus. Zur Vervollständigung der Kristallisation wurde der Ansatz 12 h bei 5 °C belassen. Der entstandene Feststoff wurde abgesaugt. Das Hemicitrat von 1,1-(3-Dimethylamino-3-phenylpentamethylen)-6-brom-1,3,4,9-tetrahydropyrano[3,4-b]indol wurde so in einer Ausbeute von 510 mg erhalten (weißer Feststoff, Smp. 262-267 °C).

Referenz-Beispiel 45: 1,1-(3-Dimethylamino-3-phenylpentamethylen)-1,3,4,9-tetrahydropyrano[3,4-b]indo1-6-ol Citrat

4-Dimethylamino-4-phenylcyclohexanon (490 mg, 2,26 mmol) und 3-(2-Hydroxy-ethyl)-1H-indol-5-ol (400 mg, 2,26 mmol) wurden in DCM (150 ml) vorgelegt. Anschließend erfolgte eine schnelle Zugabe von Trifluormethansulfonsäuretrimethylsilylester (0,45 ml, 2,3 mmol). Der Ansatz wurde 3 d bei RT gerührt. Zur Aufarbeitung wurde das Reaktionsgemisch mit 1 M NaOH (30 ml) versetzt und 30 min gerührt. Das Gemisch wurde filtriert, die organische Phase abgetrennt und die verbliebene wässrige Phase mit DCM (3 × 60 ml) extrahiert. Die vereinigten organischen Phasen wurden mit Wasser (2 × 30 ml) gewaschen und über Natriumsulfat getrocknet. Der nach Abdestillieren des Lösungsmittels erhaltene feste Rückstand wurde mit Methanol (50 ml) versetzt. Die entstandene klare Lösung wurde auf ca. 10 ml eingeengt und 2 h bei 5 °C stehen gelassen. Der aus Methanol ausgefallene Feststoff wurde abgesaugt. Es wurde eines der beiden
diastereoisomeren Zielprodukte erhalten (180 mg, Smp. 252-257 °C). 160 mg hiervon wurden in heißem Ethanol (20 ml) gelöst und mit einer ebenfalls heißen ethanolischen Citronensäurelösung (150 mg, 0,78 mmol in 10 ml) versetzt. Bereits in der Siedehitze fiel ein kristalliner Feststoff aus. Zur Vervollständigung der Kristallisation wurde der Ansatz 20 h bei 5 °C belassen. Der entstandene Feststoff wurde abgesaugt. Das Citrat von 1,1-(3-Dimethylamino-3-phenylpentamethylen)-1,3,4,9-tetrahydropyrano[3,4-b]indol-6-ol wurde so in einer Ausbeute von 125 mg erhalten (weißer Feststoff, Smp. 248-254 °C).

Referenz-Beispiel 46: (3S)-1,1-(3-Dimethylamino-3-phenylpentamethylen)-3,4-dihydro-3-methoxycarbonyl-1 H-2,9-diazafluoren Citrat

4-Dimethylamino-4-phenylcyclohexanon (434,8 mg, 2 mmol) und L-Tryptophanmethylester ((2S)-2-Amino-3-(1H-indol-3-yl)propionsäuremethylester, 436,5 mg, 2 mmol) wurden in trockenem Methanol (20 ml) gelöst. Nach einer Reaktionszeit von 24 h wurde Methanol abdestilliert und der gelbe, ölige Rückstand in 1,2-Dichlorethan (20 ml) suspendiert. Die Reaktionsmischung wurde mit Trifluoressigsäure (2 ml) versetzt und 18 h bei RT gerührt. Zur Aufarbeitung wurde der Ansatz mit Wasser (20 ml) verdünnt und mit NaOH (5 mol/l) auf pH 11 eingestellt. Nach Zugabe von EE (20 ml) fiel ein weißer Feststoff aus, der abgesaugt wurde. Der Feststoff wurde mit Wasser (3 × 5 ml) gewaschen und getrocknet. Es handelte sich dabei um ein Gemisch der Diastereoisomeren des Zielprodukts (70 % unpolar: 30 % polar), das als weißer Feststoff in einer Ausbeute von 600 mg erhalten werden konnte. Diese 600 mg wurden in heißem Ethanol (30 ml) gelöst und mit einer ebenfalls heißen Lösung von Citronensäure (276 mg, 1,44 mmol) in Ethanol (5 ml) versetzt. Nach dem Abkühlen auf ca. 5 °C wurde der Ansatz 4 h stehen gelassen. Der entstandene Feststoff wurde abgesaugt. Das Citrat von (3S)-1,1-(3-Dimethylamino-3-phenylpentamethylen)-3,4-dihydro-3-methoxycarbonyl-1 H-2,9-diazafluoren konnte so als ca. 70:30 Gemisch des unpolareren und polareren Diastereoisomers in einer Ausbeute von 875 mg erhalten werden (weißer Feststoff, Smp. 193-196 °C).

Referenz-Beispiel 47: (3S)-1,1-(3-Dimethylamino-3-phenylpentamethylen)-3,4-dihydro-1H-2,9-diazafluoren-3-methanol Citrat

4-Dimethylamino-4-phenylcyclohexanon (434,8 mg, 2 mmol) und L-Tryptophanol ((2S)-2-Amino-3-(1H-indol-3-yl)-propan-1-ol, 380,5 mg, 2 mmol) wurden in trockenem Methanol (20 ml) gelöst. Nach einer Reaktionszeit von 24 h wurde Methanol abdestilliert und der gelbe, ölige Rückstand in 1,2-Dichlorethan (20 ml) suspendiert. Die Reaktionsmischung wurde mit Trifluoressigsäure (2 ml) versetzt und 18 h bei RT gerührt. Zur Aufarbeitung wurde der Ansatz mit Wasser (20 ml) verdünnt und mit NaOH (5 mol/l) auf pH 11 eingestellt. Nach Zugabe von EE (20 ml) fiel ein weißer Feststoff aus, der abgesaugt wurde. Der Feststoff wurde mit Wasser (3 × 5 ml) gewaschen und getrocknet. Es handelte sich dabei um ein Gemisch der Diastereoisomeren des Zielprodukts (30 % unpolar : 70 % polar), das als weißer Feststoff mit einer Ausbeute von 700 mg erhalten werden konnte. Diese 700 mg wurden in heißem Ethanol (40 ml) gelöst und mit einer ebenfalls heißen Lösung von Citronensäure (346 mg, 1,8 mmol) in Ethanol (5 ml) versetzt. Nach dem Abkühlen auf ca. 5 °C wurde der Ansatz 4 h stehen gelassen. Der entstandene Feststoff wurde abgesaugt. Das Citrat von (3S)-1,1-(3-Dimethylamino-3-phenylpentamethylen)-3,4-dihydro-1H-2,9-diazafluoren-3-methanol konnte so in einer Ausbeute von 1,0 g als ca. 30:70 Gemisch des unpolareren und polareren Diastereoisomers erhalten werden (weißer Feststoff, Smp. 265-270 °C).

Referenz-Beispiel 48: 1,1-(3-Dimethylamino-3-phenylethyl-pentamethylen)-3,4-dihydro-1H-2,9-diazafluoren

4-Dimethylamino-4-phenethyl-cyclohexanon (5 g, 20 mmol) und Tryptamin (3,2 g, 20 mmol) wurden in trockenem Methanol (200 ml) gelöst. Nach einer Reaktionszeit von 24 h wurde Methanol abdestilliert und der gelbe, ölige Rückstand in 1,2-Dichlorethan (200 ml) suspendiert. Die Reaktionsmischung wurde mit Trifluoressigsäure (20 ml) versetzt und 2 h bei RT gerührt. Zur Aufarbeitung wurde der Ansatz mit Wasser (100 ml) verdünnt und mit NaOH (5 mol/l) auf pH 11 eingestellt. Nach Zugabe von EE (50 ml) fiel ein weißer Feststoff aus, der abgesaugt wurde. Der Feststoff wurde mit Wasser (3 × 25 ml) gewaschen und über Natriumsulfat getrocknet. Es handelte sich dabei um ein Gemisch der Diastereoisomeren des Zielprodukts (10 % unpolar : 90 % polar), das als weißer Feststoff (Smp. 225-230 °C) in einer Ausbeute von 4,42 g erhalten wurde.

Referenz-Beispiel 49: 1,1-(3-Methylamino-3-phenylpentamethylen)-6-fluor-1,3,4,9-tetrahydropyrano[3,4-b]indol Hemicitrat

4-Methylamino-4-phenyl-cyclohexanon (406 mg, 2 mmol) und 5-Fluor-3-(2-trimethylsilanyloxyethyl)-1 H-indol (503 mg, 2 mmol) wurden in DCM (50 ml) vorgelegt. Anschließend erfolgte eine schnelle Zugabe von Trifluormethansulfonsäure (0,18 ml, 2,03 mmol). Der Ansatz wurde 20 h bei RT gerührt Zur Aufarbeitung wurde das Reaktionsgemisch mit 2M NaOH (20 ml) versetzt und 20 min gerührt. Die organische Phase wurde abgetrennt und die verbleibende wässrige Phase mit DCM (3 × 30 ml) extrahiert. Die vereinigten organischen Phasen wurden mit Wasser (2 × 30 ml) gewaschen und über Natriumsulfat getrocknet. Der nach Abdestillieren des Lösungsmittels erhaltene feste Rückstand wurde mit Methanol (25 ml) versetzt, erwärmt und anschließend 4 h bei RT gerührt. Der im Methanol suspendierte Feststoff wurde abgesaugt. Auf diese Weise wurde eins der beiden möglichen Diastereoisomeren des Zielprodukts in einer Ausbeute von 490 mg erhalten (Smp. 248-252 °C). 450 mg hiervon wurden in heißem Ethanol (50 ml) gelöst und mit einer ebenfalls heißen ethanolischen Citronensäurelösung (384 mg, 2 mmol in 10 ml) versetzt. Bereits in der Siedehitze fiel ein kristalliner Feststoff aus. Zur Vervollständigung der Kristallisation wurde der Ansatz 15 h lang bei ca. 5 °C belassen. Der entstandene Feststoff wurde abgesaugt. Das Hemicitrat von 1,1-(3-Methylamino-3-phenylpentamethylen)-6-fluor-1,3,4,9-tetrahydropyrano[3,4-b]indol wurde so in einer Ausbeute von 550 mg erhalten (weißer Feststoff, Smp. 226-228 °C).

Referenz-Beispiel 50: 1,1-(3-Dimethylamino-3-(4-fluorphenyl)pentamethylen)-3,4-dihydro-1H-2,9-dithiafluoren Methansulfonat

Unter Argon wurden 4-Dimethylamino-4-(4-fluorphenyl)cyclohexanon (353 mg, 1,5 mmol) und 2-(Benzo[b]thiophen-3-yl)ethanthiol (297 mg in 11,5 ml Lösung, 1,5 mmol) in absolutem DCM (20 ml) vorgelegt. Anschließend erfolgte die Zugabe von Methansulfonsäure (194,5 μl, 3,0 mmol). Der Ansatz wurde 24 h bei RT gerührt. Das Reaktionsgemisch wurde mit weiteren 100 μl Methansulfonsäure versetzt und erneut 20 h bei RT gerührt. Zur Aufarbeitung wurde das klare Reaktionsgemisch mit Wasser (4 ml) versetzt und 1 h gerührt. Dabei fiel ein Niederschlag aus. Der Niederschlag wurde abgesaugt, mit Wasser (2 × 1 ml) und Diethylether (2 × 2 ml) gewaschen und getrocknet. Der weiße Feststoff war das Methansulfonat von 1,1-(3-Dimethylamino-3-(4-fluorphenyl)pentamethylen)-3,4-dihydro-1H-2,9-dithiafluoren (262 mg, Smp. 256-258 °C).

Referenz-Beispiel 51: 1,1-(3-Dimethylamino-3-phenylpentamethylen)-3,4-dihydro-1H-2,9-dithiafluoren Citrat

Unter Argon wurden 4-Dimethylamino-4-phenethyl-cyclohexanon (326 mg, 1,5 mmol) zusammen mit 2-(Benzo[b]thiophen-3-yl)ethanthiol (297 mg, 1,5 mmol) in absolutem Dichlormethan (20 ml) vorgelegt und Methansulfonsäure zugegeben (195 μl, 3,0 mmol). Der Ansatz wurde 24 h bei RT gerührt. Das Reaktionsgemisch wurde mit weiteren 100 μl Methansulfonsäure versetzt und erneut 20 h bei RT gerührt. Zur Aufarbeitung wurde das klare Reaktionsgemisch mit Wasser (5 ml) versetzt und 1 h gerührt. Dann wurde mit 1 M NaOH auf pH 11 eingestellt und mit DCM (5 ml) verdünnt. Die Phasen wurden getrennt. Die wässrige Phase wurde mit DCM (3 × 10 ml) extrahiert. Die Extrakte wurden vereinigt, einmal mit gesättigter NaCl-Lösung gewaschen und über Natriumsulfat getrocknet. Der Rückstand nach dem Abdestillieren des DCMs war ein gelber Feststoff. Zur Reinigung wurde dieser mit Ethanol (5 ml) versetzt und 10 min unter Rückfluss gekocht. Nach Abkühlung auf RT wurde 24 h gerührt. Der vorhandene Niederschlag wurde abgesaugt, mit kaltem Ethanol (3 × 2 ml) gewaschen und getrocknet. Eine der zwei möglichen freien Basen des Zielproduktes (335 mg, beige, 57 %) wurde so mit einem Smp. von 210-214 °C erhalten. 120 mg hiervon wurden in heißem Ethanol (40 ml) gelöst, mit Citronensäure (59,2 mg, 0,308 mmol, gelöst in 1 ml Ethanol) versetzt und 10 min bei 65 °C gerührt. Nach dem Abkühlen auf RT wurde der Ansatz 20 h gerührt. Da kein Niederschlag ausgefallen war, wurde Ethanol bis auf 2 ml eingeengt und langsam mit Diethylether (30 ml) versetzt. Der entstandene Feststoff wurde abgesaugt, mit Diethylether (3 × 2 ml) gewaschen und anschließend getrocknet. Es wurden 152 mg des Citrats von 1,1-(3-Dimethylamino-3-phenylpentamethylen)-3,4-dihydro-1H-2,9-dithiafluoren als weißer Feststoff erhalten (Smp. 125-128 °C).

Referenz-Beispiel 52: 1,1-(3-Dimethylamino-3-phenylpentamethylen)-2-oxo-1,3,4,9-tetrahydro-2-thia-9-azafluoren Citrat

1,1-(3-Dimethylamino-3-phenylpentamethylen)-3,4-dihydro-1H-2,9-dithiafluoren (200 mg, 0,53 mmol) wurde in Eisessig (3 ml) suspendiert, unter Rühren 30-proz. Wasserstoffperoxyd (200 μl) tropfenweise zugesetzt und 2 h bei RT gerührt. Zur Aufarbeitung wurde das Reaktionsgemisch mit 5 ml Wasser versetzt und mit 5M NaOH alkalisch gemacht. Dabei entstand eine Suspension, die sich auch nach der Zugabe von EE (50 ml) nicht vollständig löste. Der Niederschlag wurde abgesaugt, mit Wasser (2 × 1 ml) gewaschen und verworfen. Die wässrige Mutterlauge wurde mit 5M NaOH auf pH 11 eingestellt. Dabei fiel ein weißer Niederschlag aus. Der Feststoff wurde abgesaugt, mit Wasser (1 × 2 ml) und Ether (3 × 1 ml) gewaschen und getrocknet. Es wurden 76 mg 1,1-(3-Dimethylamino-3-phenylpentamethylen)-2-oxo-1,3,4,9-tetrahydro-2-thia-9-azafluoren erhalten (Smp. 188-192 °C). 61 mg, hiervon wurden in heißem Ethanol (8 ml) gelöst, mit Citronensäure (32,8 mg, 0,17 mmol) versetzt und 10 min bei 65 °C gerührt. Nach dem Abkühlen auf RT wurde der Ansatz 20 h gerührt. Weil nur wenig weißer Niederschlag ausgefallen war, wurde Ethanol bis auf 2 ml eingeengt und langsam mit Ether (30 ml) versetzt. Der entstandene Feststoff wurde abgesaugt, mit Ether (3 × 2 ml) gewaschen und anschließend getrocknet. Es wurden 74 mg des Citrats von 1,1-(3-Dimethylamino-3-phenylpentamethylen)-2-oxo-1,3,4,9-tetrahydro-2-thia-9-azafluoren erhalten (weißer Feststoff, Smp. 162-167 °C).

Referenz-Beispiel 53: 1,1-(3-Dimethylamino-3-benzylpentamethylen)-3,4-dihydro-1H-2,9-dithiafluoren

4-Benzyl-4-dimethylaminocyclohexanon (3,47 g, 15 mmol) und Tryptamin (2,40 g, 15 mmol) wurden unter Argon in trockenem Methanol (150 ml) gelöst. Nach einer Reaktionszeit von 24 h wurde Methanol abdestilliert und der Rückstand in 1,2-Dichlorethan (150 ml) suspendiert. Die Reaktionsmischung wurde mit Trifluoressigsäure (15 ml) versetzt und 2 h bei RT gerührt. Zur Aufarbeitung wurde der Ansatz mit Wasser (100 ml) versetzt und mit NaOH (5 mol/l) auf pH 11 eingestellt. Nach Zugabe von EE (70 ml) fiel beim Rühren ein weißer Feststoff aus, der über eine Fritte abgesaugt wurde. Der Feststoff wurde mit Wasser (5 × 20 ml) gewaschen und getrocknet. Es handelte sich dabei um das Diastereoisomerengemisch von 1,1-(3-Dimethylamino-3-benzylpentamethylen)-3,4-dihydro-1H-2,9-dithiafluoren (15 % unpolar: 85 % polar), welches als weißer Feststoff mit einem Smp. von 195-200 °C und einer Ausbeute von 3,0 g erhalten wurde.

Herstellung verwendeter Bausteine:

Trimethylsilylether - Allgemeine Vorschrift am Beispiel von 3-(2-Trimethylsilanyloxyethyl)-1 H-indol

Tryptophol (4,83 g, 30 mmol) wurde in trockenem THF (80 ml) vorgelegt und bei RT zunächst mit Hexamethyldisilazan (30 ml, 141 mmol) und anschließend mit Chlortrimethylsilan (8 ml, 62,6 mmol) versetzt. Der Ansatz wurde 20 h bei RT gerührt. Das THF wurde abdestilliert und der Rückstand bis zur basischen Reaktion mit gesättigter Natriumhydrogencarbonatlösung versetzt. Die wässrige Lösung wurde mit Ether extrahiert. Die organische Phase wurde mit Wasser gewaschen und über Natriumsulfat getrocknet. Nach Entfernung des Lösungsmittels wurde der Trimethylsilylether in einer Ausbeute von 6,99 g als kristalliner Feststoff erhalten (Smp. 47 - 48 °C).

2-(Benzofuran-3-yl)ethanthiol

Triphenylphosphandibromid (5,52 g, 14,4 mmol) wurde in abs. Acetonitril (15 ml) unter Argon suspendiert, im Wasserbad auf 19 °C gebracht und innerhalb von 15 min mit 2-(Benzofuran-3-yl)ethanol (2,11 g, 13,1 mmol) in abs. Acetonitril (7 ml) versetzt. Während der Zugabe wurde die Temperatur des Reaktionsgemisches zwischen 19 und 21 °C gehalten. Anschließend wurde der Ansatz 12 h ohne weitere Kühlung stehen gelassen. Das Reaktionsgemisch wurde filtriert, das erhaltene Filtrat eingeengt. Der erhaltene Rückstand wurde in Cyclohexan (20 ml) aufgenommen und über eine etwa 3 cm dicke Kieselgelschicht (15 g) filtriert. Das Kieselgel wurde mit Cyclohexan (5 × 20 ml) gewaschen und das erhaltene Filtrat eingeengt. Es wurden 2,47 g 3-(2-Bromethyl)benzofuran als gelbliches Öl erhalten.

Natriumthiosulfat Pentahydrat (5,44 g, 21,9 mmol) wurde in Wasser (22 ml) gelöst und innerhalb von 10 min unter Rühren mit dem in Ethanol (40 ml) gelösten 3-(2-Bromethyl)benzofuran (2,90 g, 12,9 mmol) versetzt. Die Reaktionsmischung wurde anschließend 4 h unter Rückfluss gekocht. Zur Aufarbeitung wurde das im Lösungsmittelgemisch enthaltene Ethanol im Vakuum abdestilliert. Der wässrige Rückstand wurde mit Diethylether (3 × 20 ml) extrahiert, die organische Phase mit Wasser (2 × 20 ml) gewaschen. Die vereinigten wässrigen Phasen wurden am Rotationsverdampfer eingedampft. Der so erhaltene weiß-gelbliche Rückstand (3,63 g) besteht aus dem Natriumsalz von Thioschwefelsäure-S-[2-(benzofuran-3-yl)ethyl]ester ("Buntesalz") enthält eine nicht definierte Restmenge Wasser. Die nachfolgende Umsetzung zum Thiol erfolgte ohne weitere Reinigung.

Unter Argon wurden die erhaltenen 3,63 g des Natriumsalzes von Thioschwefelsäure-S-[2-(benzofuran-3-yl)ethyl]ester in 50 m% Phosphorsäure (60 ml) suspendiert. Das erhaltene Reaktionsgemisch wurde anschließend mit Diethylether (75 ml) überschichtet und unter kräftigem Rühren unter Rückfluss (7 h) erhitzt, bis in der wässrigen Phase kein Feststoff mehr zu beobachten war. Nach Abkühlung wurden die beiden Phasen getrennt und die wässrige Phase mit Diethylether (4 × 15 ml) extrahiert. Die vereinigten etherischen Phasen wurden mit Wasser (2 × 10 ml) gewaschen und über Natriumsulfat getrocknet. Der nach Entfernung des Diethylethers erhaltene Rückstand (gelbliches Öl, 1,71 g) enthielt laut NMR ca. 80 % des gewünschten 2-(Benzofuran-3-yl)ethanthiols, das ohne weitere Reinigung eingesetzt wurde.

3-(2-Hydroxy-ethyl)-1H-indol-5-ol (5-Hydroxy-tryptophol)

Unter Argon wurde 5-Hydroxyindol-3-essigsäure (1,91 g, 10 mmol) in DCM (40 ml) vorgelegt, auf - 78 °C gekühlt und unter Rühren innerhalb von 20 min mit Diisopropylaluminiumhydrid (0,2 M in Toluol, 40 ml, 48 mmol) versetzt. Nach beendeter Zugabe des Reduktionsmittels wurde der Ansatz innerhalb von 5 h auf RT kommen gelassen und anschließend eine weitere Stunde bei RT belassen. Zur Aufarbeitung wurde das Reaktionsgemisch vorsichtig mit Methanol (2 ml) versetzt. Die zuvor durchgehend feste Masse wurde während der Zugabe wieder flüssig. Zu dem Ansatz wurde nun portionsweise eine gesättigte NaCl-Lösung (10 ml) gegeben. Das erhaltene Gemisch wurde über Nacht stehen gelassen und dann über Kieselgur abgesaugt. Der Filterkuchen wurde mit insgesamt 400 ml DCM gewaschen. Das Filtrat wurde über Natriumsulfat getrocknet und eingeengt. Es wurden 730 mg 3-(2-Hydroxy-ethyl)-1H-indol-5-ol erhalten (Smp. 98-102 °C).

Referenz-Beispielübersicht:

| Referenz-Beispiel Nr. | Struktur | Salzform | Kommentare |
|---|---|---|---|
| 1 | 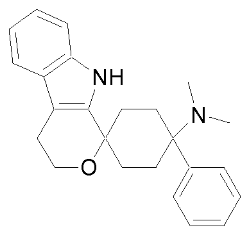 | Hydrochlorid | Unpolareres Diastereomer |
| 2 | 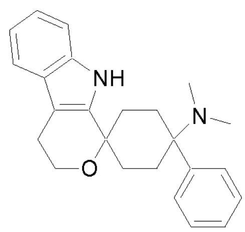 | Hydrochlorid | Polareres Diastereomer |

(fortgesetzt)

| Referenz-Beispiel Nr. | Struktur | Salzform | Kommentare |
|---|---|---|---|
| 3 | 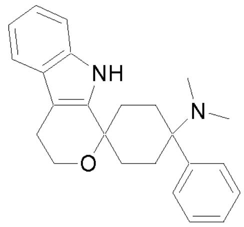 | Hemicitrat | Unpolareres Diastereomer |
| 4 | 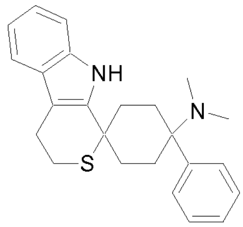 | Hemicitrat | Unpolareres Diastereomer |
| 5 | 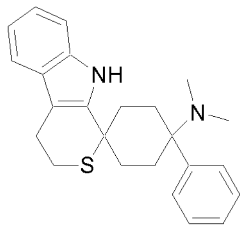 | Citrat | Polareres Diastereomer |
| 6 | 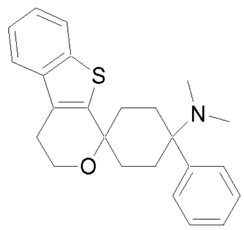 | Tartrat | Eins von 2 Diastereomeren |
| 7 | 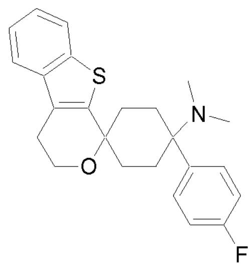 | Triflat | Eins von 2 Diastereomeren |
| 8 | 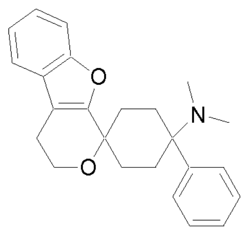 | Hemicitrat | Eins von 2 Diastereomeren |

(fortgesetzt)

| Referenz-Beispiel Nr. | Struktur | Salzform | Kommentare |
|---|---|---|---|
| 9 | 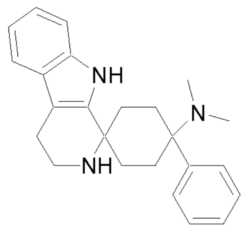 | Dihydrochlorid | 70:30 unpolareres:polareres D. |
| 10 | 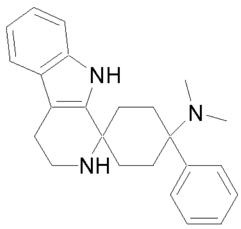 | Dihydrochlorid | Polareres Diastereomer |
| 11 | 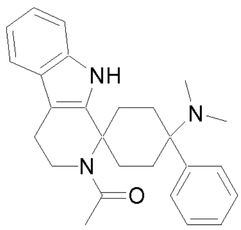 | Hydrochlorid | Unpolareres Diastereomer |
| 12 | 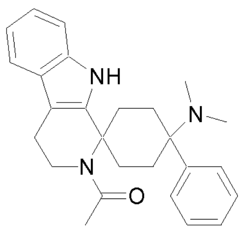 | Hydrochlorid | Polareres Diastereomer |
| 13 | 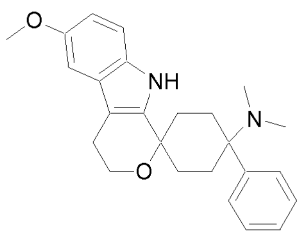 | Hydrochlorid | Unpolareres Diastereomer |
| 14 | 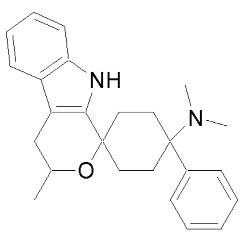 | Hemicitrat | Unpolareres Diastereomer |

(fortgesetzt)

| Referenz-Beispiel Nr. | Struktur | Salzform | Kommentare |
|---|---|---|---|
| 15 | 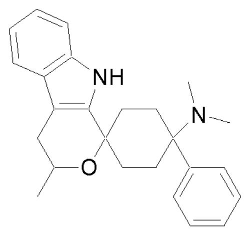 | Citrat | Polareres Diastereomer |
| 16 | 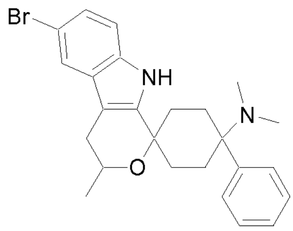 | Hemicitrat | Eins von 2 Diastereomeren |
| 17 | 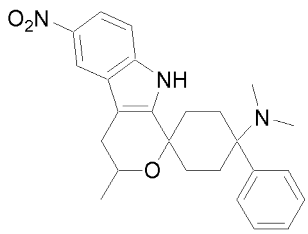 | Citrat | Unpolareres Diastereomer |
| 18 | 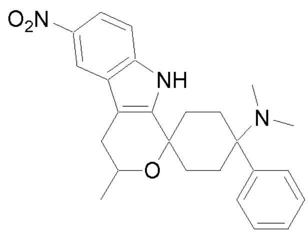 | Citrat | Polareres Diastereomer |
| 19 | 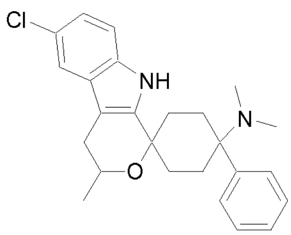 | Citrat | Unpolareres Diastereomer |
| 20 | 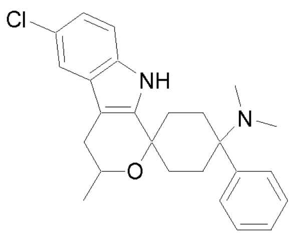 | Citrat | Polareres Diastereomer |

(fortgesetzt)

| Referenz-Beispiel Nr. | Struktur | Salzform | Kommentare |
|---|---|---|---|
| 21 | 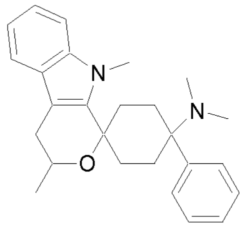 | Citrat | Eins von 2 Diastereomeren |
| 22 | 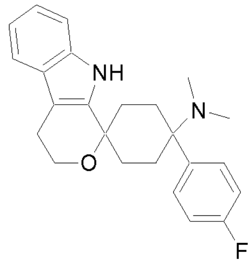 | Hemicitrat | Eins von 2 Diastereomeren |
| 23 | 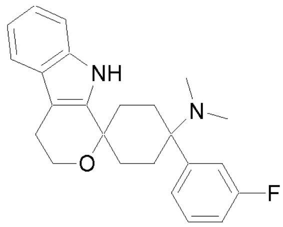 | Hemicitrat | Eins von 2 Diastereomeren |
| 24 | 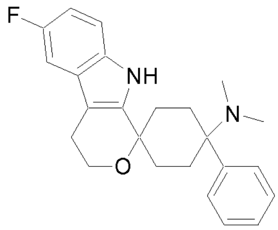 | Hemicitrat | Unpolareres Diastereomer |
| 25 | 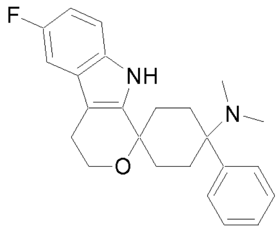 | Hemicitrat | Polareres Diastereomer |
| 26 | 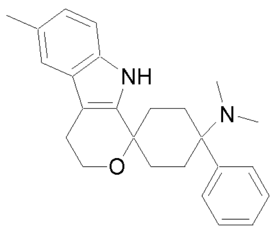 | Hemicitrat | Eins von 2 Diastereomeren |

(fortgesetzt)

| Referenz-Beispiel Nr. | Struktur | Salzform | Kommentare |
|---|---|---|---|
| 27 | 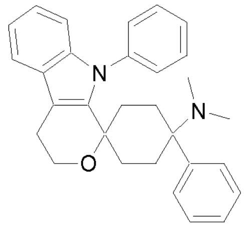 | Citrat | Eins von 2 Diastereomeren |
| 28 | 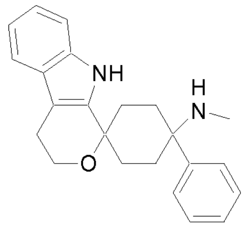 | Hemicitrat | Eins von 2 Diastereomeren |
| 29 | 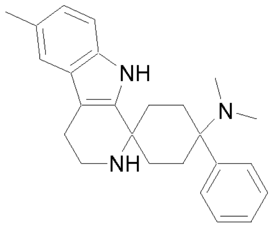 | Citrat | Polareres Diastereomer |
| 30 | 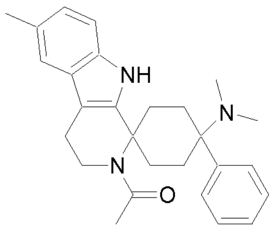 | Citrat | Polareres Diastereomer |
| 31 | 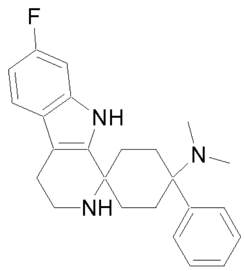 | Citrat | Polareres Diastereomer |
| 32 | 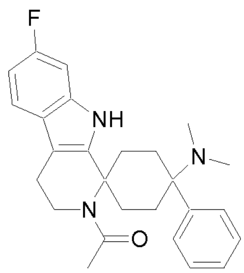 | Citrat | Unpolareres Diastereomer<br>Rotamere |

(fortgesetzt)

| Referenz-Beispiel Nr. | Struktur | Salzform | Kommentare |
|---|---|---|---|
| 33 | 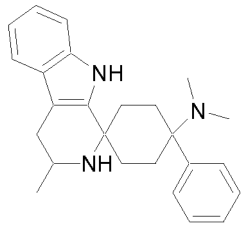 | Citrat | Eins von 2 Diastereomeren |
| 34 | 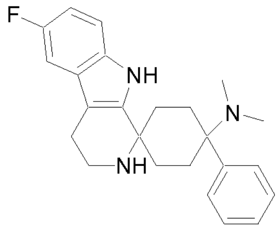 | Dihydrochlorid | Gemisch der Diastereomere |
| 35 | 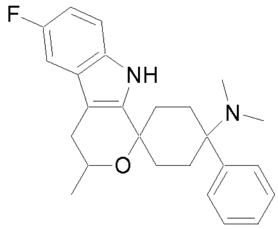 | Hemicitrat | Eins von 2 Diastereomeren |
| 36 | 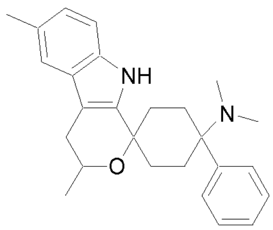 | Hemicitrat | Unpolareres Diastereomer |
| 37 | 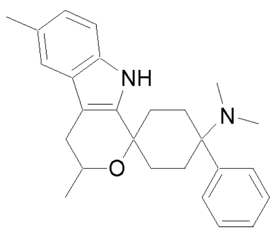 | Citrat | Polareres Diastereomer |
| 38 | 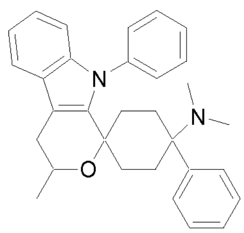 | Citrat | Eins von 2 Diastereomeren |

(fortgesetzt)

| Referenz-Beispiel Nr. | Struktur | Salzform | Kommentare |
|---|---|---|---|
| 39 | 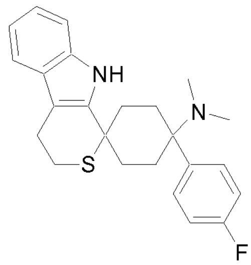 | Methansulfonat | Eins von 2 Diastereomeren |
| 40 | 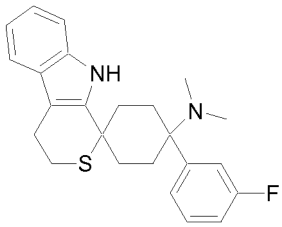 | Methansulfonat | Eins von 2 Diastereomeren |
| 41 | 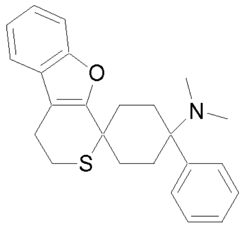 | Citrat | |
| 42 | 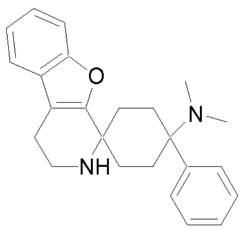 | Citrat | Eins von 2 Diastereomeren |
| 43 | 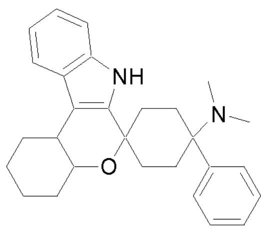 | Citrat | Gemisch der Diastereomeren |
| 44 | 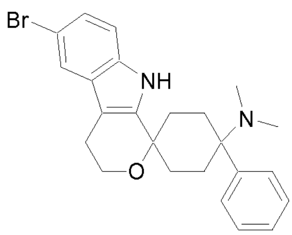 | Hemicitrat | Eins von 2 Diastereomeren |

(fortgesetzt)

| Referenz-Beispiel Nr. | Struktur | Salzform | Kommentare |
|---|---|---|---|
| 45 | 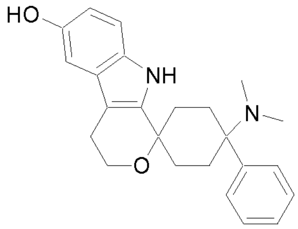 | Citrat | Eins von 2 Diastereomeren |
| 46 | 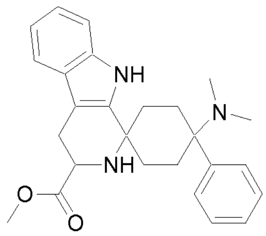 | Citrat | 70:30 unpolareres:polareres Diastereomer |
| 47 | 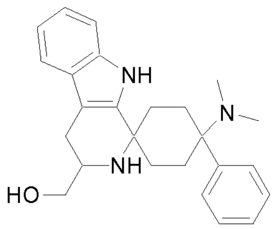 | Citrat | 30:70 unpolareres:polareres Diastereomer |
| 48 | 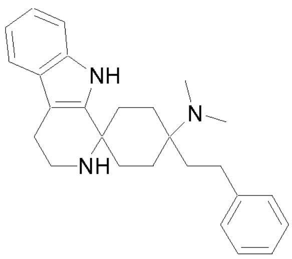 | Base | |
| 49 | 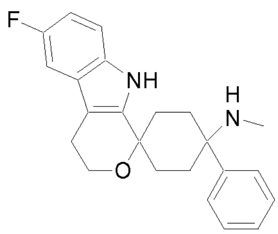 | Hemicitrat | Eins von 2 Diastereomeren |
| 50 | 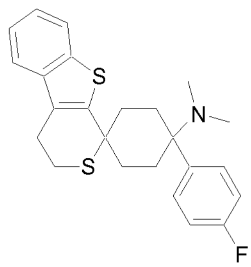 | Methansulfonat | Eins von 2 Diastereomeren |

(fortgesetzt)

| Referenz-Beispiel Nr. | Struktur | Salzform | Kommentare |
|---|---|---|---|
| 51 | 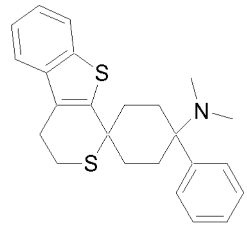 | Citrat | Eins von 2 Diastereomeren |
| 52 | 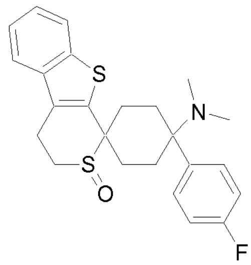 | Citrat | Eins von 2 Diastereomeren |
| 53 | 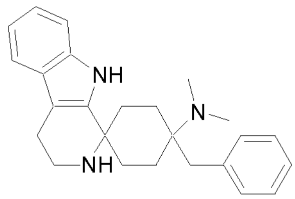 | Base | 15:85 unpolareres:polareres Diastereomer |

Untersuchungen zur Wirksamkeit der Referenz-Verbindungen: Die in den folgenden Assays und Modellen erhobenen Daten sind in Tabelle 1 zusammengefaßt.

Messung der ORL1-Bindung

Die Cyclohexan-Derivate der allgemeinen Formel I wurden in einem Rezeptorbindungsassay mit 3H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von 3H-Nociceptin/Orphanin FQ betrug bei diesen Versuchen 0.5 nM. Die Bindungsassays wurden mit je 20 μg Membranprotein je 200 μl Ansatz in 50 mM Hepes, pH 7,4, 10 mM MgCl2 und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschliessende Messung im Szintillationscounter Trilux (Wallac, Finnland), bestimmt. Die Affinität wird in Tabelle 1 als nanomolarer Ki-Wert in oder % Inhibition bei c=1 μM angegeben.

Messung der μ-Bindung

Die Rezeptoraffinität zum humanen μ-Opiatrezeptor wurde in einem homogenen Ansatz in Mikrotiterplatten bestimmt. Hierzu wurden Verdünnungsreihen des jeweils zu prüfenden substituierten spirocyclischen Cyclohexan-Derivates mit einer Rezeptormembranpräparation (15-40 μg Protein pro 250 μl Inkubationsansatz) von CHO-K1-Zellen, welche den humanen μ-Opiatrezeptor exprimieren (RB-HOM-Rezeptormembran-Präparation der Firma NEN, Zaventem, Belgien) in Gegenwart von 1 nmol/l des radioaktiven Liganden [3H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 μl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 μmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen μ-

Opiatrezeptor bei einer Konzentration der Prüfsubstanzen von 1 μmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I IC50 Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten.

Analgesieprüfung im Writhing-Test an der Maus

Die Untersuchung auf analgetische Wirksamkeit wurde im Phenylchinon-induzierten Writhing an der Maus (modifiziert nach I.C. Hendershot und J. Forsaith (1959) J. Pharmacol. Exp. Ther. 125, 237-240) durchgeführt. Dazu wurden männliche NMRI-Mäuse im Gewicht von 25 bis 30 g verwendet. Gruppen von 10 Tieren pro Substanzdosis erhielten 10 Minuten nach intravenöser Gabe der Prüfsubstanzen 0,3 ml/Maus einer 0,02%igen wäßrigen Lösung von Phenylchinon (Phenylbenzochinon, Fa. Sigma, Deisenhofen; Herstellung der Lösung unter Zusatz von 5 % Äthanol und Aufbewahrung im Wasserbad bei 45oC) intraperitoneal appliziert. Die Tiere wurden einzeln in Beobachtungskäfige gesetzt. Mittels eines Drucktastenzähler wurde die Anzahl der schmerzinduzierten Streckbewegungen (sogenannte Writhingreaktionen, d.h. das Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhalten. Alle Substanzen wurden in der Standarddosierung von 10 mg/kg getestet. Die prozentuale Hemmung (%Hemmung) der Writhingreaktion durch eine Substanz wurde nach folgender Formel berechnet:

Writhingreaktionen

$$\% \text{ Hemmung} = 100 - \text{der behandelten Tiere} * 100$$

Writhingreaktionen
der Kontrolltiere

Für einige Substanzen wurde aus der dosisabhängigen Abnahme der Writhingreaktionen im Vergleich zu parallel untersuchten Phenylchinon-Kontrollgruppen mittels Regressionsanalyse (Auswerteprogramm Martens EDV Service, Eckental) die ED50-Werte mit 95 % Vertrauensbereich der Writhingreaktion berechnet.

Analgesieprüfung im Tail-Flick-Test an der Maus

Die Mäuse wurden jeweils einzeln in einen Testkäfig gesetzt und die Schwanzbasis dem fokussierten Wärmestrahl einer elektrischen Lampe (Tail-flick-Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Mäusen 3 bis 5 Sekunden betrug. Vor der Applikation der Lösungen enthaltend die Referenz-Verbindung bzw. der jeweiligen Vergleichslösungen wurden die Mäuse innerhalb von fünf Minuten zweimal vorgetestet und der Mittelwert dieser Messungen als Vortestmittelwert berechnet.

Die Lösungen der Referenz-Verbindung der allgemeinen Formel I sowie die Vergleichslösungen wurden dann intravenös appliziert. Die Schmerzmessung wurde jeweils 10, 20, 40 und 60 Minuten nach der intravenösen Applikation durchgeführt. Die analgetische Wirkung wurde als Zunahme der Schmerzlatenz (% des maximal möglichen antinociceptiven Effektes) nach der folgenden Formel bestimmt:

$$[(T1-T0)/(T2-T0)] \times 100$$

Hierbei ist die Zeit T0 die Latenzzeit vor der Applikation, die Zeit T1 die Latenzzeit nach der Applikation der Wirkstoffkombination und die Zeit T2 die maximale Expositionsdauer (12 Sekunden).

Tabelle 1:

| Referenz-Beispiel Nr. | ORL1 Ki [nM] oder % Hemmung [1 μM] | μ Ki [nM] oder % Hemmung [1 μM] | Writhing (Maus, i.v.) ED50 [mg/kg] oder %Hemmung (Dosis [mg/kg]) | Tail Flick (Maus, i.v.) ED50 [mg/kg] oder %Hemmung (Dosis [mg/kg]) |
|---|---|---|---|---|
| 1 | 0,3 | 0,6 | | 0,0035 |
| 2 | | 310 | | |
| 3 | 0,6 | 1,3 | | 0,0182 (i.p.) |
| 4 | 3,7 | 3,1 | | |
| 6 | | 53 % | | |
| 7 | | 76 % | | |
| 8 | | 80 % | 89 % (10) | |
| 9 | 0,26 | 0,36 | | 94%(1) |
| 10 | 3,4 | 4,5 | | |
| 11 | 2,9 | 4,4 | | |
| 12 | 2,4 | 2,2 | | 67 % (0,1) |
| 13 | 5,8 | 2,0 | 0,0033 | 0,02 |
| 14 | 1,2 | 12,0 | | 0,029 |
| 15 | 42,0 | 58,0 | | |
| 16 | 23,0 | 14,0 | | |
| 17 | 70,0 | 6,6 | | |
| 18 | 29,0 | 25,0 | | |
| 19 | 91% | 95% | | |
| 20 | 56% | 75% | | |
| 21 | | 75% | | |
| 22 | 3,2 | 7,2 | | 100 % (0,1) |
| 23 | 1,2 | 2,1 | | 0,018 |
| 24 | 2,9 | 1,5 | | 0,019 |
| 25 | 22,0 | 12,0 | | 100 % (1) |
| 26 | 4,5 | 2,7 | | 0,039 |
| 28 | 1,4 | 1,2 | | 0,042 |
| 29 | 32,0 | 15,0 | | |
| 30 | 58% | 99% | | |
| 31 | 6,9 | 17,0 | | |
| 32 | 1,1 | 1,7 | | 100 % (0,1) |
| 33 | 0,5 | 0,5 | | 100 % (1) |
| 34 | 1,4 | 0,7 | | 89%(1) |
| 35 | 83,0 | 61,0 | | |
| 36 | 4,4 | 14,0 | | 100 % (1) |
| 37 | 56% | 90% | | |
| 38 | | 43% | | |

(fortgesetzt)

| Referenz-Beispiel Nr. | ORL1 Ki [nM] oder % Hemmung [1 μM] | μ Ki [nM] oder % Hemmung [1 μM] | Writhing (Maus, i.v.) ED50 [mg/kg] oder %Hemmung (Dosis [mg/kg]) | Tail Flick (Maus, i.v.) ED50 [mg/kg] oder %Hemmung (Dosis [mg/kg]) |
|---|---|---|---|---|
| 39 | | 90% | | |
| 40 | 55% | 100% | | |
| 42 | 75% | 86% | | |
| 43 | 91% | 96% | | |
| 44 | 52,0 | 19,0 | | |
| 45 | 1,6 | 1,1 | | 0,013 |
| 46 | 0,9 | 2,3 | | |
| 47 | 99% | 2,7 | | |
| 49 | 10,0 | 6,8 | | 0,22 |
| 52 | 62,0 | 58,0 | | |
| 53 | 1,1 | 0,6 | | |

Referenz-Beispiel 54:

Parenterale Lösung eines spirocyclischen Cyclohexan-Derivats 38 g eines der erfindungsgemäßen spirocyclischen Cyclohexan-Derivate, hier Referenz-Beispiel 3, wird in 1 l Wasser für Injektionszwecke bei Raumtemperatur gelöst und anschließend durch Zugabe von wasserfreier Glukose für Injektionszwecke auf isotone Bedingungen eingestellt.

## Patentansprüche

**1.** Spirocyclische Cyclohexan-Derivate der allgemeinen Formel I,

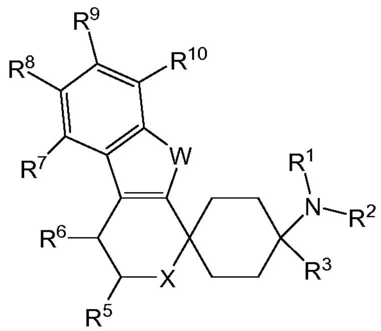

I ,

worin

$R^1$ und $R^2$ unabhängig voneinander für H, $C_{1-5}$-Alkyl, verzweigt oder unverzweigt, gesättigt oder ungesättigt, unsubstituiert, oder CHO stehen;
oder die Reste $R^1$ und $R^2$ zusammen für $CH_2CH_2OCH_2CH_2$, $CH_2CH_2NR^{11}CH_2CH_2$ oder $(CH_2)_{3-6}$ stehen; wobei $R^{11}$ H; oder $C_{1-5}$-Alkyl, jeweils gesättigt oder ungesättigt, verzweigt oder unverzweigt, unsubstituiert bedeutet;
$R^3$ für $C_{1-5}$-Alkyl, jeweils gesättigt oder ungesättigt, verzweigt oder unverzweigt, einfach oder mehrfach substituiert oder unsubstituiert; $C_{3-8}$-(Hetero-)Cycloalkyl, jeweils gesättigt oder ungesättigt, einfach oder mehrfach substituiert oder unsubstituiert; Aryl, jeweils unsubstituiert oder einfach oder mehrfach substituiert; über $C_{1-3}$-Al-

kyl-Gruppe, jeweils gesättigt oder ungesättigt, verzweigt oder unverzweigt, gebundenes Aryl oder $C_{3-8}$-(Hetero-)Cycloalkyl, jeweils unsubstituiert oder einfach oder mehrfach substituiert, steht;

W für $NR^4$, O oder S steht; wobei

$R^4$ für H; $C_{1-5}$-Alkyl, gesättigt oder ungesättigt, verzweigt oder unverzweigt, unsubstituiert oder einfach oder mehrfach substituiert; oder Aryl, substituiert oder unsubstituiert steht; wobei

$R^{12}$ H; $C_{1-5}$-Alkyl, jeweils gesättigt oder ungesättigt, verzweigt oder unverzweigt, einfach oder mehrfach substituiert oder unsubstituiert; $C_{3-8}$-(Hetero-)Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkyl, jeweils gesättigt oder ungesättigt, verzweigt oder unverzweigt, gebundenes Aryl, $C_{3-8}$-(Hetero-)Cycloalkyl oder Heteroaryl, jeweils einfach oder mehrfach substituiert oder unsubstituiert; $OR^{13}$; $NR^{14}R^{15}$ bedeutet;

$R^5$ für $C_{3-8}$-(Hetero-)Cycloalkyl, gesättigt oder ungesättigt, unsubstituiert oder einfach oder mehrfach substituiert; Aryl-, oder Heteroaryl, unsubstituiert oder einfach oder mehrfach substituiert; oder über $C_{1-3}$-Alkyl, jeweils gesättigt oder ungesättigt, verzweigt oder unverzweigt, gebundenes $C_{3-8}$-(Hetero-)Cycloalkyl oder Heteroaryl, unsubstituiert oder einfach oder mehrfach substituiert, steht;

$R^6$ für H; F, Cl, $NO_2$, $CF_3$, $OR^{13}$, $SR^{13}$, $SO_2R^{13}$, $SO_2OR^{13}$, CN, $COOR^{13}$, $NR^{14}R^{15}$; $C_{1-5}$-Alkyl, gesättigt oder ungesättigt, verzweigt oder unverzweigt, unsubstituiert oder einfach oder mehrfach substituiert; Aryl-, unsubstituiert oder einfach oder mehrfach substituiert; oder über $C_{1-3}$-Alkyl, jeweils gesättigt oder ungesättigt, verzweigt oder unverzweigt, gebundenes Aryl, unsubstituiert oder einfach oder mehrfach substituiert, steht;

$R^7$, $R^8$, $R^9$ und $R^{10}$ unabhängig voneinander für H, F, Cl, Br, I, $NO_2$, $CF_3$, $OR^{13}$, $SR^{13}$, $SO_2R^{13}$, $SO_2OR^{13}$, $SO_2NH_2$, CN, $COOR^{13}$, $NR^{14}R^{15}$; $C_{1-5}$-Alkyl, $C_{3-8}$-(Hetero-)Cycloalkyl, unsubstituiert oder einfach oder mehrfach substituiert; Aryl-, oder Heteroaryl, unsubstituiert oder einfach oder mehrfach substituiert; oder über $C_{1-3}$-Alkyl, jeweils gesättigt oder ungesättigt, verzweigt oder unverzweigt, gebundenes Aryl, $C_{3-8}$-(Hetero-)Cycloalkyl oder Heteroaryl, unsubstituiert oder einfach oder mehrfach substituiert, stehen; wobei

$R^{13}$ H; $C_{1-5}$-Alkyl jeweils gesättigt oder ungesättigt, verzweigt oder unverzweigt, unsubstituiert; oder $C_{3-8}$-(Hetero-)Cycloalkyl, jeweils gesättigt oder ungesättigt, unsubstituiert bedeutet;

$R^{14}$ und $R^{15}$ unabhängig voneinander H; $C_{1-5}$-Alkyl, jeweils gesättigt oder ungesättigt, verzweigt oder unverzweigt, unsubstituiert bedeuten;

oder $R^{14}$ und $R^{15}$ zusammen $CH_2CH_2OCH_2CH_2$, $CH_2CH_2NR^{16}CH_2CH_2$ oder $(CH_2)_{3-6}$ bilden, wobei $R^{16}$ H; $C_{1-5}$-Alkyl gesättigt oder ungesättigt, verzweigt oder unverzweigt, unsubstituiert, bedeutet;

X für $NR^{17}$ steht; wobei

$R^{17}$ für H; $C_{1-5}$-Alkyl, gesättigt oder ungesättigt, verzweigt oder unverzweigt; $COR^{12}$ oder $SO_2R^{12}$ steht;

wobei

im Zusammenhang mit "Alkyl" unter dem Begriff "substituiert" die Substitution eines oder mehrerer Wasserstoffreste durch F, Cl, Br, I, -CN, $NH_2$, NH-Alkyl, NH-(Hetero-)Cycloalkyl, NH-Alkyl-OH, $N(Alkyl)_2$, $N((Hetero\text{-})Cycloalkyl)_2$, $N(Alkyl\text{-}OH)_2$, $NO_2$, SH, S-Alkyl, S-(Hetero-)Cycloalkyl, S-Alkyl-OH, S-Alkyl-SH, OH, O-Alkyl, O-(Hetero-)Cycloalkyl, O-Alkyl-OH, CHO, $C(=O)C_{1-6}$-Alkyl, $C(=S)C_{1-6}$-Alkyl, C(=O)-(Hetero-)Cycloalkyl, C(=S)-(Hetero-)Cycloalkyl, $CO_2H$, $CO_2$-Alkyl, $C(=O)NH_2$, C(=O)NH-Alkyl, C(=O)NH-(Hetero-)Cycloalkyl, $C(=O)N(Alkyl)_2$, $C(=O)N((Hetero\text{-})Cycloalkyl)_2$, SO-Alkyl, $SO_2$-Alkyl, $SO_2NH_2$, $SO_3H$, oder (Hetero-)Cycloalkyl, Aryl oder Heteroaryl verstanden wird; und

in Bezug auf "Aryl" sowie "(Hetero-)Cycloalkyl" unter "ein- oder mehrfach substituiert" die ein- oder mehrfache, z.B. zwei-, drei- vier- oder fünffache, Substitution eines oder mehrerer Wasserstoffatome des Ringsystems durch F, Cl, Br, I, CN, $NH_2$, NH-Alkyl, NH-(Hetero-)Cycloalkyl, NH-Alkyl-OH, $N(Alkyl)_2$, $N((Hetero\text{-})Cycloalkyl)_2$, $N(Alkyl\text{-}OH)_2$, $NO_2$, SH, S-Alkyl, S-(Hetero-)Cycloalkyl, S-Alkyl-OH, S-Alkyl-SH, OH, O-Alkyl, O-(Hetero-)Cycloalkyl, O-Alkyl-OH, CHO, $C(=O)C_{1-6}$-Alkyl, $C(=S)C_{1-6}$-Alkyl, C(=O)-(Hetero-)Cycloalkyl, C(=S)-(Hetero-)Cycloalkyl, $CO_2H$, $CO_2$-Alkyl, $C(=O)NH_2$, C(=O)NH-Alkyl, C(=O)NH-(Hetero-)Cycloalkyl, $C(=O)N(Alkyl)_2$, $C(=O)N((Hetero\text{-})Cycloalkyl)_2$, S(O)-Alkyl, $SO_2$-Alkyl, $SO_2NH_2$, $SO_3H$, $CF_3$, =O, =S; Alkyl, (Hetero-)Cycloalkyl, oder Heteroaryl verstanden wird;

in Bezug auf "Heteroaryl" unter "ein- oder mehrfach substituiert" die ein- oder mehrfache, z.B. zwei-, drei- vier- oder fünffache, Substitution eines oder mehrerer Wasserstoffatome des Ringsystems durch F, Cl, Br, I, CN, $NH_2$, NH-Alkyl, NH-Aryl, NH-Heteroaryl. NH-Alkyl-Aryl, NH-Alkyl-Heteroaryl, NH-(Hetero-)Cycloalkyl, NH-Alkyl-OH, $N(Alkyl)_2$, $N(Alkyl\text{-}Aryl)_2$, $N(Alkyl\text{-}Heteroaryl)_2$, $N((Hetero\text{-})Cycloalkyl)_2$, $N(Alkyl\text{-}OH)_2$, $NO_2$, SH, S-Alkyl, S-(Hetero-)Cycloalkyl, S-Aryl, S-Heteroaryl, S-Alkyl-Aryl, S-Alkyl-Heteroaryl, S-Alkyl-OH, S-Alkyl-SH, OH, O-Alkyl, O-(Hetero-)Cycloalkyl, O-Aryl, O-Heteroaryl, O-Alkyl-Aryl, O-Alkyl-Heteroaryl, O-Alkyl-OH, CHO, $C(=O)C_{1-6}$-Alkyl, $C(=S)C_{1-6}$-Alkyl, C(=O)-(Hetero-)Cycloalkyl, C(=S)-(Hetero-)Cycloalkyl, $CO_2H$, $CO_2$-Alkyl, $C(=O)NH_2$, C(=O)NH-Alkyl, C(=O)NH-(Hetero-)Cycloalkyl, $C(=O)N(Alkyl)_2$, $C(=O)N((Hetero\text{-})Cycloalkyl)_2$, S(O)-Alkyl, $SO_2$-Alkyl, $SO_2NH_2$, $SO_3H$, $CF_3$, =O, =S; Alkyl, (Hetero-)Cycloalkyl, Aryl und/oder Heteroaryl verstanden wird;

der Ausdruck "Aryl" ein carbocyclisches Ringsystem mit mindestens einem aromatischen Ring, aber ohne Heteroatome in nur einem der Ringe, bedeutet, wobei die ArylReste ggf. mit weiteren gesättigten, (partiell) ungesättigten oder aromatischen Ringsystemen kondensiert sein können;
der Ausdruck "Heteroaryl" für einen 5-, 6- oder 7-gliedrigen cyclischen aromatischen Rest steht, der mindestens 1, ggf. auch 2, 3, 4 oder 5 Heteroatome, enthält, wobei die Heteroatome gleich oder verschieden Stickstoff, Sauerstoff oder Schwefel sind; und wobei der Heterocyclus auch Teil eines bi- oder polycyclischen Systems sein kann; der Ausdruck "(Hetero-)Cycloalkyl" für einen cyclischen Kohlenwasserstoff mit 3, 4, 5, 6, 7 oder 8 Kohlenstoffatomen steht, wobei die Kohlenwasserstoffe gesättigt oder ungesättigt, aber nicht aromatisch sein können, und wobei ggf. ein oder zwei Kohlenstoffatome durch ein Heteroatom S, N oder O ersetzt sein können; in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

**2.** Spirocyclische Cyclohexan-Derivate gemäß Anspruch 1, **dadurch gekennzeichnet, dass** $R^3$ für Phenyl, Benzyl oder Phenethyl, jeweils unsubstituiert oder am Ring ein- oder mehrfach substituiert, steht.

**3.** Spirocyclische Cyclohexan-Derivate gemäß einem der Ansprüche 1 bis 2, **dadurch gekennzeichnet, dass** $R^7$, $R^8$, $R^9$ und $R^{10}$ unabhängig voneinander für H; $C_{1-5}$-Alkyl, verzweigt oder unverzweigt, unsubstituiert oder ein- oder mehrfach substituiert; F, Cl, Br, I, $CF_3$, OH, $OCH_3$, $NH_2$, COOH, $COOCH_3$, $NHCH_3$, $N(CH_3)_2$, $NO_2$, $SO_3H$, $SO_2NH_2$, Pyridyl oder Phenyl stehen.

**4.** Spirocyclische Cyclohexan-Derivate gemäß einem der Ansprüche 1 bis 3, **dadurch gekennzeichnet, dass** $R^1$ und $R^2$ unabhängig voneinander H oder $CH_3$ bedeuten, wobei $R^1$ und $R^2$ nicht gleichzeitig H bedeuten.

**5.** Spirocyclische Cyclohexan-Derivate gemäß einem der Ansprüche 1 bis 4, **dadurch gekennzeichnet, dass** $R^3$ Phenyl, Benzyl oder Phenethyl, jeweils unsubstituiert oder am Ring ein- oder mehrfach substituiert;
insbesondere Phenyl, Benzyl, Phenethyl, 2-Fluorphenyl, 3-Fluorphenyl, 4-Fluorphenyl, 2-Chlorphenyl, 3-Chlorphenyl, 4-Chlorphenyl, 2-Bromphenyl, 3-Bromphenyl, 4-Bromphenyl, 2-Cyanophenyl, 3-Cyanophenyl, 4-Cyanophenyl, 2-Methylphenyl, 3-Methylphenyl, 4-Methylphenyl, 2-Methoxyphenyl, 3-Methoxyphenyl, 4-Methoxyphenyl, 2-Trifluormethylphenyl, 3-Trifluormethylphenyl, 3-Trifluormethylphenyl, 2-Ethylphenyl, 3-Ethylphenyl, 4-Ethylphenyl, 2-Ethoxyphenyl, 3-Ethoxyphenyl, 4-Ethoxyphenyl, 2-Hydroxyphenyl, 3-Hydroxyphenyl, 4-Hydroxyphenyl, 2,3-Dichlorphenyl, 3,4-Dichlorphenyl, 3,5-Dichlorphenyl, 2,4-Dichlorphenyl, 2,3-Difluorphenyl, 3,4-Difluorphenyl, 3,5-Difluorphenyl, 2,4-Difluorphenyl, 2-Fluor-3-chlorphenyl, 2-Chlor-3-fluorphenyl, 2-Chlor-4-fluorphenyl, 2-Fluor-4-chlorphenyl, 4-Fluor-3-chlorphenyl, 4-Fluor-3-methylphenyl, 4-tert.-Butylphenyl, 4-Fluor-3-chlorphenyl, 4-Brom-3-fluorphenyl, 3,5-Bis(trifluormethyl)phenyl, 4-Chlor-2-trifluormethylphenyl, 2-Methoxy-5-methylphenyl, 5-Chlor-2-methoxyphenyl, 4-Phenoxyphenyl, 2-Methylthiophenyl, 3-Methylthiophenyl, 4-Methylthiophenyl, 5-Fluor-2-methoxyphenyl, 4-Chlor-3-trifluormethyl oder 4-Brom-2-methylphenyl;
besonders bevorzugt Phenyl, Benzyl, Phenethyl, 4-Fluorphenyl und 3-Fluorphenyl, bedeutet.

**6.** Spirocyclische Cyclohexan-Derivate gemäß einem der Ansprüche 1 bis 5, **dadurch gekennzeichnet, dass** $R^1$ und $R^2$ $CH_3$ bedeuten und $R^3$ Phenyl bedeutet.

**7.** Spirocyclische Cyclohexan-Derivate gemäß einem der Ansprüche 1 bis 6, **dadurch gekennzeichnet, dass** W $NR^4$ bedeutet, wobei $R^4$ für H, $CH_3$, $C_2H_5$, oder Phenyl, steht, und X NH oder $NCOR^{12}$ bedeutet.

**8.** Verfahren zur Herstellung von spirocyclischen Cyclohexan-Derivaten gemäß einem der Ansprüche 1 bis 7, **dadurch gekennzeichnet, dass** ein Edukt der allgemeinen Formel A

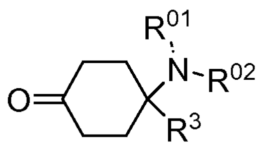

**A**

wobei die Reste $R^{01}$ und $R^{02}$ die für $R^2$ angegebene Bedeutung haben und zusätzlich für eine Schutzgruppe stehen können, unter Zugabe von Säure, oder deren Trimethylsilylester, in einem geeigneten Lösungsmittel, mit einem Edukt der allgemeinen Formel B

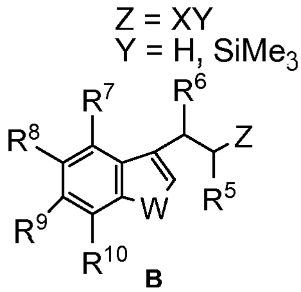

B

umgesetzt wird, wobei die Reste $R^1$-$R^{10}$ und X die in Anspruch 1 angegebenen Bedeutungen haben.

**9.** Verfahren zur Herstellung von spirocyclischen Cyclohexan-Derivaten gemäß Anspruch 1, bei denen X $NR^{17}$ und $R^{17}$ $COR^{12}$ oder $SO_2R^{12}$ bedeutet, **dadurch gekennzeichnet, dass** ein spirocyclisches Cyclohexan-Derivat, bei dem X NH bedeutet, unter Zugabe von Base mit einem Anhydrid oder einem Säurechlorid umgesetzt wird.

**10.** Arzneimittel enthaltend wenigstens ein spirocyclisches Cyclohexan-Derivat gemäß einem der Ansprüche 1 bis 7, gegebenenfalls in Form seines Razemats, der reinen Stereoisomeren, in einem beliebigen Mischungsverhältnis; in Form seiner Säuren oder seiner Basen oder in Form seiner Salze; oder in Form seiner Solvate, sowie gegebenenfalls enthaltend geeignete Zusatz- und/oder Hilfsstoffe und/oder gegebenenfalls weiterer Wirkstoffe.

**11.** Verwendung eines spirocyclischen Cyclohexan-Derivates gemäß einem der Ansprüche 1 bis 7, gegebenenfalls in Form seines Razemats, der reinen Stereoisomeren, in einem beliebigen Mischungsverhältnis; in Form seiner Säuren oder seiner Basen oder in Form seiner Salze; oder in Form seiner Solvate; zur Herstellung eines Arzneimittels zur Behandlung von Schmerz.

**12.** Verwendung eines spirocyclisches Cyclohexan-Derivats gemäß einem der Ansprüche 1 bis 7 zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmissbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

**13.** Verwendung gemäß Anspruch 11 wobei der Schmerz akuter, neuropathischer oder chronischer Schmerz ist.

### Claims

**1.** Spirocyclic cyclohexane derivatives of the general formula I,

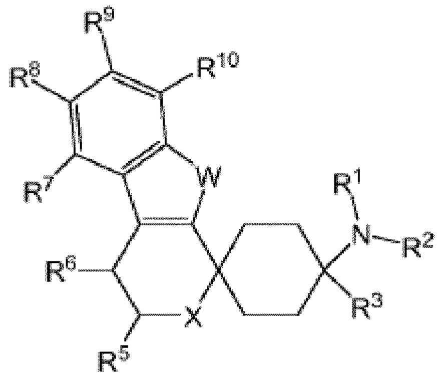

I ,

in which

$R^1$ and $R^2$ independently of one another represent H, $C_{1-5}$-alkyl, branched or straight-chain, saturated or unsaturated, unsubstituted, or CHO;
or the radicals $R^1$ and $R^2$ together represent $CH_2CH_2OCH_2CH_2$, $CH_2CH_2NR^{11}CH_2CH_2$ or $(-CH_2)_{3-6}$; where $R^{11}$ represents H; or $C_{1-5}$-alkyl, in each case saturated or unsaturated, branched or straight-chain, unsubstituted;
$R^3$ represents $C_{1-5}$-alkyl, in each case saturated or unsaturated, branched or straight-chain, mono or polysubstituted or unsubstituted; $C_{3-8}$-(hetero)cycloalkyl, in each case saturated or unsaturated, mono- or polysubstituted or unsubstituted; aryl, in each case unsubstituted or mono- or polysubstituted; aryl or $C_{3-8}$-(hetero)cycloalkyl, in each case unsubstituted or mono- or polysubstituted and attached via a $C_{1-3}$-alkyl group, in each case saturated or unsaturated, branched or straight-chain;
W represents $NR^4$, 0 or S; where
$R^4$ represents H; $C_{1-5}$-alkyl, saturated or unsaturated, branched or straight-chain, unsubstituted or mono- or polysubstituted; or aryl, substituted or unsubstituted; where $R^{12}$ represents H; $C_{1-5}$-alkyl, in each case saturated or unsaturated, branched or straight-chain, mono- or polysubstituted or unsubstituted; $C_{3-8}$-(hetero)cycloalkyl, in each case saturated or unsaturated, mono- or polysubstituted or unsubstituted; aryl or heteroaryl, in each case mono- or polysubstituted or unsubstituted; or aryl, $C_{3-8}$-(hetero)cycloalkyl or heteroaryl, in each case mono- or polysubstituted or unsubstituted and attached via $C_{1-3}$-alkyl, in each case saturated or unsaturated, branched or straight-chain; $OR^{13}$; $NR^{14}R^{15}$;
$R^5$ represents $C_{3-8}$-(hetero)cycloalkyl saturated or unsaturated, unsubstituted or mono- or polysubstituted; aryl or heteroaryl, unsubstituted or mono- or polysubstituted; or $C_{3-8}$-(hetero)cycloalkyl or heteroaryl, unsubstituted or mono- or polysubstituted and attached via $C_{1-3}$-alkyl, in each case saturated or unsaturated, branched or straight-chain;
$R^6$ represents H; F, Cl, $NO_2$, $CF_3$, $OR^{13}$, $SR^{13}$,$SO_2R^{13}$, $SO_2OR^{13}$, CN, $COOR^{13}$, $NR^{14}R^{15}$, $C_{1-5}$-alkyl, saturated or unsaturated, branched or straight-chain, unsubstituted or mono- or polysubstituted; aryl, unsubstituted or mono- or polysubstituted; or aryl, unsubstituted or mono- or polysubstituted and attached via $C_{1-3}$-alkyl, in each case saturated or unsaturated, branched or straight-chain;
$R^7$, $R^8$, $R^9$ and $R^{10}$ independently of one another represent H, F, Cl, Br, I, $NO_2$, $CF_3$, $OR^{13}$, $SR^{13}$, $SO_2R^{13}$, $SO_2OR^{13}$, $SO_2NH_2$, CN, $COOR^{13}$, $NR^{14}R^{15}$, $C_{1-5}$-alkyl, $C_{3-8}$-(hetero)cycloalkyl, unsubstituted or mono- or polysubstituted; aryl or heteroaryl, unsubstituted or mono- or polysubstituted; or aryl, $C_{3-8}$-(hetero)cycloalkyl or heteroaryl, unsubstituted or mono- or polysubstituted and attached via $C_{1-3}$-alkyl, in each case saturated or unsaturated, branched or straight-chain; where $R^{13}$ represents H; $C_{1-5}$-alkyl, in each case saturated or unsaturated, branched or straight-chain, unsubstituted; or $C_{3-8}$-(hetero)cycloalkyl, in each case saturated or unsaturated, unsubstituted;
$R^{14}$ and $R^{15}$ independently of one another represents H; $C_{1-5}$-alkyl, in each case saturated or unsaturated, branched or straight-chain, unsubstituted;
or $R^{14}$ and $R^{15}$ together represents $CH_2CH_2OCH_2CH_2$, $CH_2CH_2NR^{16}CH_2CH_2$ or $(CH_2)_{3-6}$, where $R^{16}$ represents H; $C_{1-5}$-alkyl, saturated or unsaturated, branched or straight-chain, unsubstituted;
X represents $NR^{17}$; where
$R_{17}$ represents H; $C_{1-5}$-alkyl, saturated or unsaturated, branched or straight-chain; $COR^{12}$ or $SO_2R^{12}$;
where
in the context with "alkyl" the term "substituted" is to be understood as the substitution of one or more hydrogen

radicals by F, Cl, Br, I, -CN, $NH_2$, NH-alkyl, NH-(hetero)cycloalkyl, NH-alkyl-OH, $N(alkyl)_2$, $N((hetero)cycloalkyl)_2$, $N(alkyl-OH)_2$, $NO_2$, SH, S-alkyl, S-(hetero)cycloalkyl, S-alkyl-OH, S-alkyl-SH, OH, O-alkyl, O-(hetero)cycloalkyl, O-alkyl-OH, CHO, C (=0) $C_{1-6}$-alkyl, C (=S) $C_{1-6}$-alkyl, C(=O)-(hetero)cycloalkyl, C(=S)-(hetero)cycloalkyl, $CO_2H$, $CO_2$-alkyl, $C(=O)NH_2$, C(=O)NH-alkyl, C(=O)NH-(hetero)cycloalkyl, $C(=O)N(alkyl)_2$, C(=O)N((hetero)cycloalkyl)$_2$, SO-alkyl, $SO_2$-alkyl, $SO_2NH_2$, $SO_3H$ or (hetero)cycloalkyl, aryl or heteroaryl; and

with respect to "aryl" and "(hetero)cycloalkyl" "mono- or polysubstituted" is to be understood as mono- or poly-, e.g. di-, tri-, tetra- or pentasubstitution of one or more hydrogen atoms of the ring system by F, Cl, Br, I, CN, $NH_2$, NH-alkyl, NH-(hetero)cycloalkyl, NH-alkyl-OH, $N(alkyl)_2$, N ((hetero) cycloalkyl)$_2$, N (alkyl-OH)$_2$, $NO_2$, SH, S-alkyl, S-(hetero)cycloalkyl, S-alkyl-OH, S-alkyl-SH, OH, O-alkyl, O-(hetero)cycloalkyl, O-alkyl-OH, CHO, C(=O)$C_{1-6}$-alkyl, C(=S)$C_{1-6}$-alkyl, C(=O)-(hetero)cycloalkyl, C(=S)-(hetero)cycloalkyl, $CO_2H$, $CO_2$-alkyl, $C(=O)NH_2$, C(=O)NH-alkyl, C(=O)NH-(hetero)cycloalkyl, $C(=O)N(alkyl)_2$, C(=O)N((hetero)cycloalkyl)$_2$, S(O)-alkyl, $SO_2$-alkyl, $SO_2NH_2$, $SO_3H$, $CF_3$, =O, =S; alkyl, (hetero) cycloalkyl or heteroaryl;

with respect to "heteroaryl" or "mono- or polysubstituted" is to be understood as mono- or poly-, e.g. di-, tri-, tetra- or pentasubstitution of one or more hydrogen atoms of the ring system by F, Cl, Br, I, CN, $NH_2$, NH-alkyl, NH-aryl, NH-heteroaryl, NH-alkyl-aryl, NH-alkyl-heteroaryl, NH-(hetero)cycloalkyl, NH-alkyl-OH, $N(alkyl)_2$, $N(alkyl-aryl)_2$, $N(alkyl-heteroaryl)_2$, $N((hetero)cycloalkyl)_2$, $N(alkyl-OH)_2$, $NO_2$, SH, S-alkyl, S-(hetero)cycloalkyl, S-aryl, S-heteroaryl, S-alkyl-aryl, S-alkyl-heteroaryl, S-alkyl-OH, S-alkyl-SH, OH, O-alkyl, O-(hetero)cycloalkyl, O-aryl, O-heteroaryl, O-alkyl-aryl, O-alkyl-heteroaryl, O-alkyl-OH, CHO, C(=O)$C_{1-6}$-alkyl, C(=S)$C_{1-6}$-alkyl, C(=O)-(hetero)cycloalkyl, C(=S)-(hetero)cycloalkyl, $CO_2H$, $CO_2$-alkyl, $C(=O)NH_2$, C(=O)NH-alkyl, C(=O)NH-(hetero)cycloalkyl, $C(=O)N(alkyl)_2$, C(=O)N((hetero)cycloalkyl)2, S(O)-alkyl, $SO_2$-alkyl, $SO_2NH_2$, $SO_3H$, $CF_3$, =O, =S; alkyl, (hetero)cycloalkyl, aryl and/or heteroaryl;

the term "aryl" means a carbocyclic ring system having at least one aromatic ring but without heteroatoms in only one of the rings, where the aryl radicals may optionally be fused with further saturated, (partially) unsaturated or aromatic ring systems;

the term "heteroaryl" represents a 5-, 6- or 7-numbered cyclic aromatic radical which contains at least one, optionally also two, three, four or five heteroatoms, where the heteroatoms are identical or different and are nitrogen, oxygen or sulphur; and where the heterocycle may also be part of a bi- or polycyclic system;

the term "(hetero)-CyCloalkyl" represents a cyclic hydrocarbon having 3, 4, 5, 6, 7 or 8 carbon atoms, where the hydrocarbons may be saturated or unsaturated, but not aromatic, and where optionally one or two carbon atoms may be replaced by a heteroatom S, N or 0;

in the form of the racemate; the enantiomers, diastereomers, mixtures of the enantiomers or diastereomers or of an individual enantiomer or diastereomer, the bases and/or salts of physiologically acceptable acids or cations.

2. Spirocyclic cyclohexane derivatives according to Claim 1, **characterized in that** $R^3$ represents phenyl, benzyl or phenethyl, in each case unsubstituted or mono- or polysubstituted on the ring.

3. Spirocyclic cyclohexane derivatives according to Claim 1 or 2, **characterized in that** $R^7$, $R^8$, $R^9$ and $R^{10}$ independently of one another represents H; $C_{1-5}$-alkyl, branched or straight-chain, unsubstituted or mono- or polysubstituted; F, Cl, Br, I, $CF_3$, OH, $OCH_3$, $NH_2$, COOH, $COOCH_3$, $NHCH_3$, $N(CH_3)_2$, $NO_2$, $SO_3H$, $SO_2NH_2$, pyridyl or phenyl.

4. Spirocyclic cyclohexane derivatives according to any of Claims 1 to 3, **characterized in that** $R^1$ and $R^2$ independently of one another represent H or $CH_3$, where $R^1$ and $R^2$ do not both represent H.

5. Spirocyclic cyclohexane derivatives according to any of Claims 1 to 4, **characterized in that** $R^3$ represents phenyl, benzyl or phenethyl, in each case unsubstituted or mono- or polysubstituted on the ring;
in particular phenyl, benzyl, phenethyl, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 2-cyanophenyl, 3-cyanophenyl, 4-cyanophenyl, 2-methylphenyl, 3-methylphenyl, 4-methylphenyl, 2-methoxyphenyl, 3-methoxyphenyl, 4-methoxyphenyl, 2-trifluoromethylphenyl, 3-trifluoromethylphenyl, 3-trifluoromethylphenyl, 2-ethylphenyl, 3-ethylphenyl, 4-ethylphenyl, 2-ethoxyphenyl, 3-ethoxyphenyl, 4-ethoxyphenyl, 2-hydroxyphenyl, 3-hydroxyphenyl, 4-hydroxyphenyl, 2,3-dichlorophenyl, 3,4-dichlorophenyl, 3,5-dichlorophenyl, 2,4-dichlorophenyl, 2,3-difluorophenyl, 3,4-difluorophenyl, 3,5-difluorophenyl, 2,4-difluorophenyl, 2-fluoro-3-chlorophenyl, 2-chloro-3-fluorophenyl, 2-chloro-4-fluorophenyl, 2-fluoro-4-chlorophenyl, 4-fluoro-3-chlorophenyl, 4-fluoro-3-methylphenyl, 4-tert-butylphenyl, 4-fluoro-3-chlorophenyl, 4-bromo-3-fluorophenyl, 3,5-bis(trifluoromethyl)phenyl, 4-chloro-2-trifluoromethylphenyl, 2-methoxy-5-methylphenyl, 5-chloro-2-methoxyphenyl, 4-phenoxyphenyl, 2-methylthiophenyl, 3-methylthiophenyl, 4-methylthiophenyl, 5-fluoro-2-methoxyphenyl, 4-chloro-3-trifluoromethyl or 4-bromo-2-methylphenyl;
particularly preferably phenyl, benzyl, phenethyl, 4-fluorophenyl and 3-fluorophenyl.

**6.** Spirocyclic cyclohexane derivatives according to any of Claims 1 to 5, **characterized in that** $R^1$ and $R^2$ represent $CH_3$ and $R^3$ represents phenyl.

**7.** Spirocyclic cyclohexane derivatives according to any of Claims 1 to 6, **characterized in that** W represents $NR^4$, where $R^4$ represents H, $CH_3$, $C_2H_5$ or phenyl, and X represents NH or $NCOR^{12}$.

**8.** Process for preparing spirocyclic cyclohexane derivatives according to any of Claims 1 to 7, **characterized in that** a starting material of the general formula A

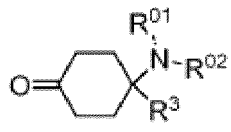

A

where the radicals $R^{01}$ and $R^{02}$ have the meaning given for $R^2$ and may additionally represent a protective group, with the addition of acid, or the trimethylsilyl ester thereof, is reacted in a suitable solvent with a starting material of the general formula B

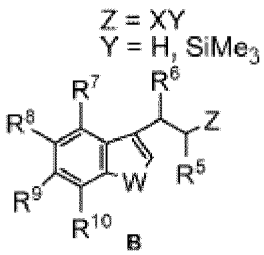

B

where the radicals $R^1$-$R^{10}$ and X have the meanings given in Claim 1.

**9.** Process for preparing spirocyclic cyclohexane derivatives according to Claim 1 in which X represents $NR^{17}$ and $R^{17}$ represents $COR^{12}$ or $SO_2R^{12}$, **characterized in that in that** a spirocyclic cyclohexane derivative in which X represents NH is, with addition of base, reacted with an anhydride or an acid chloride.

**10.** Medicament, comprising at least one spirocyclic cyclohexane derivative according to any of Claims 1 to 7, optionally in the form of its racemate, the pure stereoisomers, in any mixing ratio; in the form of its acids or its bases or in the form of its salts; or in the form of its solvates, and optionally comprising suitable additives and/or auxiliaries and/or optionally further active compounds.

**11.** Use of a spirocyclic cyclohexane derivative according to any of Claims 1 to 7, optionally in the form of its racemate, the pure stereoisomers, in any mixing ratio, in the form of its acids or its bases or in the form of its salts; or in the form of its solvates; for preparing a medicament for treating pain.

**12.** Use of a spirocyclic cyclohexane derivative according to any of Claims 1 to 7 for preparing a medicament for the treatment of states of anxiety, of stress and syndromes associated with stress, depression, epilepsy, Alzheimer's disease, senile dementia, general cognitive dysfunctions, impaired learning and memory (as a nootropic), withdrawal symptoms, alcohol and/or drug and/or medicament abuse and/or dependency, sexual dysfunctions, cardiovascular disorders, hypotension, hypertension, tinnitus, pruritus, migraine, deafness, lack of intestinal motility, impaired food uptake, anorexia, obesity, impaired locomotion, diarrhoea, cahexia, urinary incontinence, or as muscle relaxant, anticonvulsant or anaesthetic or for the coadministration during treatment with an opioid analgesic or with an anaesthetic, for diuresis or antinatriuresis, anxiolysis, for modulation of physical activity, for modulation of neurotransmitter release and for the treatment of neurodegenerative disorders associated therewith, for the treatment of withdrawal symptoms and/or for reducing the addictive potential of opioids.

**13.** Use according to Claim 11, where the pain is acute, neuropathic or chronic pain.

## Revendications

1. Dérivés de cyclohexane spirocycliques de formule générale I

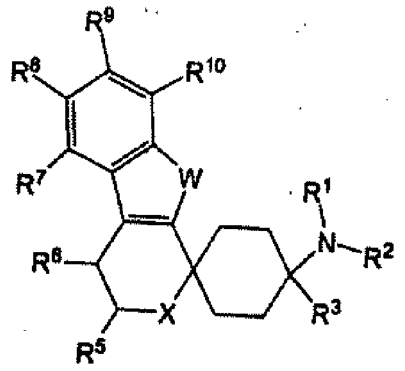

I

dans laquelle

$R^1$ et $R^2$ représentent, indépendamment l'un de l'autre, H, un groupe alkyle en $C_1$-$C_5$ ramifié ou non ramifié, saturé ou insaturé, non substitué, ou CHO ;
ou les radicaux $R^1$ et $R^2$ représentent ensemble un groupe $CH_2CH_2OCH_2CH_2$, $CH_2CH_2NR^{11}CH_2CH_2$ ou $(CH_2)_{3-6}$ ;
$R^{11}$ représentant H, ou un groupe alkyle en $C_1$-$C_5$ respectivement ramifié ou non ramifié, saturé ou insaturé, non substitué ;
$R^3$ représente un groupe alkyle en $C_1$-$C_5$ respectivement ramifié ou non ramifié, saturé ou insaturé, non substitué ou une ou plusieurs fois substitué ; (hétéro)cycloalkyle en $C_3$-$C_8$ respectivement saturé ou insaturé, non substitué ou une ou plusieurs fois substitué ; aryle, respectivement non substitué ou une ou plusieurs fois substitué ; aryle ou (hétéro)cycloalkyle en $C_3$-$C_8$, chacun non substitué ou une ou plusieurs fois substitué ; respectivement ramifié ou non ramifié, saturé ou insaturé, lié par un groupe alkyle en $C_1$-$C_3$ ;
W représente $NR^4$, 0 ou S ;
$R^4$ représentant H, un groupe alkyle en $C_1$-$C_5$ ramifié ou non ramifié, saturé ou insaturé, non substitué ou une ou plusieurs fois substitué ; ou aryle substitué ou non substitué ;
$R^{12}$ représentant H, un groupe alkyle en $C_1$-$C_5$ respectivement ramifié ou non ramifié, saturé ou insaturé, non substitué ou une ou plusieurs fois substitué ; (hétéro)cycloalkyle en $C_3$-$C_8$ respectivement saturé ou insaturé, non substitué ou une ou plusieurs fois substitué ; aryle ou hétéroaryle chacun non substitué ou une ou plusieurs fois substitué ; ou aryle, (hétéro)cycloalkyle en $C_3$-$C_8$ ou hétéroaryle, chacun non substitué ou une ou plusieurs fois substitué, liés par un groupe alkyle en $C_1$-$C_3$ ; respectivement ramifié ou non ramifié, saturé ou insaturé ; $OR^{13}$ ; $NR^{14}R^{15}$ ;
$R^5$ représente (hétéro)cycloalkyle en $C_3$-$C_8$ saturé ou insaturé, non substitué ou une ou plusieurs fois substitué ; aryle ou hétéroaryle, non substitué ou une ou plusieurs fois substitué ; ou (hétéro)cycloalkyle en $C_3$-$C_8$ ou hétéroaryle non substitué ou une ou plusieurs fois substitué, lié par alkyle en $C_1$-$C_3$ ; respectivement ramifié ou non ramifié, saturé ou insaturé ;
$R^6$ représente H, F, f Cl, $NO_2$, $CF_3$, $OR^{13}$, $SR^{13}$, $SO_2R^{13}$, $SO_2OR^{13}$, CN, $COOR^{13}$, $NR^{14}R^{15}$, alkyle en $C_1$-$C_5$ saturé ou insaturé, ramifié ou non ramifié, non substitué ou une ou plusieurs fois substitué ; aryle non substitué ou une ou plusieurs fois substitué ; ou aryle non substitué ou une ou plusieurs fois substitué, lié par alkyle en $C_1$-$C_3$ ; respectivement ramifié ou non ramifié, saturé ou insaturé ;
$R^7$, $R^8$, $R^9$ et $R^{10}$ représentent, indépendamment les uns des autres, H, F, Cl, Br, I, $NO_2$, $CF_3$, $OR^{13}$, $SR^{13}$, $SO_2R^{13}$, $SO_2OR^{13}$, $SO_2NH_2$, CN, $COOR^{13}$, $NR^{14}R^{15}$, alkyle en $C_1$-$C_5$, (hétéro)cycloalkyle en $C_3$-$C_8$ non substitué ou une ou plusieurs fois substitué ; aryle ou hétéroaryle, non substitué ou une ou plusieurs fois substitué ; ou aryle, (hétéro)cycloalkyle en $C_3$-$C_8$ ou hétéroaryle, non substitué ou une ou plusieurs fois substitué, lié par alkyle en $C_1$-$C_3$ ; respectivement ramifié ou non ramifié, saturé ou insaturé ;
$R^{13}$ représentant H, un groupe alkyle en $C_1$-$C_5$ respectivement saturé ou insaturé, ramifié ou non ramifié, non substitué, ou (hétéro)cycloalkyle en $C_3$-$C_8$ respectivement saturé ou insaturé, non substitué ;
$R^{14}$ et $R^{15}$ représentant indépendamment l'un de l'autre H, un groupe alkyle en $C_1$-$C_5$ respectivement ramifié

ou non ramifié, saturé ou insaturé, non substitué ;
ou $R^{14}$ et $R^{15}$ forment ensemble un groupe $CH_2CH_2OCH_2CH_2$, $CH_2CH_2NR^{16}CH_2CH_2$ ou $(CH_2)_{3-6}$, $R^{16}$ représentant H, un groupe alkyle en $C_1$-$C_5$ ramifié ou non ramifié, saturé ou insaturé, non substitué ;
X représente NMR ;
$R^{17}$ représentant H, un groupe alkyle en $C_1$-$C_5$ ramifié ou non ramifié, saturé ou insaturé, $COR^{19}$ ou $SO_2R^{12}$ ;
en relation avec "alkyle", par le terme "substitué", étant entendu le remplacement d'un ou plusieurs atomes d'hydrogène par F, Cl, Br, I, -CN, $NH_2$, NH-alkyle, NH-(hétéro)cycloalkyle, NH-alkyl-OH, $N(alkyle)_2$, $N[(hétéro)cycloalkyle]_2$, $N(alkyl-OH)_2$, $NO_2$, SH, S-alkyle, S-(hétéro)cycloalkyle, S-alkyl-OH, S-alkyl-SH, OH, O-alkyle, O-(hétéro)cycloalkyle, O-alkyl-OH, CHO, C(=O)-alkyle($C_1$-$C_6$), C(=S)alkyle($C_1$-$C_6$), C(=O)-(hétéro)cycloalkyle, C(=S)-(hétéro)cycloalkyle, $CO_2H$, $CO_2$-alkyle, C(=O)$NH_2$, C(=O)NH-alkyle, C(=O)NH-(hétéro)cycloalkyle, C(=O)-$N(alkyle)_2$, C(=O)$N[(hétéro)cycloalkyle]_2$, SO-alkyle, $SO_2$-alkyle, $SO_2NH_2$, $SO_3H$ ou (hétéro)cycloalkyle, aryle ou hétéroaryle ;
et
en ce qui concerne les radicaux "aryle" ainsi que "(hétéro)cycloalkyle", par l'expression "une ou plusieurs fois substitué" étant entendu le remplacement une ou plusieurs fois, par exemple deux, trois, quatre ou cinq fois, d'un ou plusieurs atomes d'hydrogène par F, Cl, Br, I, CN, NH, NH-alkyle, NH-(hétéro)cycloalkyle, NH-alkyl-OH, $N(alkyle)_2$, $N[(hétéro)cycloalkyle]_2$, $N(alkyl-OH)_2$, $NO_2$, SH, S-alkyle, S-(hétéro)cycloalkyle, S-alkyl-OH, S-alkyl-SH, OH, O-alkyle, O-(hétéro)cycloalkyle, O-alkyl-OH, CHO, C(=0)-alkyle ($C_1$-$C_6$), C(=S)alkyle ($C_1$-$C_6$), C(=O)-(hétéro)cycloalkyle, C(=S)-(hétéro)cycloalkyle, $CO_2H$, $CO_2$-alkyle, C(=O)$NH_2$, C(=O)NH-alkyle, C(=O)NH-(hétéro)cycloalkyle, C(=O)-$N(alkyle)_2$, C(=O)$N[(hétéro)cycloalkyle]_2$, S(O)-alkyle, $SO_2$-alkyle, $SO_2NH_2$, $SO_3H$, $CF_3$, =O, =S ; alkyle, (hétéro)cycloalkyle ou hétéroaryle ;
eu égard à "hétéroaryle", par "une ou plusieurs fois substitué" étant entendu le remplacement une ou plusieurs fois, par exemple deux, trois, quatre ou cinq fois, d'un ou plusieurs atomes d'hydrogène du système cyclique par F, Cl, Br, I, CN, $NH_2$, NH-alkyle, NH-aryle, NH-hétéroaryle, NH-alkyl-aryle, NH-alkyl-hétéroaryle, NH-(hétéro)cycloalkyle, NH-alkyl-OH, $N(alkyle)_2$, $N(alkyl-aryle)_2$, $N(alkyl-hétéroaryle)_2$, $N[(hétéro)cycloalkyle]_2$, $N(alkyl-OH)_2$, $NO_2$, SH, S-alkyle, S-(hétéro)cycloalkyle, S-aryle, S-hétéroaryle, S-alkyl-aryle, S-alkyl-hétéroaryle, S-alkyl-OH, S-alkyl-SH, OH, O-alkyle, O-(hétéro)-cycloalkyle, O-aryle, O-hétéroaryle, O-alkyl-aryle, O-alkyl-hétéroaryle, O-alkyl-OH, CHO, C(=O)-alkyle($C_1$-$C_6$), C(=S)alkyle($C_1$-$C_6$), C(=O)-(hétéro)-cycloalkyle, C(=S)-(hétéro)cycloalkyle, $CO_2H$, $CO_2$-alkyle, C(=O)$NH_2$, C(=O)NH-alkyle, C(=O)NH-(hétéro)-cycloalkyle, C(=O)-$N(alkyle)_2$, C(=O)$N[(hétéro)-cycloalkyle]_2$, S(O)-alkyle, $SO_2$-alkyle, $SO_2NH_2$, $SO_3H$, $CF_3$, =O, =S ; alkyle, (hétéro)cycloalkyle, aryle et/ou hétéroaryle ;
le terme "aryle" signifie un système cyclique carbocyclique comportant au moins un cycle aromatique, mais sans hétéroatomes dans aucun des cycles, les radicaux aryle pouvant éventuellement être condensés avec d'autres systèmes cycliques saturés, (partiellement) insaturés ou aromatiques ;
le terme "hétéroaryle" signifie un radical cyclique aromatique à 5, 6 ou 7 chaînons, qui contient au moins 1, éventuellement également 2, 3, 4 ou 5 hétéroatomes, les hétéroatomes étant des atomes d'azote, d'oxygène ou de soufre identiques ou différents, et l'hétérocycle pouvant également faire partie d'un système bi- ou polycyclique ;
le terme "(hétéro)cycloalkyle" signifie un radical hydrocarboné cyclique ayant 3, 4, 5, 6, 7 ou 8 atomes de carbone, les radicaux hydrocarbonés pouvant être saturés ou insaturés, mais non aromatiques, et éventuellement un ou deux atomes de carbone pouvant être remplacés par un hétéroatome S, N ou 0 ;
et
sous forme du racémate, des énantiomères, de diastéréoisomères, de mélanges des énantiomères ou diastéréoisomères ou d'un énantiomère ou diastéréoisomère individuel ; des bases et/ou de sels avec des cations ou des acides physiologiquement acceptables.

2. Dérivés de cyclohexane spirocycliques selon la revendication 1, **caractérisés en ce que** $R^3$ représente un groupe phényle, benzyle ou phénéthyle, chacun non substitué ou une ou plusieurs fois substitués sur le cycle.

3. Dérivés de cyclohexane spirocycliques selon l'une quelconque des revendications 1 à 2, **caractérisés en ce que** $R^7$, $R^8$, $R^9$ et $R^{10}$ représentent, indépendamment les uns des autres, H, un groupe alkyle en $C_1$-$C_5$ ramifié ou non ramifié, non substitué ou une ou plusieurs fois substitué, F, Cl, Br, I, $CF_3$, OH, $OCH_3$, $NH_2$, COOH, $COOCH_3$, $NHCH_3$, $N(CH_3)_2$, $NO_2$, $SO_3H$, $SO_2NH_2$, le groupe pyridyle ou phényle.

4. Dérivés de cyclohexane spirocycliques selon l'une quelconque des revendications 1 à 3, **caractérisés en ce que** $R^1$ et $R^2$ représentent, indépendamment l'un de l'autre, H ou $CH_3$, $R^1$ et $R^2$ ne représentant pas simultanément H.

5. Dérivés de cyclohexane spirocycliques selon l'une quelconque des revendications 1 à 4, **caractérisés en ce que**

$R^3$ représente un groupe phényle, benzyle ou phénéthyle, chacun non substitué ou une ou plusieurs fois substitué sur le cycle ;
en particulier phényle, benzyle, phénéthyle, 2-fluorophényle, 3-fluorophényle, 4-fluorophényle, 2-chlorophényle, 3-chlorophényle, 4-chlorophényle, 2-bromophényle, 3-bromophényle, 4-bromophényle, 2-cyanophényle, 3-cyanophényle, 4-cyanophényle, 2-méthylphényle, 3-méthylphényle, 4-méthylphényle, 2-méthoxyphényle, 3-méthoxyphényle, 4-méthoxyphényle, 2-trifluorométhylphényle, 3-trifluorométhylphényle, 3-trifluorométhylphényle, 2-éthylphényle, 3-éthylphényle, 4-éthylphényle, 2-éthoxyphényle, 3-éthoxyphényle, 4-éthoxyphényle, 2-hydroxyphényle, 3-hydroxyphényle, 4-hydroxyphényle, 2,3-dichlorophényle, 3,4-dichlorophényle, 3,5-dichlorophényle, 2,4-dichlorophényle, 2,3-difluorophényle, 3,4-difluorophényle, 3,5-difluorophényle, 2,4-difluorophényle, 2-fluoro-3-chlorophényle, 2-chloro-3-fluorophényle, 2-chloro-4-fluorophényle, 2-fluoro-4-chlorophényle, 4-fluoro-3-chlorophényle, 4-fluoro-3-méthylphényle, 4-tert-butylphényle, 4-fluoro-3-chlorophényle, 4-bromo-3-fluorophényle, 3,5-bis(trifluorométhyl)phényle, 4-chloro-2-trifluorométhylphényle, 2-méthoxy-5-méthylphényle, 5-chloro-2-méthoxyphényle, 4-phénoxyphényle, 2-méthylthiophényle, 3-méthylthiophényle, 4-méthylthiophényle, 5-fluoro-2-méthoxyphényle, 4-chloro-3-trifluorométhyle ou 4-bromo-2-méthylphényle ;
de façon particulièrement préférée phényle, benzyle, phénéthyle, 4-fluorophényle et 3-fluorophényle.

6. Dérivés de cyclohexane spirocycliques selon l'une quelconque des revendications 1 à 5, **caractérisés en ce que** $R^1$ et $R^2$ représentent $CH_3$ et $R^3$ représente le groupe phényle.

7. Dérivés de cyclohexane spirocycliques selon l'une quelconque des revendications 1 à 6, **caractérisés en ce que** W représente $NR^4$, $R^4$ représentant H, $CH_3$, $C_2H_5$ ou phényle, et X représente NH ou $NCOR^{12}$.

8. Procédé pour la préparation de dérivés de cyclohexane spirocycliques selon l'une quelconque des revendications 1 à 7, **caractérisés en ce qu'**on fait réagir un produit de départ de formule générale A

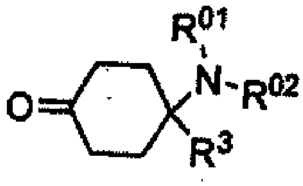

**A**

dans laquelle les radicaux $R^{01}$ et $R^{02}$ ont la signification indiquée pour $R^2$ et peuvent en outre représenter un groupe protecteur, avec addition d'acide, ou de son ester de triméthylsilyle, dans un solvant approprié avec un produit de départ de formule générale B

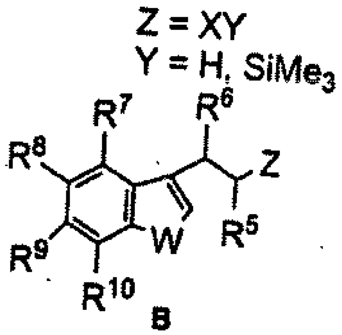

**B**

les radicaux $R^1$-$R^{10}$ et ayant les significations indiquées dans la revendication 1.

9. Procédé pour la préparation de dérivés de cyclohexane spirocycliques selon la revendication 1, dans lesquels X représente $NR^{17}$ et $R^{17}$ représente $COR^{12}$ ou $SO_2R^{12}$, **caractérisé en ce qu'**on fait réagir un dérivé de cyclohexane spirocyclique, dans lequel X représente NH, avec addition d'une base avec un anhydride ou un chlorure d'acide.

10. Médicament contenant au moins un dérivé de cyclohexane spirocyclique selon l'une quelconque des revendications 1 à 7, éventuellement sous forme de son racémate, des stéréoisomères purs, en un rapport de mélange quelconque ;

sous forme de ses acides ou de ses bases ou sous forme de ses sels ; ou sous forme de ses produits de solvatation ainsi qu'éventuellement contenant des additifs et/ou adjuvants et/ou éventuellement d'autres substances actives.

**11.** Utilisation d'un dérivé de cyclohexane spirocyclique selon l'une quelconque des revendications 1 à 7, éventuellement sous forme de son racémate, des stéréoisomères purs, en un rapport de mélange quelconque ; sous forme de ses acides ou de ses bases ou sous forme de ses sels ; ou sous forme de ses produits de solvatation pour la fabrication d'un médicament destiné au traitement de la douleur.

**12.** Utilisation d'un dérivé de cyclohexane spirocyclique selon l'une quelconque des revendications 1 à 7, pour la fabrication d'un médicament destiné au traitement d'états anxieux, du stress et de syndromes liés au stress, de dépressions, de l'épilepsie, de la maladie d'Alzheimer, de la démence sénile de dysfonctionnements cognitifs généraux, de troubles de la mémoire et de l'apprentissage (en tant qu'agent nootrope), de manifestations inflammatoires, d'abus d'alcool et/ou de drogues et/ou de médicaments et/ou de leur dépendance, de dysfonctionnements sexuels, de maladies cardiovasculaires, de l'hypotension, de l'hypertension, de l'acouphène, du prurit, de la migraine, de troubles de l'audition, de la motilité intestinale insuffisante, de l'alimentation perturbée, de l'anorexie, de l'adiposité, de troubles locomoteurs, de la diarrhée, de la cachexie, de l'incontinence urinaire ou en tant que myorelaxant, anticonvulsivant ou anesthésique, ou à l'administration simultanée dans le traitement par un analgésique opiacé ou par un anesthésique, à la diurèse ou à l'antinatriurèse, à l'anxiolyse, à la modulation de l'activité motrice, à la modulation de l'excrétion de neurotransmetteurs et au traitement de maladies neurodégénératives qui y sont liées, au traitement de manifestations inflammatoires et/ou à la réduction du potentiel de dépendance des opiacés.

**13.** Utilisation selon la revendication 11, dans laquelle la douleur est une douleur aiguë, névropathique ou chronique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **MEUNIER et al.** *Nature,* 1995, vol. 377, 532-535
- **REINSCHEID et al.** *Science,* 1995, vol. 270, 792-794
- **MOGIL et al.** *Neuroscience,* 1996, vol. 75, 333-337
- **JENCK et al.** *Proc. Natl. Acad. Sci. USA,* 1997, vol. 94, 14854-14858
- **KING et al.** *Neurosci. Lett.,* 1997, vol. 223, 113-116
- **ABDULLA ; SMITH.** *J. Neurosci.,* 1998, vol. 18, 9685-9694
- **MANABE et al.** *Nature,* 1997, vol. 394, 577-581
- **NISHI et al.** *EMBO J.,* 1997, vol. 16, 1858-1864
- **CALO et al.** *Br.J. Pharmacol.,* 2000, vol. 129, 1261-1283
- **JIRKOVSKY et al.** *J. Heterocycl. Chem.,* 1975, vol. 12, 937-940
- **CAMPAIGNE et al.** *J. Heterocycl. Chem.,* 1965, vol. 2, 231-235
- **EFANGE et al.** *J. Med. Chem.,* 1998, vol. 41, 4486-4491
- **ELLINGBOE et al.** *J. Med. Chem.,* 1992, vol. 35, 1176-1183
- **PEARSON et al.** *Aust. J. Chem.,* 1991, vol. 44, 907-917
- **YOKOHAMA et al.** *Chem. Pharm. Bull.,* 1992, vol. 40, 2391-2398
- **BECK et al.** *J. Chem. Soc. Perkin,* 1992, vol. 1, 813-822
- **SHINADA et al.** *Tetrahedron Lett.,* 1996, vol. 39, 7099-7102
- **GARDEN et al.** *Tetrahedron,* 2002, vol. 58, 8399-8412
- **LEDNICER et al.** *J. Med. Chem.,* 1980, vol. 23, 424-430
- **ARDATI et al.** *Mol. Pharmacol.,* 1997, vol. 51, 816-824
- **I.C. HENDERSHOT ; J. FORSAITH.** *J. Pharmacol. Exp. Ther.,* 1959, vol. 125, 237-240